(19) Europäisches Patentamt
European Patent Office
Office européen des brevets

(11) EP 0 684 954 B1

(12) FASCICULE DE BREVET EUROPEEN

(45) Date de publication et mention de la délivrance du brevet: **22.07.1998 Bulletin 1998/30**

(21) Numéro de dépôt: **94906099.0**

(22) Date de dépôt: **18.02.1994**

(51) Int Cl.6: **C07H 21/00**, C12Q 1/68

(86) Numéro de dépôt international: **PCT/BE94/00013**

(87) Numéro de publication internationale: **WO 94/19364 (01.09.1994 Gazette 1994/20)**

(54) **SONDES D'ACIDES NUCLEIQUES CHIMIQUEMENT MODIFIEES EN 5'(OH) ET/OU EN 3'(OH) EN VUE DE L'INTRODUCTION EN CES POSITIONS D'UN OU PLUSIEURS ELEMENTS DE MARQUAGE NON RADIOACTIF ET PROCEDE DE PREPARATION**

5'(OH) UND/ODER 3'(OH) ZUR EINFÜHRUNG VON EINEM ODER MEHREREN NICHTRADIOAKTIVMARKIERTEN MARKERN CHEMISCH MODIFIZIERTE NUKLEINSÄURESONDEN, UND VERFAHREN ZU IHRER HERSTELLUNG

NUCLEIC ACID PROBES CHEMICALLY 5'(OH)- AND/OR 3'(OH)-MODIFIED FOR INSERTING ONE OR MORE NON-RADIOACTIVE LABELING ELEMENTS THEREAT, AND METHOD FOR PREPARING SAME

(84) Etats contractants désignés:
**AT BE CH DE ES FR GB IT LI NL**

(30) Priorité: **19.02.1993 BE 9300160**

(43) Date de publication de la demande:
**06.12.1995 Bulletin 1995/49**

(73) Titulaire: **LA REGION WALLONNE**
**1040 Bruxelles (BE)**

(72) Inventeurs:
- **DE VOS, Marie-Joelle**
  **B-7181 Feluy (BE)**
- **BOLLEN, Alex**
  **B-1701 Itterbeek (BE)**

(74) Mandataire: **de Kemmeter, François et al**
**Cabinet Bede**
**Place de l'Alma, 3**
**1200 Bruxelles (BE)**

(56) Documents cités:

| | |
|---|---|
| **EP-A- 0 317 077** | **WO-A-90/00622** |
| **WO-A-91/17169** | **WO-A-92/03464** |

- **NUCLEIC ACIDS RESEARCH, vol.18, no.8, 1990, ARLINGTON, VIRGINIA US pages 2065 - 2068 WILK A ET AL 'Backbone-modified oligonucleotides containing a butanediol-1,3 moiety as a 'vicarious segment' for the deoxyribosyl moiety - synthesis and enzyme studies'**
- **NUCLEIC ACIDS RESEARCH, vol.18, no.15, ARLINGTON, VIRGINIA US pages 4345 - 4354 MISIURA K ET AL 'BIOTINYL AND PHOSPHOTYROSINYL PHOSPHORAMIDITE DERIVATIVES USEFUL IN THE INCORPORATION OF MULTIPLE REPORTER GROUPS ON SYNTHETIC OLIGONUCLEOTIDES'**
- **NUCLEIC ACIDS SYMPOSIUM SERIES, vol.25, 1991, OXFORD pages 85 - 86 BERLIN Y.A.ET AL 'Multiple non-radioactive labeling of oligonucleotides'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 684 954 B1

## Description

La présente invention concerne les sondes d'acides nucléiques non radioactives, et les composés chimiques utiles à la synthèse des dites sondes. L'emploi de sondes d'acides nucléiques pour détecter et diagnostiquer les maladies génétiques héréditaires, les oncogènes, les maladies virales, bactériennes ou parasitaires, se généralise de plus en plus et trouve des applications dans les domaines cliniques, vétérinaires et agro-alimentaires. Ces sondes sont généralement des séquences d'ADN ou d'ARN simples brins capables, sous certaines conditions expérimentales, de retrouver leurs séquences complémentaires et de s'hybrider avec elles pour former des duplex stables.

A l'origine, la méthode de détection des hybrides ADN-ADN ou ADN-ARN faisait appel à un marquage radioactif: la sonde est marquée avec un radioisotope, le plus souvent du $^{32}P$ et la détection après hybridation se fait par comptage ou autoradiographie.

Etant donnés les inconvénients liés à l'utilisation des radioisotopes (temps de demi-vie, coût et sécurité), l'utilisation de sondes "froides" (qui ne contiennent pas d'élément radioactif) se développe de plus en plus. Les sondes froides mettent en jeu des techniques de détection utilisant principalement des systèmes enzymatiques qui, en présence d'un substrat, donnent lieu à la production d'une couleur et, dans les systèmes plus récents, à la production d'une lumière (fluorescence, chémiluminescence, bioluminescence).

En ce qui concerne le marquage de telles sondes, les techniques qui ont été publiées peuvent se ranger en deux grandes catégories:

1. La première consiste à coupler la sonde à un élément directement détectable par exemple par couplage covalent d'une enzyme à la sonde (phosphatase alcaline, peroxydase).
2. La seconde catégorie consiste en la détection indirecte de l'hybride. Pour ce faire, on a recours à des substances intermédiaires qui reconnaissent des motifs fixés sur la sonde. Il s'agit principalement de systèmes basés sur l'interaction très forte entre la biotine et l'avidine ou la streptavidine, l'avidine ou la streptavidine étant conjuguées à une enzyme. Cette approche nécessite l'incorporation d'une ou plusieurs biotines à la sonde nucléique.

Les techniques basées sur l'utilisation de sondes froides, qu'elles relèvent d'une détection directe ou indirecte, nécessitent donc, au niveau de ces sondes, des modifications chimiques de la chaîne oligonucléotidique pour permettre son marquage.

Des oligodésoxyribonucléotides portant des fonctions chimiques telles que des amines primaires ou des thiols permettant le couplage avec des réactifs variés ont été décrits dans la littérature. L'introduction de ces groupes peut se faire, soit sur une ou plusieurs des bases, soit à une des extrémités 5' ou 3' de l'oligodésoxyribonucléotide. Des modifications chimiques au niveau des bases de la chaîne ont le désavantage d'interférer avec les appariements des bases lors de la mise en hybridation de l'oligonucléotide avec des séquences homologues. L'introduction de groupes fonctionnels en 5' ou en 3' est, à cet effet, plus judicieuse à condition que la technique proposée permette d'éloigner suffisamment la partie détectable de la partie oligonucléotidique de la sonde ainsi préparée.

Le document Nucleic Acids Research (1990), 18(15) 4345-4354 divulgue la préparation de sondes oligonucléotidiques possédant plusieurs éléments de marquage en 5'(OH).

Les éléments de marquage sont introduits par condensation d'un phosphoramidite, dans lequel les fonctions diméthoxytrityle et phosphoramidite se trouvent sur des atomes de carbone adjacents. Les groupements phosphoramidite contiennent déjà l'élément de marquage.

Cette approche ne permet pas, à partir d'une même synthèse oligonucléotidique, la préparation de plusieurs oligonucléotides diversement marqués ni l'accès à une large gamme de types de détection.

Le document ne prévoit pas non plus la possibilité d'influencer la sensibilité de détection des sondes en fonction du nombre de marqueurs.

Le document WO-A-9000622 divulgue des sondes d'hybridation oligonucléotidiques, des composés utilisables pour leur synthèse ainsi qu'un procédé de synthèse des sondes. Selon ce document, 2n groupes hydroxyles sont créés en 5'(OH) de la sonde et servent à introduire 2n fonctions acide carboxylique en vue du marquage de la sonde par chélation avec des métaux du groupe des lanthanides, par exemple l'europium. Le document exclut expressément l'utilisation d'haptènes ou autres groupes dont la visualisation est basée sur leur forte affinité à d'autres produits biologiques.

Le but de la présente invention est donc d'obtenir des sondes d'acides nucléiques :

- offrant une bonne hybridation avec les séquences cibles complémentaires;
- permettant une détection directe ou indirecte par des méthodes non radioactives et ce, avec un seuil de détection aussi bas que possible;
- facilement synthétisables, c'est-à-dire appropriées à une synthèse d'acides nucléiques automatique ou manuelle, notamment sur support solide.

A cet effet, la présente invention concerne des sondes d'acides nucléiques pour détecter une molécule d'ADN ou d'ARN, comportant

a) une partie oligonucléotidique ou oligodésoxynucléotidique constituée de
S : une séquence d'acide nucléique ADN ou ARN en fonction du type de molécule à détecter et
b) une partie non nucléotidique possédant des propriétés chimiques qui permettent la fixation directe ou indirecte d'une ou plusieurs unités de détection ou éléments de marquage M détectables de manière non isotopique par production de couleur ou de lumière, caractérisée en ce que la partie b) est constituée d'une chaîne d'unités phosphates entrecoupées de motifs alkyle à savoir
b1) certains motifs alkyle unissant les différents groupes phosphates et ne présentant aucune fonctionnalité particulière
b2) des motifs alkyle présentant des fonctions amines primaires qui permettent le couplage avec des réactifs variés pour réaliser la détection de manière directe ou indirecte, les motifs b2) étant liés à la partie a) ou séquence S par l'intermédiaire des motifs b1).

Conformément à l'invention, la séquence S est liée à son extrémité 5'et/ou 3' à un ou plusieurs éléments de marquage M.

L'édifice moléculaire qui possède les fonctions chimiques X, permettant la fixation directe ou indirecte d'une ou plusieurs unités de détection peut présenter entre autres deux types de structure:

- une structure en "PEIGNE" où les fonctions chimiques X sont disposées en chaîne linéaire.

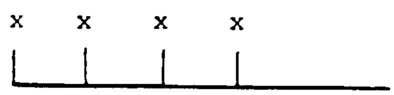


- une structure en "CANDELABRE" où les fonctions chimiques X sont disposées en chaîne ramifiée.

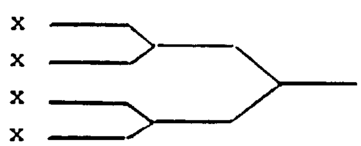


La présente invention concerne les deux types de structure.

I. **Sondes d'acides nucléiques chimiquement modifiées en 5'(OH) et (ou) en 3'(OH) par un édifice moléculaire en "PEIGNE".**

Dans de telles sondes, la partie nucléotidique consiste en une séquence d'acides nucléiques définie et homologue d'un fragment complémentaire cible, elle apporte l'énergie de stabilisation et assure l'hybridation avec la molécule d'ADN ou d'ARN que l'on veut détecter. Elle est constituée d'une chaîne d'unités phosphates entrecoupées de motifs riboses.

La partie non nucléotidique qui fournit la réactivité permettant la détection de l'hybride, directe ou indirecte, susceptible d' être introduite en l'extrémité 5'(OH) et/ou 3'(OH) de la séquence d'acides nucléiques décrite précédemment, est constituée d'une chaîne linéaire d'unités phosphates entrecoupées de motifs alkyle à savoir

L: certains motifs alkyle ne présentent aucune fonctionnalité chimique particulière; la succession de tels motifs, encore appelés "bras chimique" consistent à introduire des espacements entre les unités de détection;
M: certains motifs alkyle présentent des fonctions amine primaire qui permettent le couplage avec des réactifs variés pour conduire à la formation de motifs de détection directe ou indirecte.

Les motifs M sont séparés les uns des autres par un nombre variable de bras chimiques L, M comporte une molécule, synthétique ou naturelle, directement ou indirectement détectable de manière non isotopique. Une sonde ainsi constituée répond à la formule générale

$$[(M)_{y'}-(L)_{x'}]_{z'}-S-[(L)_x-(M)_y]_z \qquad (I)$$

dans laquelle S, L et M ont les significations données ci-dessus x, x', z, z' sont des nombres égaux ou supérieurs à 0, avec la restriction que z et z' ne sont jamais simultanément égaux à 0, y et y' sont des nombres supérieurs à 0. La modification chimique se fait donc en 5' et/ou en 3' de la séquence d'acides nucléiques de telle sorte que l'on peut détailler la formule I de la manière suivante :

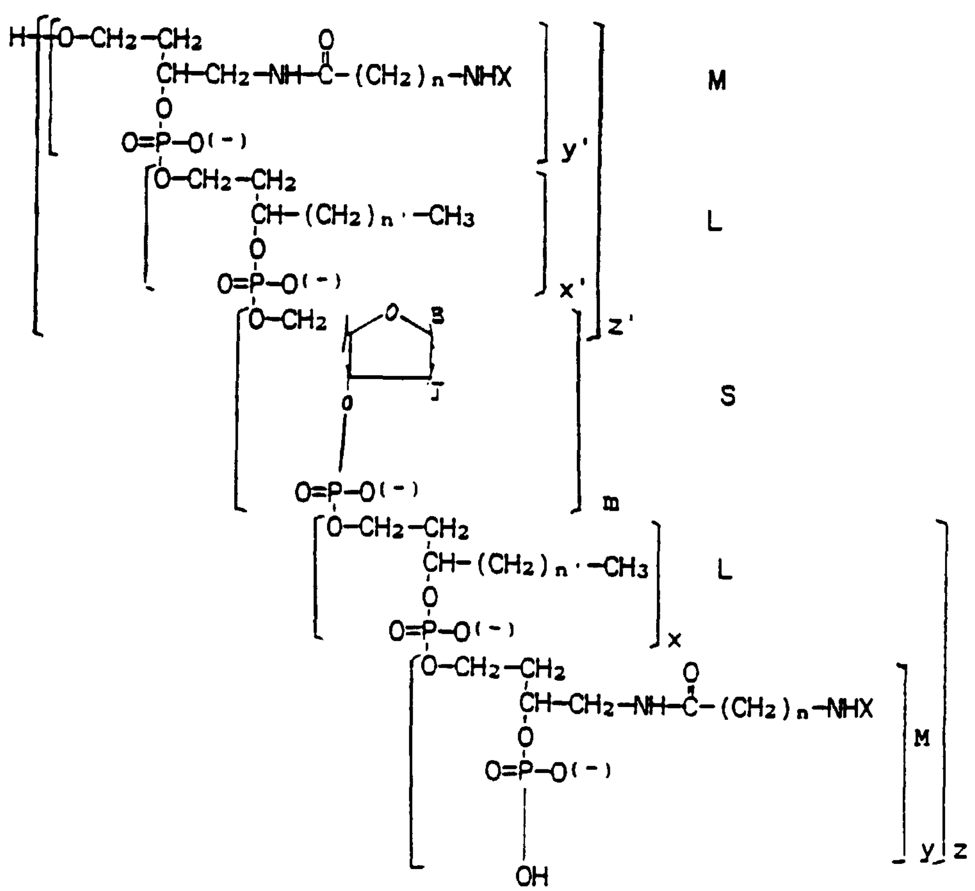

(Ia)

On peut décrire les unités S, L et M de la façon suivante :

S: la séquence d'acides nucléiques

J = H ou OH
m représente le nombre de nucléotides de 1 à 1000
B base d'acide nucléique, purique ou pyrimidique, variable selon les nucléotides.

On rappelle que les acides nucléiques sont des polymères de nucléotides, ribonucléotides dans le cas des ARN, désoxyribonucléotides dans le cas des ADN. Les monomères, c'est-à-dire les nucléotides, sont constitués dans le cas de l'ARN par de l'acide phosphorique, un ose à cinq atomes de carbone, le ribose (J=OH) et l'une des quatre bases fondamentales: adénine, guanine, cytosine, uridine. On rencontre plus rarement des bases ou nucléosides dits mineurs, tels les bases méthylées ou hydroxylées, le dihydrouracile et la pseudo-uridine. Dans le cas de l'ADN, l'ose des désoxyribonucléotides est le D-2désoxyribose (J=H) et les quatre bases principales sont l'adénine, la guanine, la cytosine et la thymine; dans de rares cas, la cytosine est remplacée par la méthylcytosine ou l'hydroxyméthylcytosine. Une des caractéristiques essentielles des polynucléotides est la liaison inter-nucléotidique 3'-5' phosphodiester.

L : le bras chimique

x, x' = 0 à 100
n'= 0 à 20

Le bras chimique est un polymère non-nucléotidique. Une des caractéristiques essentielles de ce polymère est, tout comme pour l'ADN et l'ARN, le lien phosphodiester qui unit les monomères.
Le monomère est constitué par de l'acide phosphorique, un diol: le propane 1-3 diol substitué sur le carbone 1 par une chaîne alkyle latérale.

M : l'élément de marquage

y, y' = 1 à 100
n = 2 à 20

X : le marqueur,

soit direct: phosphatase alcaline, peroxydase, fluorescéine, toute enzyme capable, en présence d'un substrat, de produire une coloration, ou une lumière;
soit indirect: biotine, digoxigénine, tout haptène capable d'être reconnu par des anticorps marqués de manière non-isotopique.

L'élément de marquage est un polymère non nucléotidique. Une des caractéristiques essentielles de ce polymère est, tout comme pour l'ADN et l'ARN, le lien phosphodiester entre les monomères.

Le monomère est constitué par de l'acide phosphorique, un diol: le propane 1,3-diol substitué sur le carbone 1 par un méthylène acétamido-alcane, la chaîne alkyle est fonctionnalisée en position terminale par une amine portant le marqueur.

Enfin, l'élément LM responsable de la détection de la sonde peut être introduit en 5'(OH) pour z'≠0; z=0 ou en 3'(OH) pour z'=0; z≠0 ou encore simultanément en 5'(OH) et en 3'(OH) pour z'≠0; z≠0 avec de façon générale z et z' = 0 à 100.

La synthèse du composé la peut donc se réaliser par synthèse internucléotidique classique puisque les différents éléments de I, à savoir S, L et M sont des polymères dont les monomères sont reliés entre eux par des liens phosphodiester.

La présente invention a donc pour objet un procédé de préparation de sondes de formule la comportant:

A1) la synthèse d'une séquence d'acides nucléiques S

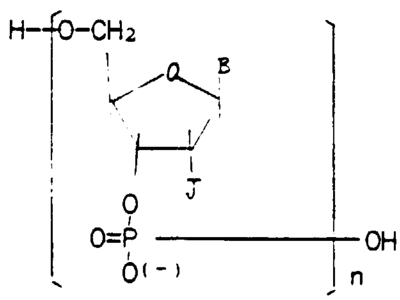

par toute méthode de synthèse d'assemblage internucléotidique manuelle ou automatique connue, de préférence sur support solide,
et
A2) la fixation d'un marqueur, caractérisé en ce que la dite séquence est soumise, de préférence par la même méthode de synthèse, notamment sur support solide, soit, à une extension en son extrémité 5'(OH) par une série d'unités M et L soit, à une extension en son extrémité 3'(OH) par une série d'unités M et L soit, à une extension en ses extrémités 5'(OH) et 3'(OH) par deux séries d'unités M et L
B) les dites unités L sont obtenues par la synthèse d'un polymère non nucléotidique L

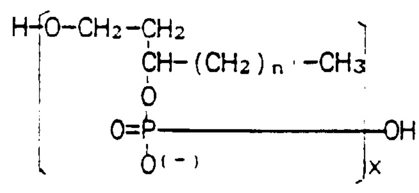

dans laquelle n'et x ont les significations données plus haut,

et

C) les dites unités M sont obtenues par la synthèse d'un polymère non nucléotidique M

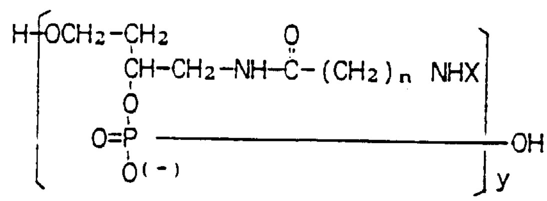

dans laquelle n et y ont les significations données plus haut, et X représente

soit un marqueur: phosphatase alcaline, peroxydase, fluorescéine, biotine, digoxigénine, tout haptène capable d'être reconnu par des anticorps marqués de manière non isotopique,

soit un groupe protecteur transitoire de la fonction amine primaire destiné à être éliminé après synthèse totale de la sonde d'acides nucléiques I. A titre illustratif, dans cette approche de synthèse, x peut représenter un acétyle VI, un trifluoroacétyle VII, un benzoyle VIII.

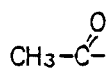

VI

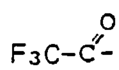

VII

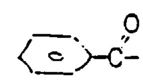

VIII

et les synthèses B et C ci-dessus se font de préférence aussi par toute méthode de synthèse d'assemblage internucléotidique manuelle ou automatique connue, et en particulier sur support solide.

Plus particulièrement, chaque élément de bras chimique L est introduit par condensation d'un composé de formule

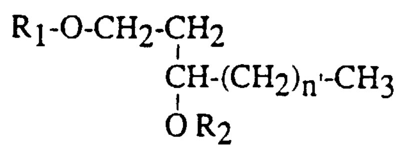

(II)

où $R_1$ et $R_2$ sont tels que définis ci-après à l'extrémité d'un nucléotide ou bien à l'extrémité d'un synthon déjà existant.

Selon une autre particularité de l'invention, chaque élément ou unité de détection M est obtenu au départ de butène-3ol-1 de formule

$$HO\text{-}CH_2\text{-}CH_2\text{-}CH{=}CH_2 \qquad (XXX)$$

par les étapes de

a) protection de la fonction alcool primaire au moyen d'un groupe protecteur $R_1$ pour obtenir un composé de formule

$R_1-O-CH_2-CH_2-CH=CH_2$ (XXXI)

b) époxydation de la double liaison du composé (XXXI) pour former un composé de formule

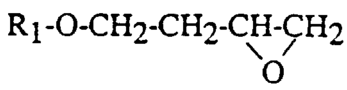 (XIIa)

c) ouverture de l'époxyde au moyen d'ammoniaque pour former un composé de formule

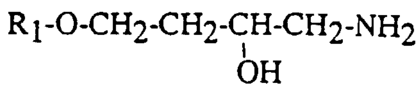 (XIIIa)

d) protection du groupe amino primaire d'un composé de formule

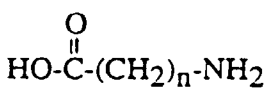 (XXXII)

pour former un composé de formule

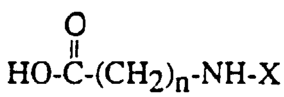 (XXXIII)

où X a les significations données plus haut

e) activation du composé (XXXIII)

f) condensation des composés (XIIIa) et (XXXIII) entre eux pour former un composé de formule

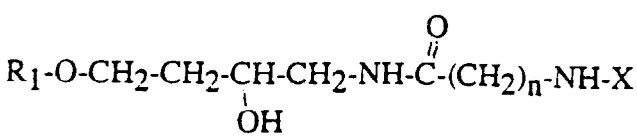 (XVa)

et

g) phosphorylation du composé (XVa) pour former un composé de formule

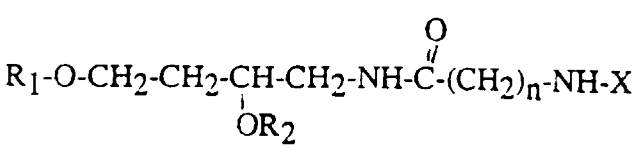 (III)

Dans les formules précitées, $R_1$ est un groupe protecteur de la fonction alcool primaire; à titre illustratif, en cas de synthèse aux phosphoramidites sur support solide, $R_1$ peut représenter le groupe 4,4'-diméthoxytrityle de formule IV (DMT) labile en milieu acide.

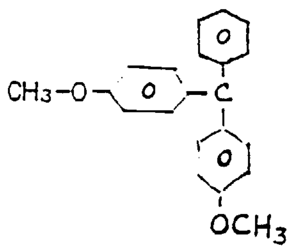

DMT (IV)

et

$R_2$ représente H ou tout groupe phosphorylé éventuellement protégé, approprié à l'introduction du synthon II ou III à l'extrémité 5' d'un nucléotide ou d'un synthon, déjà condensé sur le support solide pour un type donné de synthèse d'assemblage internucléotidique.

$R_2$ peut représenter, à titre illustratif en cas de synthèse aux phosphoramidites sur support solide, le groupe cyanoéthoxydiisopropylaminophosphoramidite de formule V.

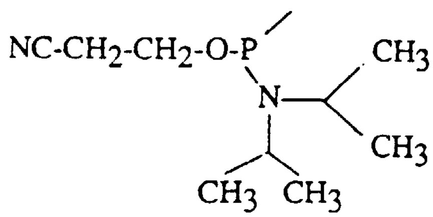

(V)

La présente invention a également pour objet deux composés synthétiques non nucléotidiques utiles respectivement comme intermédiaires pour la synthèse des fragments M et L du composé la par toute méthode de synthèse d'assemblage internucléotidique manuelle ou automatique connue, de préférence sur support solide. Les deux composés synthétiques revendiqués sont représentés par les formules II et III.

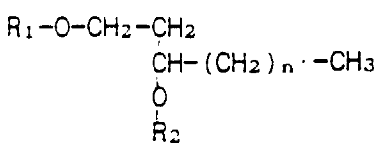

(II)

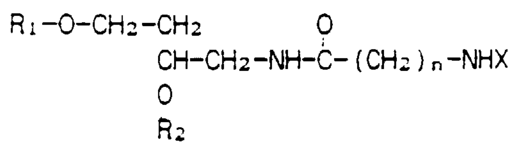

(III)

dans lesquelles $R_1$, $R_2$, n, n' et X ont les significations données précédemment.

L'invention concerne également la préparation des composés intermédiaires II et III par les procédés décrits ci-dessus.

En cas de synthèse aux phosphoramidites, la synthèse des composés II peut être représentée par le schéma suivant:

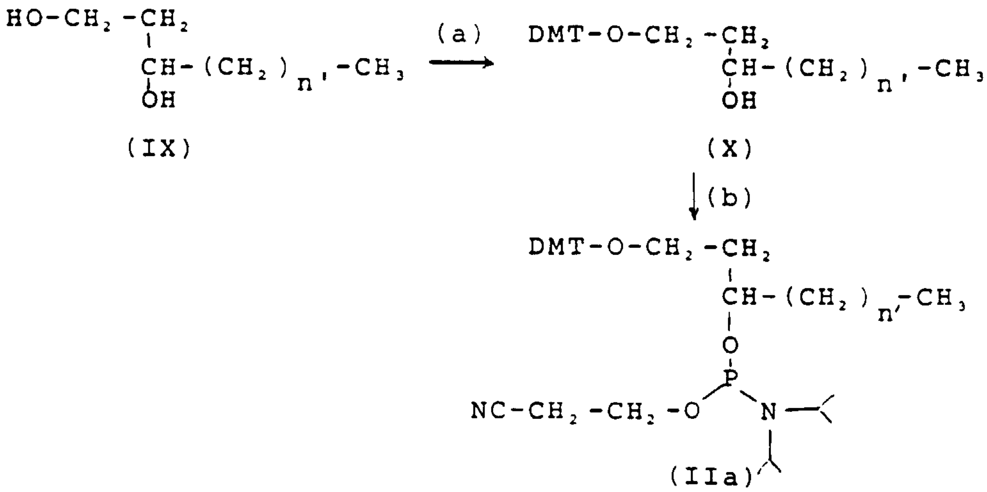


(IX) (X) (IIa)

Egalement en cas de synthèse aux phosphoramidites, le procédé de préparation des composés de formule III peut être représenté par le schéma ci-dessous:

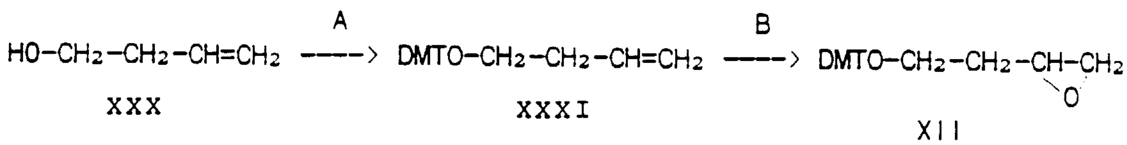


XXX XXXI XII

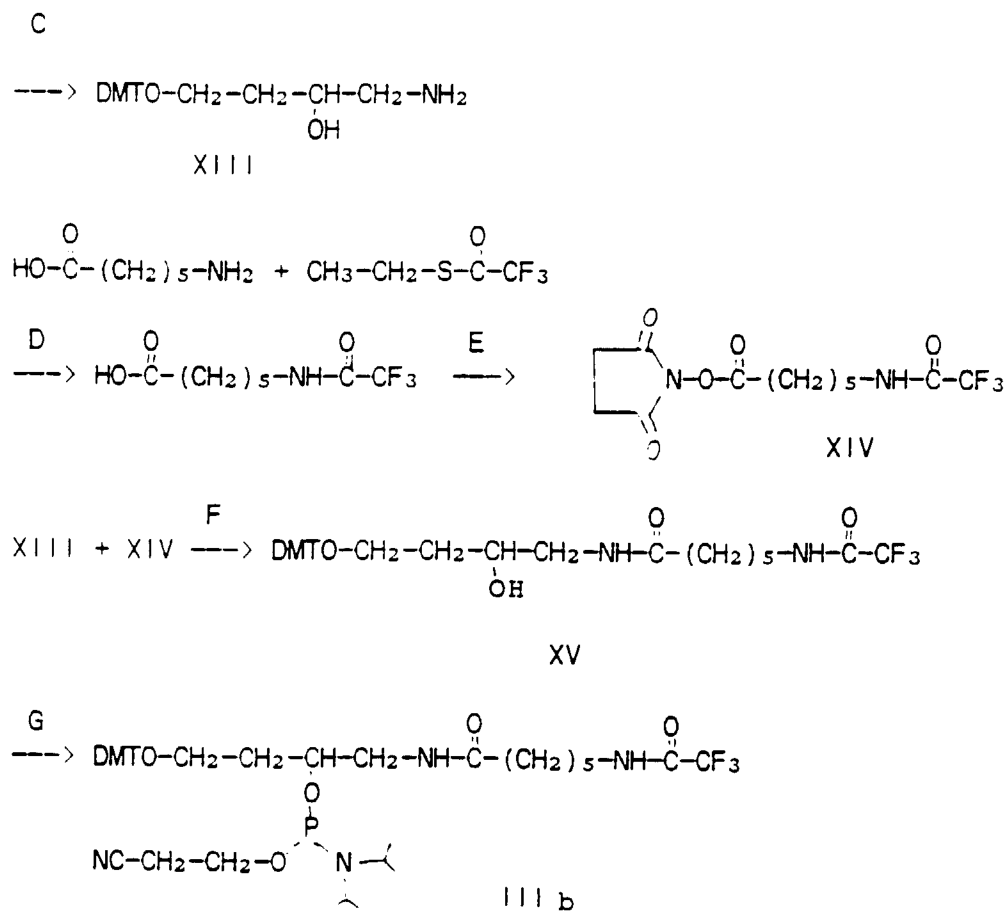


XIII

XIV

XV

III b

étape A) protection de l'alcool primaire du butèn-3 ol par le chlorure de 4,4'-diméthoxytrityle
étape B) époxydation de la double liaison par l'acide méta-chloroperbenzoïque
étape C) ouverture de l'époxyde par l'ammoniaque pour conduire au composé XIII.
étape D) protection par un groupe trifluoroacétyle de l'amine primaire de l'acide 6-aminocaproïque.
étape E) activation de l'acide 6-trifluoroacétylamidocaproïque par la N-hydroxysuccinimide pour conduire au composé XIV.
étape F) préparation du composé XV par condensation entre XIII et XIV.
étape G) phosphorylation de l'alcool secondaire XV avec un réactif qui permet le couplage ultérieur en phase solide du dérivé III ainsi obtenu par un type de synthèse d'assemblage internucléotidique. La phosphorylation, en cas de synthèse aux phosphoramidites, peut se faire avec la diisopropylaminocyanoéthoxychlorophosphine XI.

Par ailleurs, la présente invention a pour objet un procédé de fonctionnalisation d'un support solide CPG (controlled porous glass) par le composé de formule X. Le support solide ainsi fonctionnalisé permettra la préparation de sondes d'acides nucléiques de type I dans lesquelles z est non nul, c'est-à-dire dans lesquelles un marquage est introduit en 3' de la sonde. En effet, toute méthode de synthèse d'assemblage internucléotidique manuelle ou automatique connue, de préférence sur support solide, opère par une élongation de la chaîne en formation du 3' vers le 5'. Dans le cas de sondes d'acides nucléiques de type I dans lesquelles z est non nul, il est donc nécessaire de démarrer la synthèse sur un support solide fonctionnalisé par un composé de formule X ou XV. Toutefois, en raison de la facilité d'obtention du dérivé X par rapport au composé XV, nous avons choisi de fonctionnaliser le support avec X.

La méthode revendiquée pour la fonctionnalisation du support solide - le support est initialement recouvert de fonctions amine primaire - comporte les étapes suivantes :

- acylation de l'alcool secondaire de X par l'anhydride succinique pour conduire au composé de formule XVII

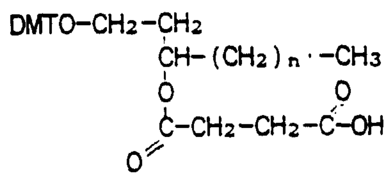

XVII

- activation de la fonction acide carboxylique du composé XVII par le pentachlorophénol en présence de carbodiimide qui produit l'ester XVIII.

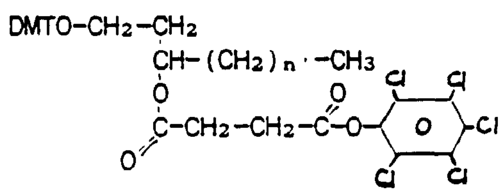

XVIII

- Fixation du dérivé XVIII sur un support solide aminé. Le support solide fonctionnalisé XIX est ainsi obtenu.

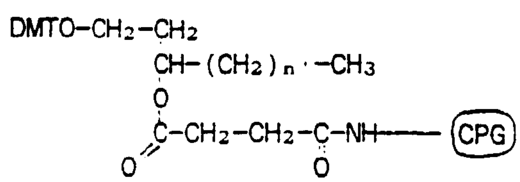

XIX

**II. <u>Sondes d'acides nucléiques chimiquement modifiées en 5'(OH) par un édifice moléculaire en "CANDELABRE"</u>**

Dans de telles sondes, la partie nucléotidique consiste en une séquence d'acides nucléiques définie et homologue d'un fragment complémentaire cible, elle apporte l'énergie de stabilisation et assure l'hybridation avec la molécule d'ADN ou d'ARN que l'on veut détecter. Elle est constituée d'une chaîne d'unités phosphates entrecoupées de motifs riboses.

La partie qui fournit la réactivité permettant la détection de l'hybride, directe ou indirecte, est introduite en l'extrémité 5'(OH) de la séquence d'acides nucléiques décrite précédemment. Elle est constituée d'une chaîne ramifiée d'unités phosphates entrecoupées de motifs alkyle:

les motifs alkyle internes à la ramification, c'est-à-dire unissant les différents groupes phosphates, ne présentent aucune fonctionnalité chimique particulière.

les motifs alkyle externes à la ramification, c'est-à-dire terminant les différents bras de la ramification, possèdent en bout de chaîne une fonction amine primaire.

La présente invention a donc pour objet une sonde d'acides nucléiques XX comportant une séquence d'acides nucléiques S, ADN ou ARN, caractérisée en ce que ladite séquence S est liée à son extrémité 5' à un élément de marquage M.

La modification chimique se fait donc en 5'(OH) de la séquence d'acides nucléiques de telle sorte que l'on peut détailler la formule XX, par exemple, de la manière suivante :

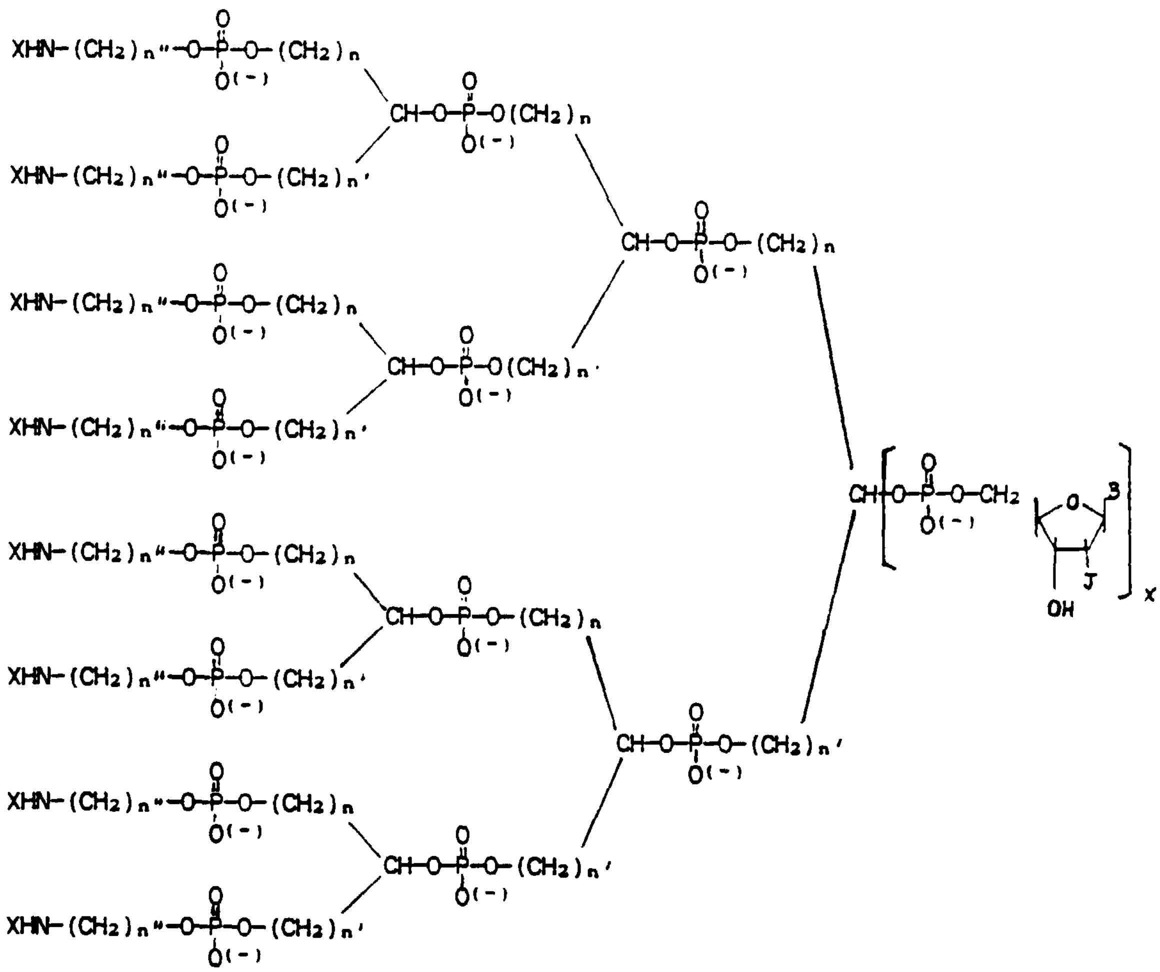

M S
XX

On peut décrire les unités S et M de la façon suivante :

S: la séquence d'acides nucléiques

J - H ou OH
x représente le nombre de nucléotides de 1 à 1000
B base nucléique, purique ou pyrimidique, variable selon les nucléotides

On rappelle que les acides nucléiques sont des polymères de nucléotides, ribonucléotides dans le cas des ARN, désoxyribonucléotides dans le cas des ADN. Les monomères, c'est-à-dire les nucléotides, sont constitués, dans le cas de l'ARN, par de l'acide phosphorique, un ose à cinq atomes de carbone, le ribose (J=OH) et l'une des quatre bases fondamentales : adénine, guanine, cytosine, uridine, Dans le cas de l'ADN, l'ose des désoxyribonucléotides est le D-2-désoxyribose (J=H) et les quatre bases principales sont l'adénine, la guanine, la cytosine et la thymine. Une des caractéristiques essentielles des polynucléotides est la liaison internucléotidique 3'-5' phosphodiester.

M: l'élément de marquage, il se termine dans l'exemple décrit cidessus par huit bras aminés. On l'appellera dès lors d'ordre huit. Néanmoins, l'ordre de ramification peut prendre des valeurs de 2 à 128 (l'ordre supérieur étant toujours double de celui qui le précède).
n, n' et n" = 1 à 20

X : le marqueur,

soit direct: phosphatase alcaline, peroxydase, fluorescéine, toute enzyme capable, en présence d'un substrat, de produire une coloration ou une lumière.
soit indirect: biotine, digoxigénine, tout haptène capable d'être reconnu par des anticorps marqués de manière non-isotopique.

L'élément de marquage est un polymère ramifié non nucléotidique. Une des caractéristiques essentielles de ce polymère est, tout comme pour l'ADN et l'ARN, le lien phosphodiester entre les monomères.

Le polymère ramifié M est constitué à partir de deux types de monomères distincts:

- le monomère responsable de la construction de l'édifice ramifié. Il est constitué par de l'acide phosphorique et un triol: UN l,n,ω alcane triol.
- le monomère responsable de l'introduction en bout de ramification, des fonctions amine primaire. Il est constitué par de l'acide phosphorique et un amino-alcool, le 1-amino hexan 6-ol.

La synthèse du composé XX peut donc se réaliser par synthèse internucléotidique classique puisque les différents éléments de XX sont des polymères dont les monomères sont reliés entre eux par des liens phosphodiesters.

La présente invention a donc également pour objet un procédé de préparation de sondes de formules XX comportant la synthèse d'une séquence d'acides nucléiques S :

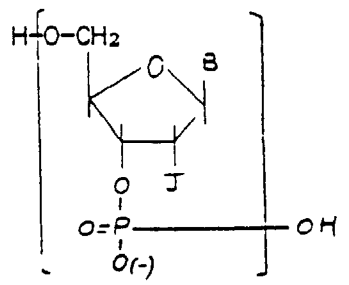

dans laquelle B et J ont les significations données précédemment, par toute méthode de synthèse d'assemblage internucléotidique manuelle ou automatique connue, de préférence sur support solide, caractérisée en ce que ladite séquence est soumise, de préférence par la même méthode de synthèse, notamment sur support solide, à une extension en son extrémité 5'(OH) par un édifice moléculaire ramifié M dont les bras terminaux portent chacun une fonction amine primaire.

L'édifice moléculaire ramifié peut répondre à la formule suivante :

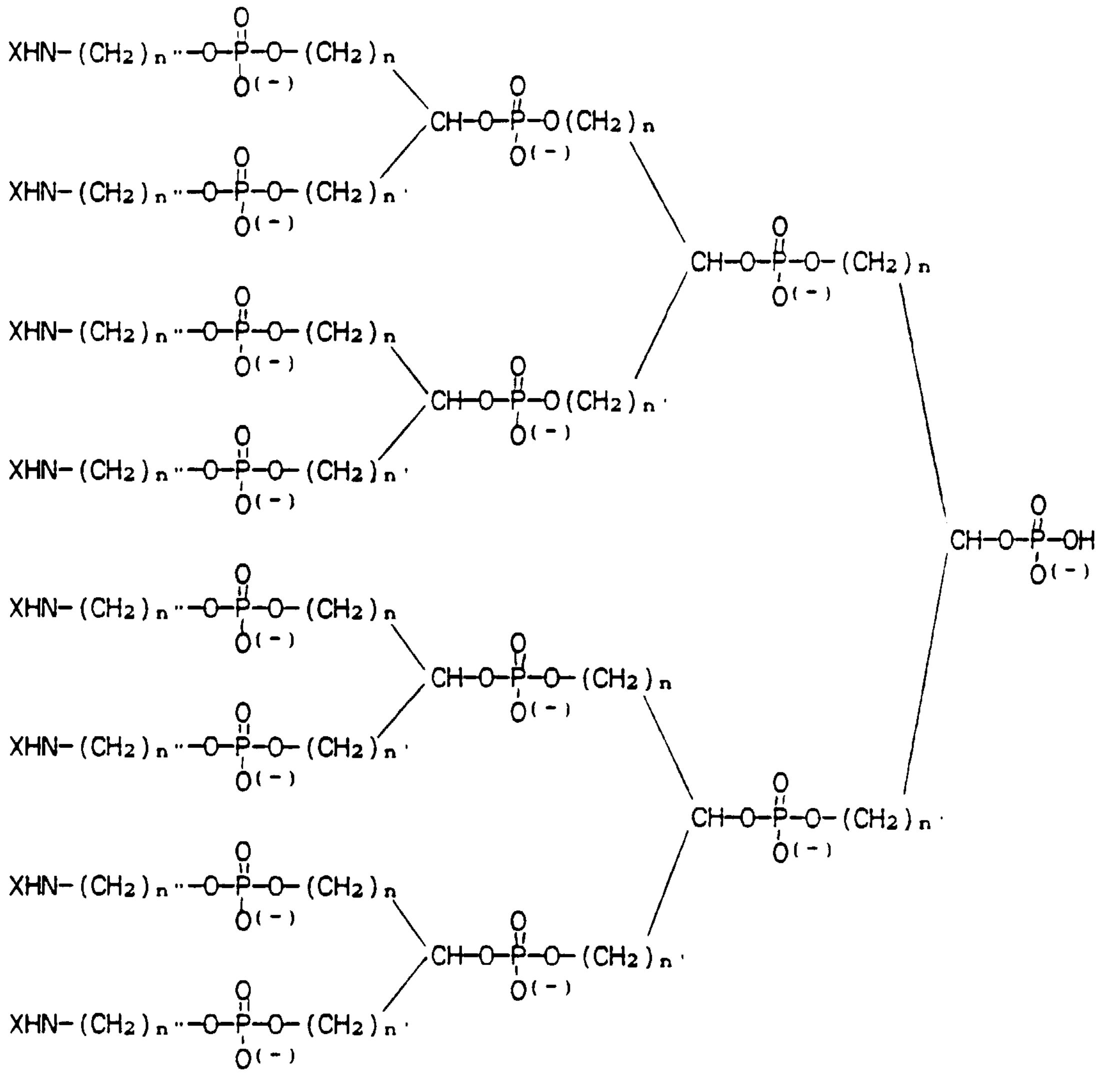

et peut être synthétisé plus particulièrement par toute méthode de synthèse d'assemblage internucléotidique manuelle ou automatique connue, de préférence sur support solide.

En particulier, après synthèse de la chaîne oligonucléotidique S dont les fonctions phosphates et les bases sont protégées, on condense à l'extrémité 5'(OH) de ladite chaîne une série de composés de formule

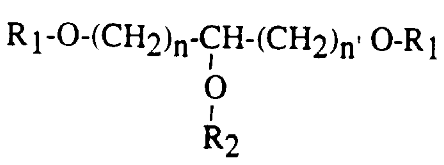

XXI

dans lesquels $R_1$ et $R_2$ ont les significations données précédemment.

Outre le composé XXI, la synthèse de M nécessite encore l'utilisation du composé XXII pour l'introduction des bras aminés en bout de ramification.

$$XHN\text{-}(CH_2)_{n''}\text{-}OR_2 \qquad XXII$$

Ce composé est connu dans la littérature.

Dans la formule XXII, X représente

- soit un marqueur: phosphatase alcaline, peroxydase, fluorescéine, biotine, digoxigénine, tout haptène capable d'être reconnu par des anticorps marqués de manière non isotopique.
- soit un groupe protecteur transitoire de la fonction amine primaire destiné à être éliminé après synthèse totale de la sonde d'acides nucléiques XX. A titre illustratif, dans cette approche de synthèse, X peut représenter un trifluoroacétyle VII, un fluorénylméthoxycarbonyle XXIII.

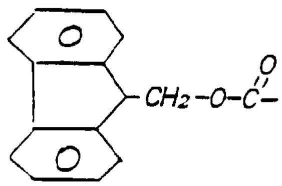

XXIII

et $R_2$ a les significations données précédemment.

Conformément à l'invention, le composé de formule XXI est obtenu au départ d'un alcanetriol de formule

$$HO-(CH_2)_n-\underset{\displaystyle OH}{CH}-(CH_2)_{n'}-OH \qquad XXIV$$

par les étapes suivantes de

a) protection de la fonction alcool primaire au moyen d'un groupe protecteur $R_1$ pour obtenir un composé de formule

$$R_1O-(CH_2)_n-\underset{\displaystyle OH}{CH}-(CH_2)_{n'}-OR_1 \qquad (XXVa)$$

b) transformation de la fonction alcool secondaire en un groupement $OR_2$ pour obtenir un composé de formule

$$R_1O-(CH_2)_n-\underset{\displaystyle OR_2}{CH}-(CH_2)_{n'}-OR_1 \qquad (XXI)$$

dans laquelle $R_2$ a les significations données précédemment.

Plus particulièrement, à l'étape a) la protection se fait au moyen du chlorure de 4,4'-diméthoxytrityle, et à l'étape b) a lieu une phosphorylation de la fonction alcool secondaire au moyen de la diisopropylaminocyanoéthoxychlorophosphine (XI).

L'invention concerne également les composés de formule XXI, à titre d'intermédiaires de synthèse des sondes de formule XX, ainsi qu'un procédé, tel que décrit ci-dessus, pour la préparation des composés intermédiaires de formule XXI. D'autres caractéristiques et avantages de la présente invention apparaîtront à la lumière des exemples qui vont suivre.

EXEMPLE I: <u>Préparation du 1-0-(4,4'-diméthoxytrityl) 3-0-[N,N'diisopropylamino-2 cyanoéthoxyphosphino] 1,3-butanediol</u>

Le dérivé 1-0-(4,4'-diméthoxytrityl) 3-0-[N,N' diisopropylamino,2cyanoéthoxyphosphino] 1,3-butanediol 1 répond à la formule :

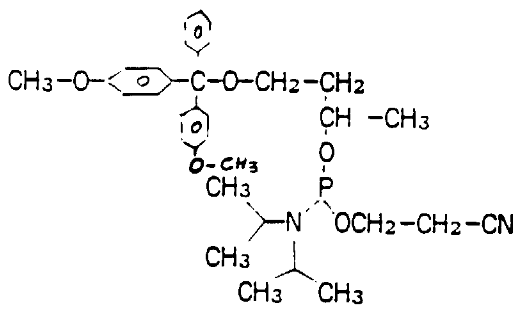

1

L'approche suivie a pour objectif la préparation d'un synthon non nucléotidique portant cependant les groupes chimiques qui permettent son introduction dans une chaîne oligonucléotidique ou dans un polymère non nucléotidique dont les monomères sont unis par un lien phosphodiester dans les conditions de synthèse automatique d'ARN ou d'ADN habituelles. Ce synthon a l'avantage de fournir à l'utilisateur un outil permettant l'introduction de "bras chimiques" en 3'(OH) et (ou) en 5'(OH) d'une séquence d'acides nucléiques sans sortir de la routine de synthèse automatique ou manuelle. Le rôle du bras chimique dans cet assemblage est d'introduire des espacements entre les différents éléments de marquage d'une part, et entre les éléments de marquage et la séquence d'acides nucléiques d'autre part, en vue d'une meilleure efficacité des deux parties respectivement dans leur rôle d'hybridation et de détection.

Les groupes 4,4'-diméthoxytrityle et 2-cyanoéthoxydiisopropylamino phosphoramidite sont appropriés à une synthèse aux phosphoramidites, notamment sur support solide.

Le chemin réactionnel pour l'obtention du composé 1 présenté dans le schéma I ci-après comporte les étapes suivantes :

1. Protection sélective de l'alcool primaire du 1,3-butanediol 2 par le groupe 4,4'-diméthoxytrityle (DMT) labile à pH acide.
2. Phosphorylation de l'alcool secondaire 3. L'exemple du phosphoramidite montré dans le schéma n'est pas limitatif; on peut envisager aussi la synthèse d'un phosphate triester ou diester ou d'un phosphonate.

Schéma I

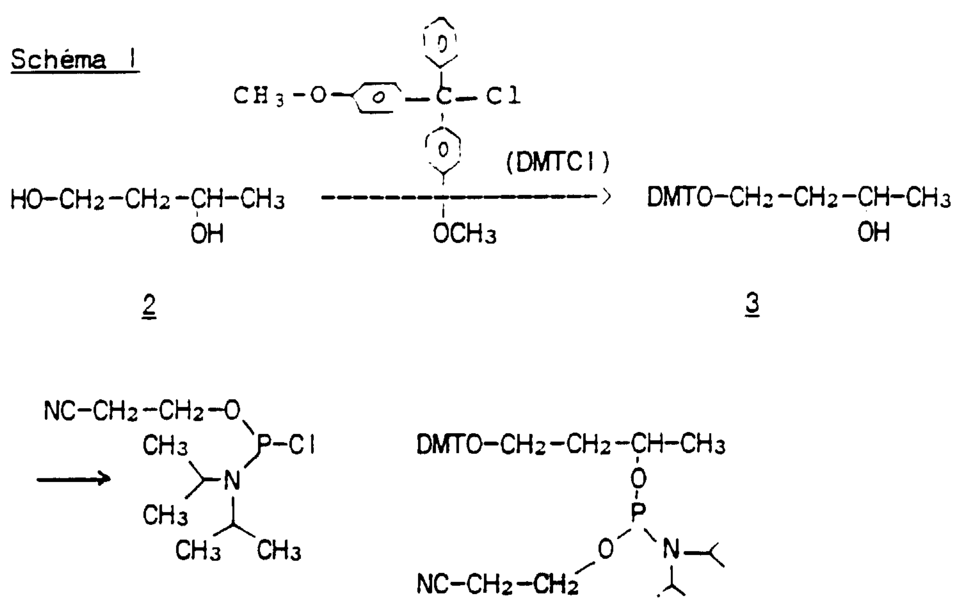


2

3

1

Procédé de préparation du 1-0-(4,4'-diméthoxytrityl)1,3-butanediol 3

Dans un ballon bicol de 100cc, on met 2,25gr (25mmoles) de 1,3butanediol 2 sous atmosphère d'argon. On ajoute 40cc de pyridine anhydre puis on ajoute à la solution, par petites portions 11 gr (32.5mmoles) de chlorure de 4,4'-diméthoxytrityle. On agite magnétiquement à température ambiante et sous argon pendant 1h30. On ajoute alors au

mélange réactionnel 10cc de méthanol. Cette opération a pour but de neutraliser l'excès de chlorure de 4,4'-diméthoxytrityle. On hydrolyse alors le mélange réactionnel (100cc de $NaHCO_3$ 5%), on extrait deux fois au dichlorométhane ($CH_2Cl_2$-2 fois 75cc). La phase organique obtenue est lavée trois fois avec une solution de bicarbonate de sodium 5% ($NaHCO_3$ 5%). La phase organique est ensuite séchée sur sulfate de magnésium ($MgSO_4$), filtrée puis évaporée (évaporateur rotatif p=20mm Hg).

Le résidu est purifié sur colonne de silice Merck 9385- éluant $CH_2Cl_2$. La silice est préalablement neutralisée par mise en suspension, dans une solution de $CH_2Cl_2$ contenant 1% de diisopropyléthylamine (DIEA). Après purification, on récupère 6.79g du composé 3 (17.32mmoles) Rdt. 69%.

Chromatographie sur couche mince (C.C.M.) plaque Merck 5735 (gel de silice $60F_{254}$) éluant $CH_2Cl_2$: RF = 0.3.

Procédé de préparation du 1-0-(4,4'-diméthoxytrityl) 3-0-[N,N' diisopropylamino)-2-cyanoéthoxyphosphinol] 1,3-butanediol 1

Dans un ballon bicol de 100cc, on introduit 6.7gr de composé 3 (17.3mmoles). On met sous atmosphère d'argon puis on ajoute 52cc de tétrahydrofuranne anhydre et 9cc (51.9mmoles) de diisopropyléthylamine. On ajoute ensuite au mélange réactionnel à la seringue et goutte à goutte 5.52g (23.35mmoles, 5cc) de 2-cyanoéthoxydiisopropylamino chlorophosphine.

Après dix minutes de réaction, un important précipité apparaît au sein du mélange réactionnel (chlorhydrate de diisopropyléthylamine). On filtre ce précipité, on ajoute au filtrat 100cc d'acétate d'éthyle, on lave la phase organique ainsi obtenue trois fois avec une solution de $NaHCO_3$ 5%. La phase organique est alors séchée sur $MgSO_4$, filtrée et évaporée (évaporateur rotatif, p=20mm Hg). Le résidu est purifié sur colonne de silice Merck 9385 - éluant acétate d'éthyle 4-hexane 6. La silice a préalablement été neutralisée par mise en suspension dans de l'éluant contenant 1% de DIEA. Après purification, on récupère 9.24g du composé 1. Rdt. 90%.

Chromatographie sur couche mince (C.C.M.) plaque Merck 5735 (gel de silice $60F_{254}$), éluant Acétate d'éthyle 5 - hexane 5. RF : 0.8 (le produit de départ de cette réaction 3 a, dans ces conditions, un RF de 0.5).

EXEMPLE II : Préparation du 1-0-(4,4'-diméthoxytrityl)3-0-(N,N'-diisopropylamino 2-cyanoéthoxyphosphino) 4-amido-(6-trifluoroacétylamido) caproate 4-amino 1,3-butanediol 4

Le dérivé 1-0-(4,4'-diméthoxytrityl)3-0-(N,N'-diisopropylamino 2-cyanoéthoxyphosphino) 4-amido-(6-trifluoroacétylamido) caproate 4-amino 1,3-butanediol 4 répond à la formule :

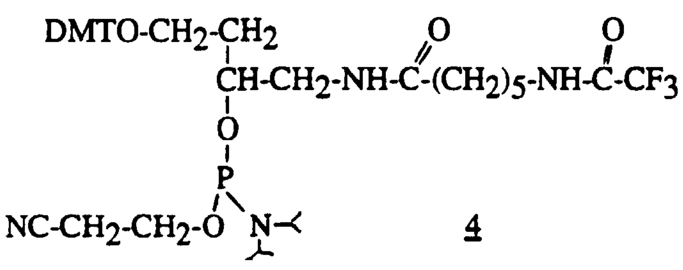

4

L'approche suivie a pour objectif la préparation d'un synthon non nucléotidique portant cependant les groupes chimiques qui permettent son introduction dans une chaîne oligonucléotidique ou dans un polymère non-nucléotidique dont les monomères sont unis par un lien phosphodiester dans les conditions habituelles de synthèse automatique ou manuelle d'ARN ou d'ADN.

Ce système a l'avantage de fournir à l'utilisateur un outil permettant l'introduction en 3'(OH) et (ou) en 5'(OH) d'une séquence d'acides nucléiques, un ou plusieurs éléments de marquage non isotopique, sans sortir de la routine de synthèse automatique ou manuelle. Les éléments de marquage sont séparés de la chaîne oligonucléotidique d'une part, et séparés les uns des autres d'autre part, par des "bras chimiques". Le rôle direct de l'élément de marquage tel que décrit dans cet exemple, est d'introduire en 5'(OH) et (ou) en 3'(OH) d'une chaîne oligonucléotidique des bras "amine primaire" séparés les uns des autres par des "bras chimiques". Les bras aminés sont capables, de par leur nucléophilicité, de fixer ultérieurement un élément de détection directe ou indirecte (enzyme, fluorescéine, biotine, digoxigénine,..). Les groupes 4,4'-diméthoxytrityle et N,N' diisopropylamino 2-cyanoéthoxy phosphoramidite sont appropriés à une synthèse aux phosphoramidites. Le chemin réactionnel pour l'obtention du composé 4 présenté dans le schéma II ci-après comporte les étapes suivantes :

1. Protection de l'alcool du 3-butène 1-ol 5 par le 4,4'-diméthoxytrityl (DMT) labile à pH acide.

2. Epoxydation de l'oléfine terminale 6 par l'acide metachloroperbenzoïque.
3. Ouverture de l'époxyde 7 par l'ammoniaque
4. Protection de l'amine primaire de l'acide 6-aminocaproïque par le groupe trifluoroacétyle.
5. Activation de l'acide carboxylique du dérivé 9 en ester N-hydroxysuccinimique 10.
6. Condensation entre le composé 8 et le composé 10.
7. Phosphorylation de l'alcool secondaire 11. L'exemple du phosphoramidite montré dans le schéma n'est pas limitatif. On peut aussi envisager la synthèse d'un phosphate triester ou diester ou d'un phosphonate.

Schéma II

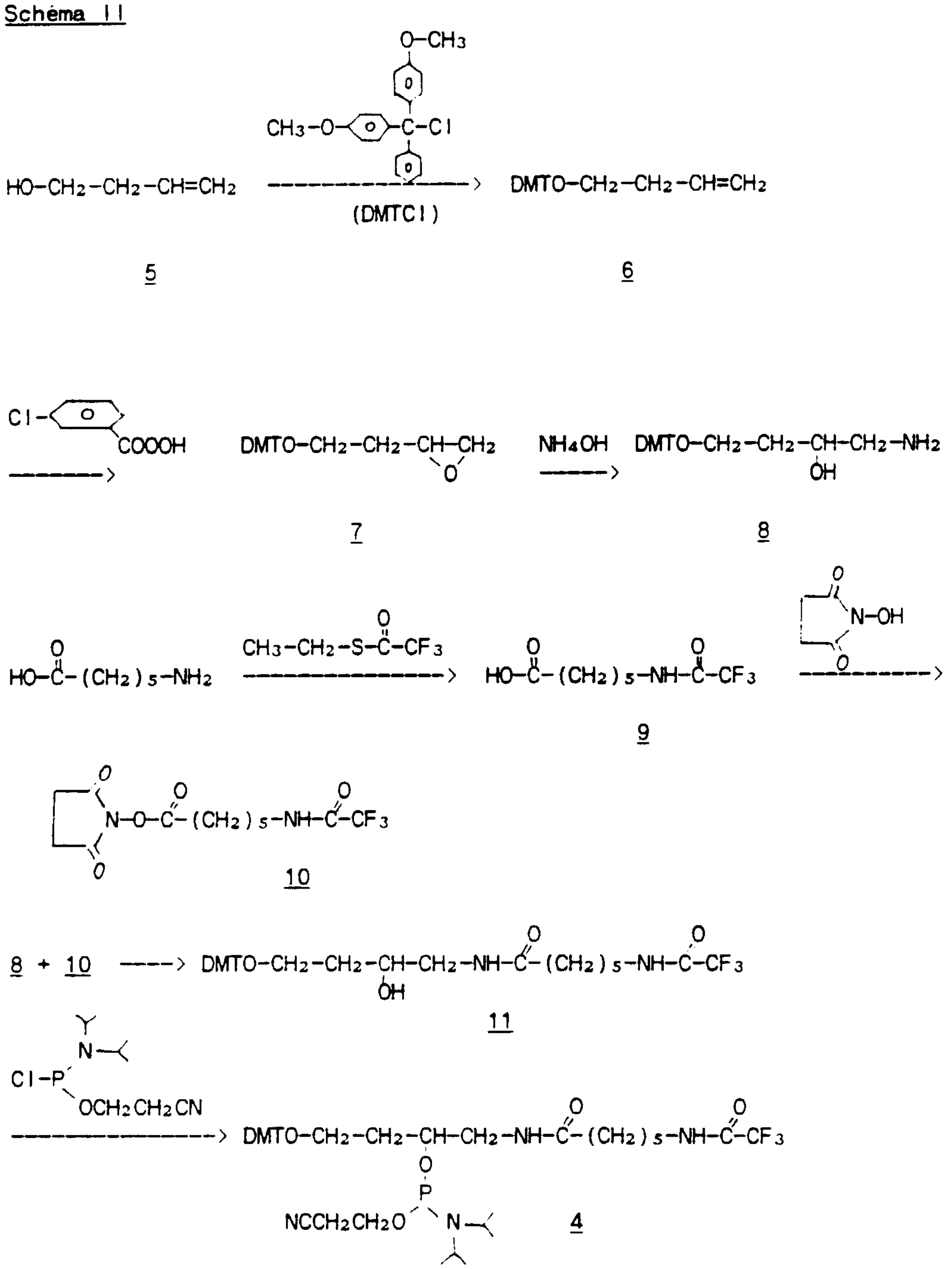


5 6 7 8 9 10 11 4

Procédé de préparation du 1-O-(4,4'-diméthoxytrityl) 3-butène 1-ol 6

Dans un ballon bicol de 250 ml. on met 3,6 gr (50 mmoles) de 3-butène 1-ol sous argon, en solution dans 80cc

de pyridine anhydre. On introduit par petites portions 22 g (65 mmoles) de chlorure de 4,4'diméthoxytrityle (la réaction est légèrement exothermique).

On maintient à température ambiante et sous agitation magnétique pendant 4 h. On ajoute alors au mélange réactionnel 10cc de méthanol afin de neutraliser l'excès de chlorure de 4,4'-diméthoxytrityle. On hydrolyse alors le mélange réactionnel avec 100cc de $NaHCO_3$ 5%. On extrait le mélange deux fois avec 150cc de dichlorométhane. La phase organique obtenue est lavée trois fois avec $NaHCO_3$ 5%, trois fois à l'eau et une fois avec une solution aqueuse saturée en NaCl. La phase organique obtenue est séchée sur $MgSO_4$, filtrée et évaporée (évaporateur rotatif p: 20 mm Hg).

Le résidu obtenu est ensuite coévaporé avec du toluène afin d'entraîner azéotropiquement la pyridine résiduelle, puis coévaporé avec du dichlorométhane afin d'entraîner azéotropiquement le toluène résiduel. Le résidu est alors purifié sur colonne de silice Merck 9385 éluant: hexane 1 - dichlorométhane - 9. La silice est préalablement neutralisée par mise en suspension dans l'éluant contenant 1% de DIEA. Le composé 6 est obtenu avec 90% de rendement.

Chromatographie sur couche mince (CCM) plaque Merck 5735 (gel de silice $60F_{254}$) éluant dichlorométhane RF: 0.85.

Procédé de préparation du 1-O-(4,4'-diméthoxytrityl) 3,4-butène oxyde 7

L'acide m-chloroperbenzoïque commercial contient 50% d'eau; afin d'éliminer cette eau, on le met en solution dans du dichlorométhane et on élimine l'eau par décantation. Dans un erlen de 100cc, on introduit 8,5 g d'acide m-chloroperbenzoïque à 50% (24.6 mmoles), on le met en solution dans 64cc de dichlorométhane. On décante la phase aqueuse surnageant. Dans un ballon de 250cc, on met 6.35 g de composé 6 (17 mmoles) sous atmosphère d'argon. On le met en solution dans 40cc de dichlorométhane. On ajoute ensuite lentement la solution de peracide préparée (comme décrit ci-dessus). On agite le mélange réactionnel magnétiquement pendant 8 h. On filtre alors le précipité qui s'est formé (acide m-chlorobenzoïque). On ajoute au filtrat 150cc de dichlorométhane et on l'hydrolyse avec 75cc de $NaHCO_3$ 5%. La phase organique est lavée quatre fois avec 75cc de $NaHCO_3$ 5%, puis séchée sur $MgSO_4$, filtrée et évaporée (évaporateur rotatif p=20 mm Hg). Le résidu est purifié sur colonne de silice Merck 9385 - éluant hexane 2 - dichlorométhane 8. La silice est préalablement neutralisée par mise en suspension dans l'éluant contenant 1% de DIEA. Après purification, on obtient 5.32 g d'epoxyde 7, Rdt 80%.

Procédé de préparation du 1-O-(4,4'-diméthoxytrityl) 4-amino 1,3-butanediol 8

Dans un ballon en pyrex de 25cc muni d'un bouchon à visser et capable de supporter des pressions importantes, on introduit 3g (7,6 mmoles) d'époxyde 7, 10cc d'acétonitrile et 25cc d'ammoniaque à 32%. On chauffe 8 heures à 60°C puis on évapore l'eau et l'acétonitrile. Le résidu obtenu est purifié sur colonne de silice Merck 9385 éluant $CH_2Cl_2$-$CH_3OH$, la proportion de méthanol est progressivement portée de 2% à 10%. La silice est préalablement neutralisée par mise en suspension dans l'éluant de départ contenant 1% de DIEA. Après purification, on obtient le dérivé 8 avec un rendement de 75%. Chromatographie sur couche mince (CCM) - plaque Merck 5735, gel de silice $60F_{254}$ - éluant 20% $CH_3OH$ - 80% $CH_2Cl_2$, RF 0.2.

Procédé de préparation de l'acide 6-trifluoroacétamidocaproïque 9

Dans un ballon rotavapor de 50cc, on introduit 2.62g ($2x10^{-2}$ moles) d'acide 6-aminocaproïque. On le met en suspension dans 4cc de diméthylformamide anhydre, puis on ajoute à la suspension 3cc ($2,35 \times 10^{-2}$ moles) de S-éthyl trifluoro thioacétate. On met sous atmosphère d'argon et on agite 4 heures, la solution est alors limpide. On évapore la diméthylformamide sous pression réduite (P = 20mm Hg). Le résidu est cristallisé dans le dichlorométhane. Le composé 9 est obtenu avec un rendement de 84%. Chromatographie sur couche mince (CCM)- plaque Merck 5735, gel de silice $60F_{254}$ - éluant 20% $CH_3OH$-80% $CH_2Cl_2$. RF 0.5.

Procédé de préparation du 6-trifluoroacétamido caproate de N-hydroxysuccinimide 10.

Dans un ballon de 100ml, on introduit 2,27 g ($10^{-2}$ mole) de composé 9, 2,04 g ($10^{-2}$ mole) de dicyclohexylcarbodiimide et 1,15 g ($10^{-2}$ mole) de N-hydroxysuccinimide. On ajoute 40cc de $CH_2Cl_2$ et on agite 5 heures sous atmosphère d'argon. Un précipité blanc s'est formé, il est filtré. Le filtrat est évaporé. Le résidu obtenu ne présente qu'une seule tache en chromatographie sur couche mince, il n'est pas purifié, le rendement est quantitatif. Chromatographie sur couche mince (CCM) - plaque Merck 5735, gel de silice $60F_{254}$ - éluant 5% $CH_3OH$-95% $CH_2Cl_2$. RF 0.7.

Procédé de préparation du 1-O-(4,4'-diméthoxytrityl) 4-amido(6trifluoroacétylamido) caproate 4-amino 1,3-butanediol 11

Dans un bicol de 50cc, on met sous atmosphère d'argon 2,44 g (6 mmoles) de composé 8 et 3,44 g de composé 10 (9,45 mmoles). On met en solution dans 25cc de diméthylformamide anhydre et on agite 16 heures. On évapore alors la diméthylformamide au rotavapor sous pression réduite (P = 20 mmHg). Le résidu obtenu est purifié sur colonne de silice Merck 9385, éluant 5 hexane - 95 $CH_2Cl_2$, puis $CH_3OH$ 2⇒10 - $CH_2$ $Cl_2$ 98⇒90. La silice est préalablement neutralisée par mise en suspension dans l'éluant initial contenant 1% de DIEA. Après purification. le composé 11 est obtenu avec 84% de rendement. Chromatographie sur couche mince (CCM)- plaque Merck 5735, gel de silice $60F_{254}$ - éluant 10% $CH_3OH$-90% $CH_2Cl_2$. RF 0.8.

Procédé de préparation du 1-O-(4,4'-diméthoxytrityl) 3-O-(N,N'diisopropylamino 2-cyanoéthoxyphosphino) 4-amido-(6-trifluoroacétylamido) caproate 4-amino 1,3-butanediol 4

Dans un ballon bicol de 50cc, on met sous atmosphère d'argon 2 g (3 mmoles) de dérivé 11 On ajoute 17 ml de tetrahydrofuranne anhydre, 1,6cc de diisopropyléthylamine et 0.88cc (4,1 mmoles) de N,N' diisopropylamino 2-cyanoéthoxychlorophosphine. On agite 1 heure à température ambiante, on ajoute alors à la solution 25cc d'acétate d'éthyle et 20cc de $NaHCO_3$ 5%. La phase organique obtenue est ensuite lavée trois fois avec $NaHCO_3$ 5%, elle est séchée sur $MgSO_4$, filtrée et évaporée sous vide (P = 20mm Hg). Le résidu est purifié sur colonne de silice Merck 9385, éluant hexane 15 - acétate d'éthyle 85. La silice est préalablement neutralisée par mise en suspension dans l'éluant contenant 1% de DIEA. Après purification, on obtient le dérivé 4 avec 62% de rendement. Chromatographie sur couche mince (CCM) - éluant hexane 10 - acétate d'éthyle 90. RF 0.75.

EXEMPLE III: Etude de la réactivité des dérivés 1 et 4

Les phosphoramidites non nucléotidiques 1 et 4 peuvent être utilisés pour introduire en 5'(OH) et (ou) en 3'(OH) d'un oligonucléotide, respectivement:

- des bras chimiques (dérivé 1)
- des bras "amine primaire" (dérivé 4)

Les bras "amine primaire" sont séparés les uns des autres et séparés de la chaîne oligonucléotidique par des bras chimiques.

Dans le but de réaliser de tels édifices biopolymériques,la réactivité des composés 1 et 4 a été testée dans les conditions de synthèse automatique d'ADN ou d'ARN habituelles.

A cet effet, les dérivés 1 et 4 ont été condensés dans l'appareil de synthèse automatique des oligonucléotides, ils ont été condensés en 5'(OH) d'un dimère thymidine-thymidine (T-T) fixé sur un support solide (support CPG - Controlled Porous Glass, classique en synthèse automatique).

Si l'on appelle N le bras aminé provenant de la condensation d'une unité 4 et S le bras chimique provenant de la condensation d'une unité 1, les dimères T-T modifiés en 5'(OH) qui ont été synthétisés sont les suivants :

NTT 12
STT 13
NSTT 14
SNSTT 15

Les dérivés 1 et 4 se fixent avec succès en 5'(OH) d'un oligonucléotide en élongation (cfr. NTT, STT). Le composé 4 se fixe avec succès à la suite d'un bras chimique (cfr. NSTT) et le composé 1 se fixe bien à la suite d'un bras aminé (cfr. SNSTT). Toutes les conditions (temps de couplage, concentration réactifs et solvants) habituellement utilisées au cours d'un cycle d'élongation oligonucléotidique se sont révélées adéquates en ce qui concerne la condensation de 1.

Néanmoins, pour la condensation de 4, les conditions habituellement utilisées (concentration en phosphoramidite 0.1M et temps de condensation 25 sec) ont dû être optimalisées. En fait, le dérivé 4 se condense avec un rendement quasi quantitatif si on utilise une concentration 0.1M en phosphoramidite et un temps de condensation de 10 minutes.

On obtient dans chacun des quatre essais de condensation effectués un produit quasiment pur ainsi que le montrent les résultats d'analyse en électrophorèse capillaire (Fig. 1). Ces résultats montrent que les réactions de condensation, d'oxydation de capping et de clivage du support solide après synthèse sont efficaces et ne dégradent pas les trimères (NTT, STT), tetramères (NSTT) et pentamères (SNSTT) formés. Par ailleurs, en synthèse oligonucléotidique, il est possible de récupérer la solution de déprotection du groupe diméthoxytrityle et de mesurer l'intensité de la coloration orange du cation en vue d'estimer le rendement de chaque condensation. Ces mesures ont été faites pour les condensations de S et N, elles indiquent des rendements quasi quantitatifs.

La labilité du groupe protecteur de l'amine (groupe trifluoroacétyle) a également été étudiée. En fait, les conditions classiques de clivage d'une chaîne oligonucléotidique du support solide ($NH_4OH$ 32%, 1h. RT) et les conditions classiques de déprotection des phosphates et des amines des bases de la chaîne oligonucléotidique ($NH_4OH$ 32%, 8h, 55°C) se sont révélées suffisantes pour déprotéger l'amine primaire introduite dans les polymères 12, 14 et 15 par condensation d'un monomère 4.

Couplage en phase solide du dérivé 1 ou du dérivé 4 avec un dimère thymidine-thymidine fixé sur un support solide insoluble.

Le dérivé 1, ou le dérivé 4, solubilisé dans l'acétonitrile anhydre à une concentration de 0.1M est activé par le tétrazole 0.5M dans le même solvant et condensé avec le groupe hydroxylique en position 5' d'un dimère thymidine-thymidine fixé sur un support solide insoluble habituellement utilisé en synthèse oligonucléotidique dans un synthétiseur ABI 394. En ce qui concerne la condensation du composé 1, toutes les conditions sont identiques à celles utilisées dans un cycle d'élongation oligonucléotidique (N.D.Sinha, J.Biernat, J.McManus et H.Köster, Nucleic Acids Research (1984) 12(11), 4539).

En ce qui concerne la condensation du composé 4, toutes les conditions sont identiques à celles utilisées dans un cycle d'élongation oligonucléotidique, à l'exception du temps de couplage qui est porté à 10 minutes alors qu'il est de 25 secondes dans les conditions classiques de couplage.

Le clivage du support solide est réalisé dans les conditions habituelles ($NH_4OH$ 32%) et le produit final est obtenu avec un rendement comparable à ceux couramment réalisés en synthèse oligonucléotidique.

Les tri-, tetra- et pentamères synthétisés 12, 13, 14 et 15 ont été analysés en électrophorèse capillaire (cfr. fig. 1) ABI 270A, colonne capillaire MICRO-GEL100, diamètre interne 50µm, longueur 50cm, voltage 15kV, tampon 75mM Tris-phosphate 10% methanol pH 7.6.

EXEMPLE IV : Préparation de molécules mixtes oligodésoxyribonucléotidiques portant en 5'(OH) un enchevêtrement linéaire de bras chimiques entrecoupés de bras aminés utiles dans la détection de séquences d'ADN par des méthodes non radioactives.

La synthèse et l'utilisation de molécules mixtes composées d'une partie oligodésoxynucléotidique et d'une autre partie possédant des propriétés chimiques caractéristiques permettent la détection facile et rapide de cibles d'acides désoxyribonucléiques par des méthodes non radioactives. La partie oligodésoxyribonucléotidique de séquence définie et homologue d'un fragment d'ADN cible apporte l'énergie de stabilisation et assure son hybridation avec la molécule d'ADN que l'on veut détecter. La partie qui fournit la réactivité permettant la détection directe ou indirecte de l'hybride peut être introduite en l'extrémité 5'(OH) et (ou) 3'(OH) de la séquence d'acides nucléiques. Dans cet exemple précis, la partie responsable de la détection sera essentiellement introduite en 5'(OH) de la séquence d'acides nucléiques.

La partie responsable de la détection sera introduite en 5'(OH) de la chaîne oligonucléotidique encore fixée sur le support solide et protégée sur ses bases et ses phosphates en procédant à une série de condensations des dérivés 1 et 4 en utilisant les mêmes cycles de réaction dans le synthétiseur automatique d'ADN que ceux mis en oeuvre pour la synthèse de la séquence d'acides nucléiques. Toutefois en ce qui concerne le dérivé 4, le temps de condensation sera porté à 10 minutes au lieu des 25 secondes du cycle classique.

Après clivage de la chaîne oligonucléotidique 5' modifiée, de son support solide, et déprotection des bases et des phosphates, la partie qui fournit la réactivité responsable de la détection sera introduite par un lien chimique covalent. Parmi les différentes molécules possédant les propriétés requises pour la détection, on utilise de préférence la biotine, la digoxigénine, la fluorescéine, la peroxydase et l'alcaline phosphatase. Ces molécules, judicieusement activées, sont fixées sur la chaîne oligonucléotidique au niveau des amines primaires introduites à cet effet en raison de leur nucléophilicité.

Si l'on appelle N le bras aminé provenant de la condensation d'une unité 4 et S le bras chimique provenant de la condensation d'une unité 1, les oligomères modifiés en 5'(OH) qui ont été synthétisés sont les suivants :

NSSTTTTCAAAGAAGATGGCAAAACA 16

NSSNSSTTTTCAAAGAAGATGGCAAAACA 17

NSSNSSNSSTTTTCAAAGAAGATGGCAAAACA 18

NSSNSSNSSNSSTTTTCAAAGAAGATGGCAAAACA 19

Après clivage de la chaîne oligonucléotidique ainsi modifiée de son support solide et déprotection des bases et des phosphates, les sondes 16, 17, 18 et 19 sont biotinylées, pour conduire aux sondes biotinylées 20, 21, 22 et 23 qui sont analysées en électrophorèse capillaire (Fig. 2a, 2b).

Si l'on appelle B le bras aminé provenant de la condensation d'une unité 4 suivie d'une biotinylation, et S le bras chimique provenant de la condensation d'une unité 1, les sondes mono- ou polybiotinylées en 5'(OH) qui ont été obtenues sont les suivantes:

BSSTTTTCAAAGAAGATGGCAAAACA 20

BSSBSSTTTTCAAAGAAGATGGCAAAACA 21

BSSBSSBSSTTTTCAAAGAAGATGGCAAAACA 22

BSSBSSBSSBSSTTTTCAAAGAAGATGGCAAAACA 23

Fig. 2a, 2b.

Procédé de préparation de molécules mixtes oligodésoxyribonucléotidiques portant en 5'(OH) un enchevêtrement linéaire de bras chimiques entrecoupés de bras aminés 16, 17, 18 et 19

Une sonde ADN a été synthétisée avec une séquence oligodésoxynucléotidique complémentaire d'une séquence cible. On prépare, en phase solide, dans un synthétiseur automatique d'ADN ABI 394 simultanément quatre sondes identiques (dans quatre réacteurs) de séquence 5'TTTTCAAAGAAGATGGCAAAACA 3'.

Le synthétiseur est programmé pour continuer des cycles de fixation en 5' d'unités 1 et 4 pour conduire respectivement aux biopolymères 16, 17, 18 et 19. Pour ce faire, on a utilisé 6 mg de support CPG fonctionnalisé avec de la diméthoxytrityl benzoyl adénosine à 34 µmoles/g, ce qui correspond à 0.2 µmole. Le dérivé 1 et le dérivé 4 sont solubilisés dans l'acétonitrile anhydre à une concentration 0.1M et introduits dans le synthétiseur aux endroits prévus pour des phosphoramidites non classiques. Après synthèse automatique des biopolymères 16, 17, 18 et 19, les chaînes oligonucléotidiques ainsi modifiées sont clivées des supports CPG par quatre traitements successifs de 15 minutes avec 500 µl de $NH_4OH$ 32%. Les solutions ammoniacales obtenues sont portées 5 h à 55°C. Ce deuxième traitement a pour but de déprotéger les bases et les phosphates des chaînes oligonucléotidiques. Les solutions ammoniacales sont liophylisées, les quatre résidus sont soumis à une chromatographie par filtration moléculaire (gel sephadex G50) puis à une électrophorèse sur gel de polyacrylamide 20%.

Procédé de biotinylation de molécules mixtes oligodésoxyribonucléotidiques portant en 5'(OH) un enchevêtrement linéaire de bras chimiques entrecoupés de bras aminés. Préparation des sondes biotinylées 20, 21, 22, 23

Dans un tube Eppendorf, on dissout 20DO de sonde aminée 16, 17, 18 ou 19 dans 120 µl de tampon phosphate 0.01M pH 7.5. On ajoute une solution de 20 mg de sulfosuccinimidyl 6-biotinamidocaproate dans 240 µl de diméthylformamide. On laisse incuber à température ambiante 16h. Chaque solution est soumise à une chromatographie par filtration moléculaire (gel sephadex G50), puis à une électrophorèse sur gel de polyacrylamide 20%. Les sondes biotinylées 20, 21, 22 et 23 ont été analysées en électrophorèse capillaire Micro-$Gel_{100}$ diamètre interne 50 µm, longueur 50 cm, voltage 15kV, tampon 75 mM Tris-phosphate - 10% methanol pH 7,6.

EXEMPLE V : Préparation d'un support solide CPG (controlled porous glass) fonctionnalisé par le 1-O-(4,4' diméthoxytrityl) 1,3-butanediol

Le support CPG fonctionnalisé par le 1-O-(4,4' diméthoxytrityl) 1,3butanediol 3 a pour formule 24

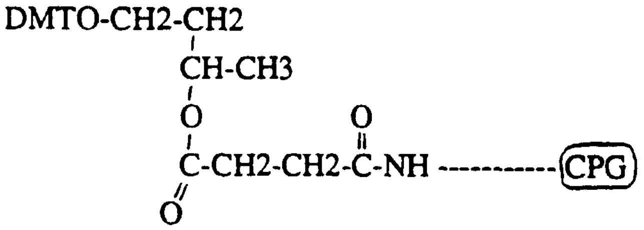

24

L'approche suivie a pour objectif la préparation d'un support solide judicieusement fonctionnalisé en vue de la préparation de sondes d'acides nucléiques dans lesquelles un ou plusieurs éléments de marquage entrecoupés de bras chimiques sont introduits en 3'(OH) de la sonde par toute méthode de synthèse d'assemblage internucléotidique manuelle ou automatique sur support solide.

La méthode adoptée pour fonctionnaliser le support CPG comporte les étapes suivantes (schéma III).

Schéma III

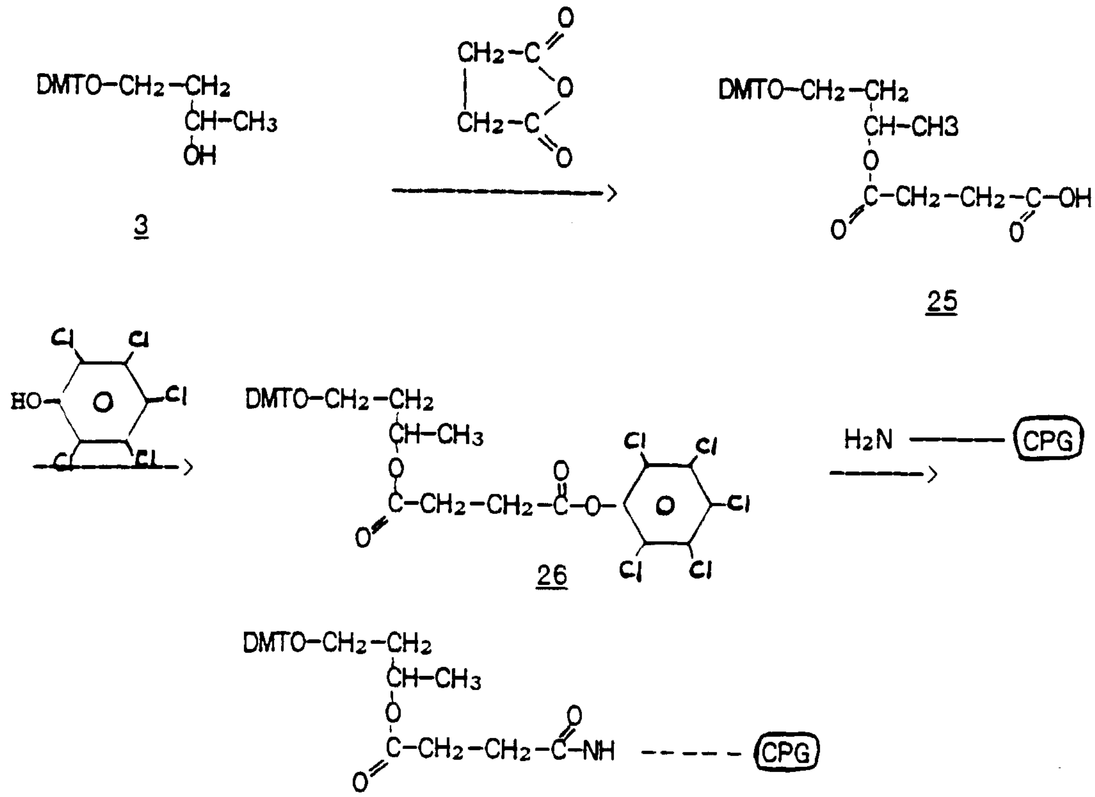

3

25

26

24

Le 1-O-(4,4' diméthoxytrityl)1,3-butanediol 3 est acylé sur l'alcool secondaire libre par l'anhydride succinique puis l'acide généré 25 est activé sous forme d'ester pentachlorophénolique 26. Le support CPG porte en surface des fonctions "amine primaire" qui fixeront le dérivé 26 par formation d'un lien amide à partir de l'ester activé.

Procédé de préparation de 1-O-(4,4' diméthoxytrityl) 3-succinate 1,3-butanediol 25

Dans un ballon bicol de 50cc, on met sous argon 2,35 g (6 mmoles) de composé 3, on ajoute 22cc de pyridine anhydre, 0,74g (6.1 mmoles) de 4diméthylaminopyridine et 0.64g (6.4 mmoles) d'anhydride succinique. On agite magnétiquement le mélange réactionnel pendant 3 jours à température ambiante. On ajoute alors du toluène au mélange réactionnel et on procède à un entraînement azéotropique de la pyridine par le toluène (évaporateur rotatif p=20mm Hg). Le résidu obtenu est dissous dans 75cc de dichlorométhane. La phase organique ainsi obtenue est lavée deux

fois avec une solution d'acide citrique $4x10^{-2}$ M. La phase organique est alors séchée sur $MgSO_4$, filtrée et évaporée (évaporateur rotatif p=20mm Hg). Le résidu est purifié sur colonne de silice Merck 9385 - éluant: gradient Hexane 15 - $CH_2Cl_2$ 85 ⇒ $CH_2Cl_2$ 100 ⇒ MeOH3-$CH_2Cl_2$ 97. La silice a préalablement été neutralisée par mise en suspension dans l'éluant de départ contenant 1% de DIEA. On obtient après purification 1 g de composé 25. Rdt: 34%.

Procédé de préparation du 1-O-(4,4' diméthoxytrityl) 3-(pentachlorophenoxysuccinate) 1,3-butanediol 26

Dans un ballon bicol de 25cc, on introduit 1g de composé 25 (2mmoles), on met sous atmosphère d'argon et on ajoute une solution de 14cc de diméthylformamide anhydre contenant 0.58g de pentachlorophénol (2.2 mmoles) et 0.62g de dicyclohexylcarbodiimide (3 mmoles). On agite le mélange réactionnel magnétiquement pendant 24h à température ambiante. On filtre le précipité de dicyclohexylurée puis le précipité est lavé avec 50cc de benzène. On évapore le benzène.

Le résidu est purifié sur colonne de silice Merck 9385 - éluant hexane 15 - dichlorométhane 85. La silice est préalablement neutralisée par mise en suspension dans l'éluant contenant 1% de DIEA. Après purification, on obtient 1.7 g de composé 26. Rdt = ± 100%.

Procédé de préparation d'un support solide CPG (Controlled Porous Glass) fonctionnalisé par des groupements 1-O-(4,4' diméthoxytrityl) 1,3-butanediol 24

Le support solide utilisé (PIERCE Ref.24875) a une porosité de 500Å, il est fonctionnalisé en surface par des groupes "amine primaire". Dans un ballon de 10cc. on introduit 0.5 g de support CPG aminé, 0.2 cc de triéthylamine fraîchement distillée, 0.385 g d'ester pentachlorophénolique 26 (0.26 mmole) et 1.3 cc de diméthylformamide anhydre. On ferme le ballon et on agite à 37°C pendant 24h. A ce moment, on contrôle sur un petit aliquot de support solide qu'il a effectivement été fonctionnalisé par 26. L'aliquot est lavé plusieurs fois à l'éthanol puis à l'éther, puis il est traité avec une solution 0.1M d'acide p toluène sulfonique dans l'acétonitrile anhydre. Cette opération a pour but de déprotéger l'alcool primaire et de faire apparaître la coloration orange du cation diméthoxytrityl formé dans ce milieu anhydre. Si donc la coloration orange apparaît, on peut poursuivre le traitement du support ainsi fonctionnalisé.

On procède alors à la neutralisation des fonctions amine primaire du support n'ayant pas réagi. Pour cela, on ajoute au mélange réactionnel 70µl d'anhydride acétique et on laisse agir 10 minutes à 37°C. On filtre ensuite le support solide. Le support est ensuite successivement lavé avec 20cc de diméthylformamide, 20cc d'alcool éthylique. 20cc de dioxanne et 20cc d'éther éthylique. Il est ensuite séché sous vide au dessicateur (p= 20mm Hg) en présence de pentoxyde de phosphore. On procède alors à une détermination du nombre de molécules 26 fixées sur le support par dosage spectroscopique (visible) du nombre de groupes 4,4'-diméthoxytrityle libérés en milieu acide par gramme de support.

Dans un flacon jaugé de 10cc, on introduit 8.1 mg de support CPG fonctionnalisé, on ajoute 10 ml d'acide p toluène sulfonique 0.1 M dans l'acétonitrile anhydre. La solution se colore en orange, la lecture se fait à 497 nm. $\varepsilon = 7.10^4$. On mesure une absorbance de 1.72.

$$C = \frac{A}{\varepsilon 1} = \frac{1.72}{7.10^4 x1} = 0.245\ 10^{-4} \text{ mole/l}$$

La fonctionnalisation du support est donc de 30.3 µmole/g.

EXEMPLE VI : Etude de la réactivité d'un support CPG fonctionnalisé par des unités 1-O-(4,4' diméthoxytrityl) 1,3-butanediol

Le support CPG 24 fonctionnalisé par des unités 1-O-(4,4' diméthoxytrityl) 1,3-butanediol peut être utilisé pour introduire en 3'(OH) d'un oligonucléotide

- des bras chimiques (condensation du dérivé 1).
- des bras "amine primaire" (condensation du dérivé 4).

Les bras "amine primaire" sont séparés les uns des autres et séparés de la chaîne oligonucléotidique par des bras chimiques.

Dans le but de réaliser de tels édifices biopolymériques, la réactivité du support CPG 24 a été testée dans les conditions de synthèse automatique d'oligonucléotides. A cet effet, le support CPG 24, introduit dans un réacteur de l'appareil de synthèse automatique d'oligonucléotides, a été soumis à une série de condensations respectivement des

phosphoramidites 1, 4 et celui de la thymidine (T).

Si l'on appelle N le bras aminé provenant de la condensation d'une unité 4 et S le bras chimique provenant de la condensation d'une unité 1, les polymères qui ont été synthétisés sont les suivants :

TTSS 27
TTNS 28
SSTT 29
NSTTSNS 30

Le tetramère SSTT a uniquement été synthétisé pour une comparaison en électrophorèse capillaire avec TTSS. Les phosphoramidites 1 et 4 se fixent donc avec succès sur le support CPG 24 dans les conditions classiques (temps de couplage, solvant, réactifs) de condensation dans un synthétiseur automatique d'oligonucléotides. Toutefois, en ce qui concerne la condensation du composé 4, le temps de couplage a dû être porté à 10 minutes au lieu des 25 secondes du cycle classique. De plus, les conditions classiques utilisées pour le clivage des oligonucléotides de leur support solide ($NH_4OH$ 32% - 4x(500 µl-15 minutes) se sont avérées adéquates puisque les composés 27, 28 et 30 ont été obtenus avec de bons rendements.

On obtient dans chacun de ces essais de condensation effectués, un produit relativement pur ainsi que le montrent les résultats d'analyse en électrophorèse capillaire (Fig. 3).

Par ailleurs, en synthèse oligonucléotidique, il est possible de récupérer la solution de déprotection du groupe diméthoxytrityle et de mesurer l'intensité de la coloration orange du cation en vue d'estimer le rendement de chaque condensation. Ces mesures ont été faites pour chaque condensation sur le support 24, elles indiquent des rendements quasi quantitatifs.

Condensation des phosphoramidites 1 et 4 sur le support CPG 24. Synthèse des polymères 27, 28 et 30.

On réalise simultanément les trois polymères 27, 28 et 30 dans un synthétiseur automatique d'oligonucléotides ABI 394. Pour ce faire, on introduit dans trois réacteurs 6,6 mgr (0.2 µmole) de support CPG 24 fonctionnalisé à 30,3 µmole/g. Le dérivé 1 et le dérivé 4 sont solubilisés dans l'acétonitrile anhydre à une concentration 0.1M et introduits dans le synthétiseur aux endroits prévus pour des phosphoramidites non classiques. Le synthétiseur est programmé pour effectuer des cycles de condensation de 1, 4 et T selon les séquences de 27, 28 et 30. Après synthèse automatique de 27, 28 et 30, les polymères sont clivés des supports CPG par quatre traitements successifs de 15 minutes avec 500 µl de $NH_4OH$ 32%. Les solutions ammoniacales sont liophylisées et les résidus sont analysés en électrophorèse capillaire (ABI 270A), colonne Micro-$Gel_{100}$ diamètre interne 50 µm, longueur 50 cm, voltage 15kV, tampon 75 mM Tris-phosphate - 10% méthanol pH 7.6.

EXEMPLE VII : Préparation de molécules mixtes oligodésoxyribonucléotidiques portant en 3'(OH) un enchevêtrement linéaire de bras chimiques entrecoupés de bras aminés utiles dans la détection de séquence d'ADN par des méthodes non-radioactives.

Les molécules mixtes composées d'une partie oligodésoxyribonucléotidique et d'une autre partie possédant des propriétés chimiques caractéristiques permettent la détection facile et rapide de cibles d'acides désoxyribonucléiques par des méthodes non radioactives. La partie oligodésoxyribonucléotidique de séquence définie et homologue d'un fragment d'ADN cible apporte l'énergie de stabilisation et assure son hybridation avec la molécule d'ADN que l'on veut détecter. La partie qui fournit la réactivité permettant la détection directe ou indirecte de l'hybride peut être introduite en l'extrémité 5'(OH) et (ou) 3'(OH) de la séquence d'acides nucléiques. Dans cet exemple précis, la partie responsable de la détection sera essentiellement introduite en 3'(OH) de la séquence d'acides nucléiques. La partie responsable de la détection sera introduite en 3'(OH) de la chaîne oligonucléotidique en procédant d'abord à une série de condensations des dérivés 1 et 4 sur un support solide CPG 24 dans le synthétiseur automatique d'oligonucléotides. Lorsque la séquence souhaitée de bras chimiques et amine primaire a été édifiée dans le synthétiseur automatique, on procède, à la suite de l'édifice bâti, à la construction de la chaîne oligonucléotidique. Les condensations des phosphoramidites 1 et 4 dans le synthétiseur automatique d'oligonucléotides sont réalisées en utilisant les mêmes cycles de réactions que ceux mis en oeuvre pour la synthèse de la séquence d'acides nucléiques. Toutefois, en ce qui concerne le dérivé 4, le temps de condensation est porté à 10 minutes au lieu des 25 secondes du cycle classique. Après clivage de la chaîne oligonucléotidique 3' modifiée de son support solide et déprotection des phosphates et des bases de la chaîne oligonucléotidique, la partie qui fournit la réactivité responsable de la détection sera introduite par un lien chimique covalent. Parmi les différentes molécules possédant les propriétés requises pour la détection, on utilise de préférence la biotine, la digoxigénine, la fluorescéine, la peroxydase et l'alcaline phosphatase. Ces molécules, judicieusement activées, sont fixées sur la chaîne oligonucléotidique au niveau des amines primaires introduites à cet effet en raison

de leur nucléophilicité.

Si l'on appelle N le bras aminé provenant de la condensation d'une unité 4 et S le bras chimique provenant de la condensation d'une unité 1, les oligomères modifiés en 3'(OH) qui ont été synthétisés sont les suivants :

TTTTCAAAGAAGATGGCAAAACASSNS 31

TTTTCAAAGAAGATGGCAAAACASSNSSNS 32

TTTTCAAAGAAGATGGCAAAACASSNSSNSSNS 33

TTTTCAAAGAAGATGGCAAAACASSNSSNSSNSSNS 34

Après clivage de la chaîne oligonucléotidique ainsi modifiée, de son support solide et déprotection des bases et des phosphates, les quatre oligonucléotides sont analysés en électrophorèse capillaire (Fig. 4).

Après déprotection des amines primaires, les sondes 31, 32, 33 et 34 sont biotinylées. Les sondes biotinylées obtenues 35, 36, 37 et 38 sont analysées en électrophorèse capillaire (Fig. 4).

Si l'on appelle B le bras aminé provenant de la condensation d'une unité 4 suivie d'une biotinylation et S le bras chimique provenant de la condensation d'une unité 1, les sondes mono ou polybiotinylées en 3'(OH) qui ont été obtenues sont les suivantes:

TTTTCAAAGAAGATGGCAAAACASSBS 35

TTTTCAAAGAAGATGGCAAAACASSBSSBS 36

TTTTCAAAGAAGATGGCAAAACASSBSSBSSBS 37

TTTTCAAAGAAGATGGCAAAACASSBSSBSSBSSBS 38

Procédé de préparation de molécules mixtes oligodésoxyribonucléotidiques portant en 3'(OH) un enchevêtrement linéaire de bras chimiques entrecoupés de bras aminés. Synthèse des sondes 31, 32, 33 et 34.

On réalise simultanément les quatre sondes 31, 32, 33 et 34 dans un synthétiseur automatique d'ADN ABI 394. Pour ce faire, on introduit dans quatre réacteurs 6,6 mg (0,2 μmole) de support CPG 24 fonctionnalisé à 30.3 μmole/gr. Le dérivé 1 et le dérivé 4 sont solubilisés dans l'acétonitrile anhydre à une concentration 0.1 M et introduits dans le synthétiseur aux endroits prévus pour des phosphoramidites non classiques. Le synthétiseur est programmé pour débuter par des cycles de fixation des phosphoramidites 1 et 4 selon les séquences respectives 31, 32, 33 et 34, puis poursuivre avec les phosphoramidites nucléotidiques, la synthèse d'une séquence d'acides nucléiques complémentaire d'une séquence cible :

5' TTTTCAAAGAAGATGGCAAAACA 3'

Après synthèse automatique des biopolymères 31, 32, 33 et 34, les chaînes oligonucléotidiques ainsi modifiées sont clivées des supports CPG par quatre traitements successifs de 15 minutes avec 500 μl de $NH_4OH$ 32%. Les solutions ammoniacales obtenues sont portées 5 heures à 55°C. Ce deuxième traitement a pour but de déprotéger les bases et les phosphates des chaînes oligonucléotidiques. Les solutions ammoniacales sont liophylisées, les quatre résidus sont soumis à une chromatographie par filtration moléculaire (gel Sephadex G50) puis à une électrophorèse sur gel de polyacrylamide 20%.

Procédé de biotinylation de molécules mixtes oligodésoxyribonucléotidiques portant en 3'(OH) un enchevêtrement linéaire de bras chimiques entrecoupés de bras aminés. Préparation des sondes biotinylées 35, 36, 37 et 38.

Dans un tube eppendorf, on dissout 20 DO de sonde aminée 31, 32, 33 ou 34 dans 120 µl de tampon phosphate 0.01 M pH 7.5. On ajoute une solution de 20 mg de sulfosuccinimidyl 6-biotinamido caproate dans 240 µl de diméthylformamide. On laisse incuber à température ambiante 16 heures. Chaque solution est alors soumise à une chromatographie par filtration moléculaire (gel Sephadex G50) puis à une électrophorèse sur gel de polyacrylamide 20%. Les sondes biotinylées 35, 36, 37 et 38 ainsi que les sondes aminées correspondantes 31, 32, 33 et 34 sont analysées en électrophorèse capillaire colonne Micro-Gel$_{100}$, diamètre interne 50 µm, longueur 50cm, voltage 15kV, tampon 75mM Tris-phosphate -10% méthanol pH 7.6.

EXEMPLE VIII : Préparation de molécules mixtes oligodésoxyribonucléotidiques portant en 3'(OH) et en 5'(OH) un enchevêtrement linéaire de bras chimiques entrecoupés de bras aminés utiles dans la détection de séquences d'ADN par des méthodes non-radioactives.

Les molécules mixtes composées d'une partie oligodésoxyribonucléotidique et d'une autre partie possédant des propriétés chimiques caractéristiques permettent la détection facile et rapide de cibles d'acides désoxyribonucléiques par des méthodes non-radioactives. La partie oligodésoxyribonucléotidique de séquence définie et homologue d'un fragment d'ADN cible apporte l'énergie de stabilisation et assure son hybridation avec la molécule d'ADN que l'on veut détecter. La partie qui fournit la réactivité permettant la détection directe ou indirecte de l'hybride peut être introduite en l'extrémité 5'(OH) et (ou) 3'(OH) de la séquence d'acides nucléiques. Dans cet exemple précis, la partie responsable de la détection sera introduite de part et d'autre de la chaîne oligonucléotidique simultanément en 5'(OH) et en 3'(OH).

La partie responsable de la détection sera introduite en 3'(OH) de la chaîne oligonucléotidique en procédant d'abord à une série de condensations des dérivés 1 et 4 sur un support solide CPG 24, dans le synthétiseur automatique d'oligonucléotides. Lorsque la séquence souhaitée de bras chimiques et amine primaire a été édifiée dans le synthétiseur, on procède, à la suite de l'édifice construit, à la construction de la chaîne oligonucléotidique.

A la suite de la chaîne oligonucléotidique, le synthétiseur est programmé pour continuer des cycles de fixation en 5' d'unités 1 et 4. Les condensations des phosphoramidites 1 et 4 dans le synthétiseur automatique d'oligonucléotides sont réalisées en utilisant les mêmes cycles de réactions que ceux mis en oeuvre pour la synthèse de la séquence d'acides nucléiques. Toutefois, en ce qui concerne le composé 4, le temps de condensation est porté à 10 minutes au lieu des 25 secondes du cycle classique.

Après clivage de son support solide de la chaîne oligonucléotidique 3'(OH) et 5'(OH) modifiée et déprotection des phosphates et des bases de la chaîne oligonucléotidique, la partie qui fournit la réactivité responsable de la détection sera introduite par un lien chimique covalent. Parmi les différentes molécules possédant les propriétés requises pour la détection, on utilise de préférence la biotine, la digoxigénine, la fluorescéine, la peroxydase et l'alcaline phosphatase. Ces molécules, judicieusement activées, sont fixées sur la chaîne oligonucléotidique au niveau des amines primaires introduites à cet effet en raison de leur nucléophilicité.

Si l'on appelle N le bras aminé provenant de la condensation d'une unité 4 et S le bras chimique provenant de la condensation d'une unité 1, les oligomères modifiés en 3'(OH) et 5'(OH) qui ont été synthétisés sont les suivants :

NSSTTTTCAAAGAAGATGGCAAAACASSNS 39

NSSNSSTTTTCAAAGAAGATGGCAAAACASSNSSNS 40

NSSNSSNSSTTTTCAAAGAAGATGGCAAAACASSNSSNSSNS 41

NSSNSSNSSNSSTTTTCAAAGAAGATGGCAAAACASSNSSNSSNSSNS 42

Après clivage de la chaîne oligonucléotidique ainsi modifiée de son support solide et déprotection des bases et des phosphates, les quatre oligonucléotides sont analysés en électrophorèse capillaire (Fig. 5).

Les sondes 39, 40, 41 et 42 sont alors biotinylées; les sondes biotinylées obtenues 43, 44, 45 et 46 sont analysées en électrophorèse capillaire (Fig. 5).

Si l'on appelle B le bras aminé provenant de la condensation d'une unité 4 suivie d'une biotinylation et S le bras chimique provenant de la condensation d'une unité 1, les sondes biotinylées en 3'(OH) et en 5'(OH) qui ont été obtenues

sont les suivantes :

BSSTTTTCAAAGAAGATGGCAAAACASSBS 43

BSSBSSTTTTCAAAGAAGATGGCAAAACASSBSSBS 44

BSSBSSBSSTTTTCAAAGAAGATGGCAAAACASSBSSBSSBS 45

BSSBSSBSSBSSTTTTCAAAGAAGATGGCAAAACASSBSSBSSBSSBS 46

Procédé de préparation de molécules mixtes oligodésoxyribonucléotidiques portant en 3'(OH) et en 5'(OH) un enchevêtrement linéaire de bras chimiques entrecoupés de bras aminés. Synthèse des sondes 39, 40, 41 et 42.

On réalise simultanément les quatre sondes 39, 40, 41 et 42 dans un synthétiseur automatique d'ADN ABI 394. Pour ce faire, on introduit dans quatre réacteurs 6,6 mg (0.2 µmole) de support CPG 24 fonctionnalisé à 30.3 µmole/g. Le dérivé 1 et le dérivé 4 sont solubilisés dans l'acétonitrile anhydre à une concentration 0.1 M et introduits dans le synthétiseur aux endroits prévus pour des phosphoramidites non classiques.

Le synthétiseur est programmé pour débuter par des cycles de fixation des phosphoramidites 1 et 4 selon les séquences respectives 39, 40, 41 et 42, puis poursuivre avec les phosphoramidites nucléotidiques, la synthèse d'une séquence d'acides nucléiques complémentaire d'une séquence cible :

5' TTTTCAAAGAAGATGGCAAAACA 3'

et enfin continuer des cycles de condensation d'unités 1 et 4 en 5' de la chaîne d'acides nucléiques.

Après synthèse automatique des biopolymères 39, 40, 41 et 42, les chaînes oligonucléotidiques ainsi modifiées sont clivées des supports CPG par quatre traitements successifs de 15 minutes avec 500 µl de $NH_4OH$ 32%. Les solutions ammoniacales obtenues sont portées 5 heures à 55°C. Ce deuxième traitement a pour but de déprotéger les bases et les phosphates des chaînes oligonucléotidiques. Les solutions ammoniacales sont liophylisées; les quatre résidus sont soumis à une chromatographie par filtration moléculaire (gel Sephadex G50) puis à une électrophorèse sur gel de polyacrylamide à 20%.

Procédé de biotinylation de molécules mixtes oligodésoxyribonucléotidiques portant en 3'(OH) et en 5'(OH) un enchevêtrement linéaire de bras chimiques entrecoupé de bras aminés. Préparation des sondes biotinylées 43, 44, 45 et 46

Dans un tube eppendorf, on dissout 20 DO de sonde 39, 40, 41 ou 42 dans 120 µl de tampon phosphate 0.01 M pH 7.5. On ajoute une solution de 30 mg de sulfosuccinimidyl 6-biotinamido caproate dans 360 µl de diméthylformamide. On laisse incuber à température ambiante pendant 16 heures. Chaque solution est alors soumise à une chromatographie par filtration moléculaire (gel Sephadex G50) puis à une électrophorèse sur gel de polyacrylamide 20%. Les sondes biotinylées 43, 44, 45 et 46 ainsi que les sondes aminées correspondantes 39, 40, 41 et 42 sont analysées en électrophorèse capillaire colonne Micro-$Gel_{100}$, diamètre interne 50 µm, longueur 50cm, voltage 15kV, tampon 75 mM Tris-phosphate - 10% méthanol pH 7.6

EXEMPLE IX : Utilisation des sondes biotinylées décrites dans les exemples IV, VII et VIII en hybridation sur support solide - limites de détection.

Afin de mettre en évidence les avantages de la présente invention, les sondes polybiotinylées en 5'(OH) 20, 21, 22 et 23, les sondes polybiotinylées en 3'(OH) 35, 36, 37 et 38, ainsi que les sondes polybiotinylées en 3'(OH) et en 5'(OH) 43, 44, 45 et 46 ont été testées en hybridation en vue d'étudier leur potentiel d'utilisation en diagnostic.

De manière générale, l'hybridation s'est faite entre un oligonucléotide (30 mer) fixé sur un support solide en plastique par son extrémité 5'(OH) et complémentaire des sondes à tester (23 mer) par son extrémité 3' (OH). Chaque support solide (tube) revêt de l'ordre de 10 ng d'oligonucléotide (30 mer) complémentaire aux sondes à tester, ce qui représente environ $10^{12}$ copies. Chaque oligonucléotide biotinylé mentionné ci-dessus est soumis à 8 hybridations

différentes partant de 1 pg d'oligonucléotide marqué (~ $10^8$ copies) et allant jusqu'à 300 atg ($3x10^4$ copies) en procédant à des dilutions 1/3 d'une cuvette à l'autre. Après hybridation, la révélation se fait par chémiluminescence (CSPD-Tropix); la lecture se fait au luminomètre. Les résultats repris au schéma IV concernent l'hybridation des sondes polybiotinylées en 5'(OH) 20, 21, 22 et 23 avec le 30 mer complémentaire fixé sur tube en plastique.

| Schéma IV | | | |
|---|---|---|---|
| OLIGO | Nombre de Biotines | Limite de détection | |
| | | fg | nombre de copies |
| 20 | 1 5'(OH) | 30 | $3 \times 10^6$ |
| 21 | 2 " | 30 | $3 \times 10^6$ |
| 22 | 3 " | 30 | $3 \times 10^6$ |
| 23 | 4 " | 3 | $3 \times 10^5$ |

Pour les sondes polybiotinylées en 3'(OH) 35, 36, 37 et 38, les résultats d'hybridation sont repris au schéma V.

| Schéma V | | | |
|---|---|---|---|
| OLIGO | Nombre de Biotines | Limite de détection | |
| | | fg | nombre de copies |
| 35 | 1 3' (OH) | 30 | $3 \times 10^6$ |
| 36 | 2 " | 10 | $10^6$ |
| 37 | 3 " | 3 | $3 \times 10^5$ |
| 38 | 4 " | 3 | $3 \times 10^5$ |

En ce qui concerne les sondes polybiotinylées en 5'(OH) et en 3'(OH) 43, 44, 45 et 46, les résultats sont repris au schéma VI.

| Schéma VI | | | |
|---|---|---|---|
| OLIGO | Nombre de Biotines | Limite de détection | |
| | | fg | nombre de copies |
| 43 | 2 3'+5'(OH) | 30 | $3 \times 10^6$ |
| 44 | 4 " | 10 | $10^6$ |
| 45 | 6 " | 3 | $3 \times 10^5$ |
| 46 | 8 " | 1 | $10^5$ |

Procédé d'hybridation d'une sonde mono ou polybiotinylée (23 mer) avec un oligomer complémentaire (30 mer) fixé sur un tube en plastique.

Les tubes, recouverts de l'oligonucléotide complémentaire (30 mer) sont d'abord saturés une heure à 37°C avec 200 µl d'une solution à 5% de lait écrémé liophylisé dans le TBS 1x contenant une trace d'azide. Les tubes sont ensuite préhybridés 2 heures à 50°C avec le tampon d'hybridation (100 µl): 5x Denhardt's, 6xSSC, 0.1% SDS, 100 µg/ml de DNA de sperme de saumon.

Pour chaque oligonucléotide à tester, on réalise 8 essais avec les quantités suivantes (dans 100 µl de tampon d'hybridation): 1 pg ($10^8$ copies), 300 fg, 100 fg ($10^7$ copies), 30 fg, 10 fg ($10^6$ copies), 3 fg, 1 fg ($10^5$ copies), 0.3 fg. On réalise également une série de huit tubes avec un oligomère non complémentaire et non biotinylé et huit tubes sans oligonucléotide.

L'ensemble des tubes est mis en hybridation 2 h à 50°C, puis chaque tube est lavé trois fois pendant dix minutes à 50°C avec une solution SSC 6x, ils sont ensuite lavés une fois pendant cinq minutes avec le tampon I.

Tampon I: O.1 M Tris, 2 mM $MgCl_2$, 0.05% triton, 1 M NaCl.

On prépare alors une solution de streptavidine-phosphatase alcaline (fournie avec le kit Tropix): 1 µl de la solution du kit dans 5.500 µl de tampon I. On met 100 µl de cette solution dans chaque tube, on laisse incuber trente minutes à température ambiante, on lave ensuite chaque tube six fois avec le tampon I. On prépare la solution d'agent chemiluminescent, le CSPD: (3-(4-méthoxyspiro[1,2-dioxétane-3,2'-tricyclo[3.3.1.1.]chlorodecane]-4-yl) phenyl phosphate.

La solution est préparée comme suit : 80 µl de CSPD (Tropix), 500 µl d' "emerald" (Tropix) et 4.420 µl de tampon de révélation.

Tampon de révélation: 0.1 M diéthanolamine, 1 mM $MgCl_2$, 0.02% azide de sodium. On dépose 100 µl de cette solution dans chaque tube, on laisse incuber 45' puis on lit au luminomètre.

EXEMPLE X : Préparation du 1,6-O-(4,4' diméthoxytrityl)2-0-(N,N'diisopropylamino 2-cyanoéthoxyphosphino) 1, 2, 6-hexane triol.

Le dérivé 1,6-O-(4,4' diméthoxytrityl) 2-O-(N,N' diisopropylamino 2-cyanoéthoxyphosphino) 1,2,6-hexane triol 47 répond à la formule:

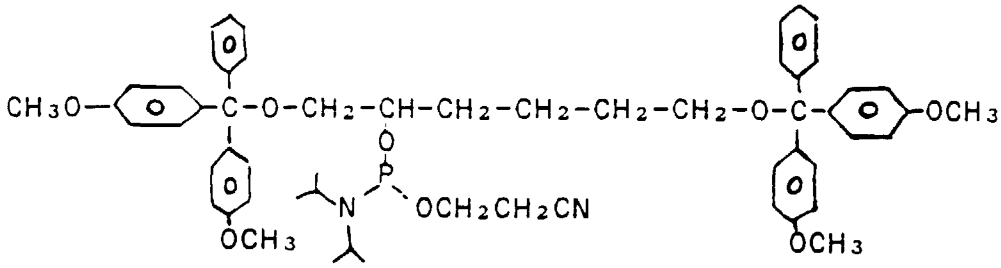

47

L'approche suivie a pour objectif la préparation d'un synthon non nucléotidique portant cependant les groupes chimiques qui permettent son introduction en 5'(OH) d'une chaîne oligonucléotidique ou dans un polymère non nucléotidique dont les monomères sont unis par un lien phosphodiester, dans les conditions de synthèse automatique d'ARN ou d'ADN habituelles.

Ce synthon a l'avantage de fournir à l'utilisateur un outil permettant l'introduction de chaînes ramifiées, en 5'(OH) d'une séquence d'acides nucléiques, sans sortir de la routine de synthèse automatique ou manuelle.

En effet, il possède deux fonctions alcool primaire protégées par un groupe diméthoxytrityle et donc, à chaque cycle de condensation, il double le nombre de sites pour la condensation suivante.

L'intérêt de multiplier le nombre de sites de condensation réside dans la possibilité de pouvoir introduire sur ces sites en fin de synthèse, des chaînes alkyle portant une amine primaire. Cette introduction est possible par condensation, en fin de synthèse, du dérivé 48 sur les n sites alcool primaire créés.

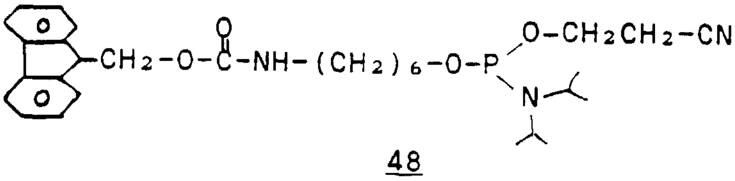

48

Le dérivé 48 est connu dans la littérature et n'est donc pas revendiqué ni décrit.

En ce qui concerne le composé 47, les groupes 4,4'-diméthoxytrityle et 2-cyanoéthoxydiisopropylaminophosphoramidite sont appropriés à une synthèse aux phosphoramidites notamment sur support solide.

Le chemin réactionnel pour l'obtention du composé 47 présenté dans le schéma VII comporte les étapes suivantes :

1) protection sélective des deux alcools primaires du 1,2,6-hexane triol 49 par le chlorure de diméthoxytrityle (DMT) labile à pH acide.

2) phosphorylation de l'alcool secondaire 50. L'exemple du phosphoramidite montré dans le schéma VII n'est pas limitatif; on peut envisager aussi la synthèse d'un phosphate triester ou diester ou d'un phosphonate.

Schéma VII

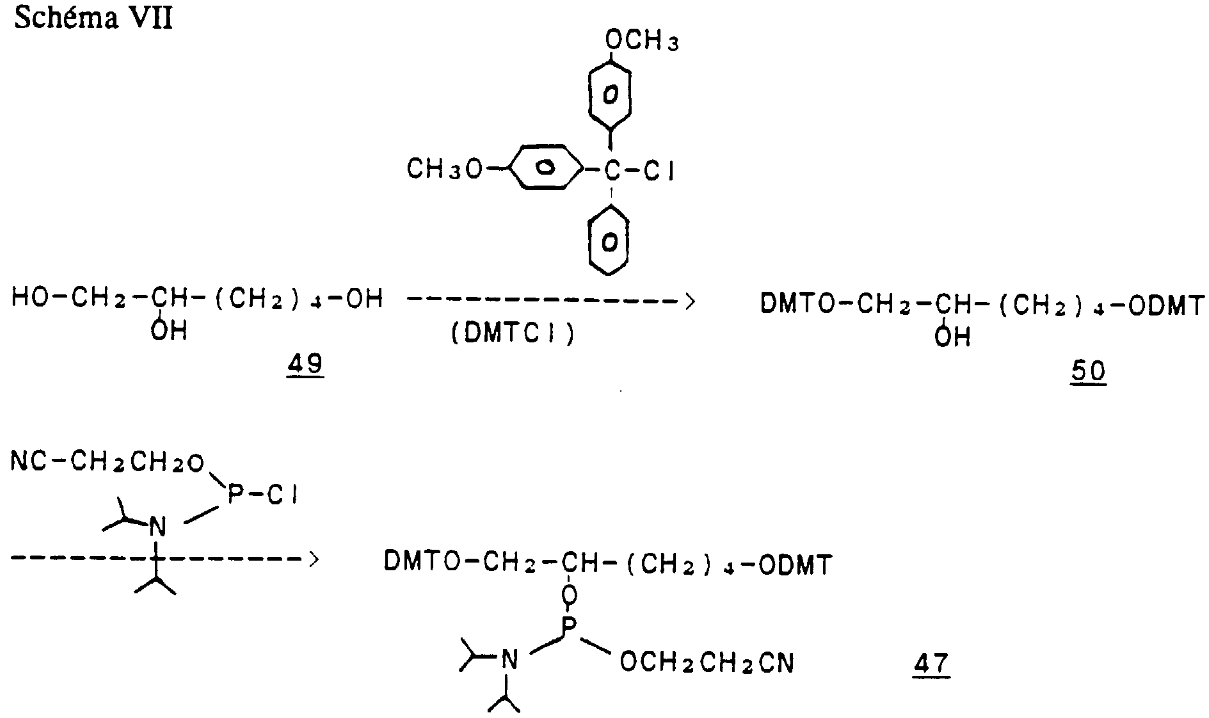


49 50 47

<u>Procédé de préparation du 1,6-O-(4,4'-diméthoxytrityl) 1,2,6-hexanetriol 50</u>

Dans un ballon bicol de 50cc, on introduit 1.34 g de 1,2,6-hexane triol <u>49</u> ($10^{-2}$ mole). On le met sous atmosphère d'argon puis on ajoute 20cc de pyridine anhydre. On ajoute ensuite à la solution 8.45 g (2.5 x $10^{-2}$ moles) de chlorure de diméthoxytrityle. On agite 2 heures à température ambiante. On hydrolyse le mélange réactionnel (25cc $NaHCO_3$ 5%) et on extrait au dichlorométhane (2 x 50cc). La phase organique obtenue est lavée trois fois à l'eau puis une fois avec une solution aqueuse saturée en chlorure de sodium, elle est séchée sur $MgSO_4$ puis filtrée et évaporée sous pression réduite (évaporateur rotatif p = 20mm Hg). La pyridine résiduelle est ensuite éliminée par entraînement azéotropique sous pression réduite avec du toluène. Le toluène résiduel est alors éliminé par entraînement azéotropique sous pression réduite avec du dichlorométhane. Le résidu obtenu est purifié sur colonne de silice Merck 9385 - éluant éther 0⇒40 - hexane 100⇒60. La silice est préalablement neutralisée par mise en suspension dans l'éluant initial contenant 1% DIEA. Après purification, on récupère 4.5 g (6.1 x $10^{-3}$ moles) Rdt. 61%. Chromatographie sur couche mince (CCM) - plaque Merck 5735 (gel de silice $60F_{254}$) éluant éther 4 - hexane 6 RF: 0.3.

<u>Procédé de préparation du 1,6-O-(4,4' diméthoxytrityl 2-O-(N,N'-diisopropylamino-2-cyanoéthoxyphosphino) 1,2,6 hexane triol 47</u>

Dans un ballon bicol de 25cc, on introduit 1.1 g de composé <u>50</u> (1.5 x $10^{-3}$ moles). On met sous atmosphère d'argon puis on ajoute 6cc de tétrahydrofuranne anhydre et 0.84cc de diisopropyléthylamine. On ajoute alors au mélange réactionnel, à la seringue et goutte à goutte, 0.45cc (2 x $10^{-3}$ moles) de 2-cyanoéthoxy diisopropylamino chlorophosphine. Après dix minutes de réaction, un important précipité apparaît au sein du mélange réactionnel (chlorhydrate de diisopropyléthylamine). On hydrolyse le mélange réactionnel (15cc $NaHCO_3$ 5%), on extrait au dichlorométhane (2 x 30cc). La phase organique obtenue est lavée à l'eau (3 x 20cc), puis avec une solution aqueuse saturée en chlorure de sodium (1 x 20cc), elle est séchée sur $MgSO_4$, filtrée et évaporée sous pression réduite (évaporateur rotatif p = 20 mm Hg). Le résidu est purifié sur colonne de silice Merck 9385 - éluant acétate d'éthyle 3 - hexane 7. La silice est préalablement neutralisée par mise en suspension dans l'éluant contenant 1% DIEA. Après purification, on obtient 1.2 g de <u>47</u>. Rdt 84%.

Chromatographie sur couche mince (CCM) plaque Merck 5735 (gel de silice $60F_{254}$) - éluant acétate d'éthyle 3 - hexane 7. RF : 0.6.

EXEMPLE XI : Etude de la réactivité du composé 47

Le phosphoramidite non nucléotidique 47 peut être utilisé pour introduire en 5'(OH) d'une séquence d'acides nucléiques, une chaîne ramifiée : l'intérêt de 47 est de multiplier par deux à chaque condensation, le nombre de sites de condensation. En fin de synthèse, il est alors possible d'introduire sur les sites de condensation ainsi créés, des chaînes alkyle portant une amine primaire. L'introduction des amines primaires est réalisée par condensation finale du dérivé 48 sur les n sites alcool primaire créés.

Dans un premier temps, le dérivé 47 a été condensé dans l'appareil de synthèse automatique d'oligonucléotides en 5'(OH) d'un dimère thymidine-thymidine T-T fixé sur un support solide (support CPG: Controlled Porous Glass) classique en synthèse automatique.

Si l'on représente par le motif provenant de la condensation d'une unité 47 dans lequel ● représente un phosphate, les dimères TT modifiés en 5'(OH) qui ont été synthétisés peuvent se représenter de la façon suivante :

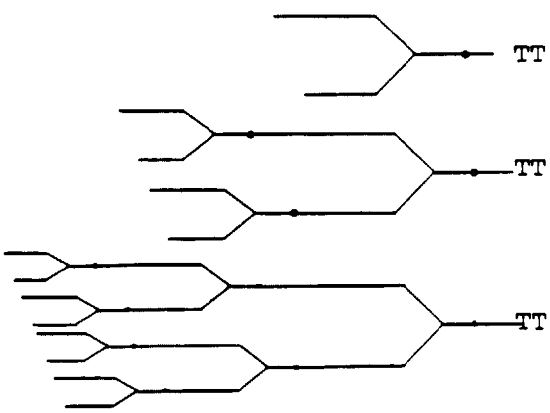


51

52

53

Comme le montrent les analyses en électrophorèse capillaire (Fig. 6), le dérivé 47 se fixe avec succès en 5'(OH) d'un oligonucléotide en élongation (cfr 51), il se fixe avec succès sur les deux sites de condensation créés après une première condensation de 47 sur TT (cfr 52) et il se fixe bien sur les quatre sites de condensation créés après deux condensations successives de 47 sur TT (cfr 53).

Etant donné que l'on multiplie par deux le nombre de sites de condensation à chaque cycle, la solution de 47 qui a été utilisée pour préparer simultanément les dérivés 51, 52 et 53 dans le synthétiseur automatique d'oligonucléotides est 0.4 M (conditions classiques: 0.1 M); de plus, le temps de couplage est porté à 10 minutes (conditions classiques 25 sec.).

Ces résultats montrent aussi que les réactions de condensation, d'oxydation, de capping et de clivage du support solide après synthèse sont efficaces et ne dégradent pas les polymères 51, 52 et 53.

Par ailleurs, en synthèse oligonucléotidique, il est possible de récupérer la solution de déprotection du groupe diméthoxytrityle et de mesurer l'intensité de la coloration orange du cation en vue d'estimer le rendement de chaque condensation. Ces mesures ont été réalisées pour la synthèse de 53 à chaque cycle; elles indiquent de bons rendements comme le montrent les résultats ci-dessous (schéma VIII).

| Schéma VIII | | |
|---|---|---|
| 1° condensation (T) | 1 DMT $A_{497}$: 0.93 | Rdt:100% (imposé) |
| 2° condensation | 2 DMT $A_{497}$: 1.78 | Rdt: 95% |
| 3° condensation | 4 DMT $A_{497}$: 3.4 | Rdt: 95% |
| 4° condensation | 8 DMT $A_{497}$: 5.3 | Rdt: 77% |

Dans un deuxième temps, nous avons réalisé un nombre x de condensations de 47 (x = 1⇒3) sur un dimère thymidine-thymidine TT fixé sur un support solide (support CPG, Controlled Porous Glass) classique en synthèse automatique, puis nous avons terminé par une condensation de 48 sur les n sites de condensation créés en vue d'introduire n sites amine primaire pour la fixation ultérieure de n éléments de marquage.

Si l'on représente

par le motif provenant de la condensation d'une unité 47 où ● représente un phosphate,
par $H_2N$—●— le motif résultant de la condensation d'une unité 48 où ● représente un phosphate,

les dimères TT modifiés qui ont été synthétisés sont les suivants:

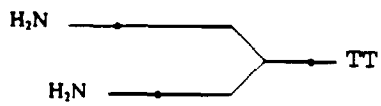
54

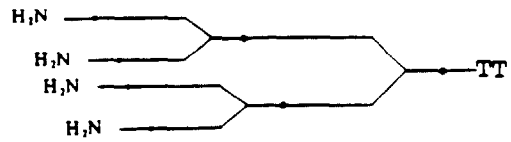
55

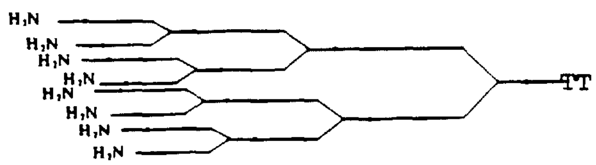
56

Comme le montrent les analyses en électrophorèse capillaire pour le composé 55 comme exemple (Fig. 7), le dérivé 48 se fixe efficacement sur les 4 sites de condensation créés dans le polymère 52 puisque l'on ne trouve qu'une trace de 52 dans l'électrophérogramme de 55.

Etant donné que l'on multiplie par deux le nombre de sites de condensation à chaque cycle, la solution de 47 qui a été utilisée pour préparer simultanément les composés 54, 55 et 56 dans le synthétiseur automatique d'oligonucléotides, est 0.4 M et la solution de 48 est 0.8 M (conditions classiques 0.1 M). De plus, le temps de couplage est porté à 10 minutes pour les condensations de 47 et 48 (conditions classiques 25 secondes).

Couplage en phase solide des dérivés 47 et 48 avec un dimère thymidine-thymidine fixé sur un support solide CPG.

Le phosphoramidite 47 est solubilisé dans l'acétonitrile anhydre à une concentration de 0.4 M et le phosphoramidite 48 est solubilisé dans le même solvant à une concentration de 0.8 M. Ils sont introduits dans le synthétiseur automatique d'oligonucléotides aux endroits prévus pour les phosphoramidites non classiques.

Pour les condensations de 47 et 48, toutes les conditions de condensation sont les conditions d'un cycle d'élongation d'oligonucléotide, exceptés la concentration en phosphoramidite et le temps de couplage. Le clivage du support solide est réalisé dans les conditions habituelles ($NH_4OH$ 32%) et le produit final est obtenu avec un rendement comparable à ceux couramment réalisés en synthèse oligonucléotidique. Les polymères 51, 52, 53, 54, 55 et 56 ont été analysés en électrophorèse capillaire ABI 270A, colonne capillaire Micro-$Gel_{100}$, diamètre interne 50 µm, longueur 50cm, voltage 15 kV, tampon 75 mM tris-phosphate 10% méthanol pH 7.6.

EXEMPLE XII: Préparation de molécules mixtes oligodésoxyribonucléotidiques portant en 5'(OH) un enchevêtrement ramifié de chaînes alkyles se terminant par des chaînes aminées utiles dans la détection de séquences d'ADN par des méthodes non radioactives

La synthèse et l'utilisation de molécules mixtes composées d'une partie oligodésoxyribonucléotidique et d'une partie possédant des propriétés chimiques caractéristiques permettent la détection facile et rapide de cibles d'acides désoxyribonucléiques par des méthodes non radioactives. La partie oligodésoxyribonucléotidique de séquence définie et homologue d'un fragment d'ADN cible apporte l'énergie de stabilisation et assure son hybridation avec la molécule d'ADN que l'on veut détecter. La partie qui fournit la réactivité permettant la détection directe ou indirecte de l'hybride peut être introduite en l'extrémité 5'(OH) et (ou) 3'(OH) de la séquence d'acides nucléiques. Dans cet exemple précis, la partie responsable de la détection sera essentiellement introduite en 5'(OH) de la séquence d'acides nucléiques.

De par la stratégie utilisée, la partie responsable de la détection ne peut être introduite qu'en 5'(OH). Elle le sera en procédant à une série de condensations du phosphoramidite 47 sur la chaîne oligonucléotidique encore fixée sur le support solide et protégée sur ses bases et ses phosphates. Après la série de condensations de 47, une condensation finale du phosphoramidite 48 permettra d'introduire n fonctions amine primaire par condensation de 48 sur les n sites de condensations (sites alcool primaire) créés par les condensations successives de 47.

Les condensations de 47 et de 48 se feront en utilisant les mêmes cycles de réaction dans le synthétiseur automatique d'oligonucléotides que ceux mis en oeuvre pour la synthèse de la séquence d'acides nucléiques. Toutefois, étant donné que les condensations successives de 47 ont pour effet de doubler à chaque cycle le nombre de sites de condensation, la concentration en 47 utilisée est 0.4 M et celle de 48 est 0.8 M (concentration classique 0.1 M). Pour les deux phosphoramidites 47 et 48, le temps de couplage est porté à 10 minutes au lieu des 25 secondes du cycle classique. Après clivage de la chaîne oligonucléotidique 5' modifiée de son support solide et déprotection des bases et des phosphates, la partie qui fournit la réactivité responsable de la détection est introduite par un lien chimique covalent. Parmi les différentes molécules possédant les propriétés requises pour la détection, on utilise de préférence la biotine, la digoxigénine, la fluorescéine, la peroxydase et la phosphatase alcaline. Ces molécules, judicieusement activées, sont fixées sur la chaîne oligonucléotidique au niveau des amines primaires introduites à cet effet en raison de leur nucléophilicité.

Si l'on représente

par le motif provenant de la condensation d'une unité 47 où ● représente un phosphate,
par $H_2N$—● le motif provenant de la condensation d'une unité 48 où ● représente un phosphate,

les oligomères 5'(OH) modifiés qui ont été synthétisés sont les suivants:

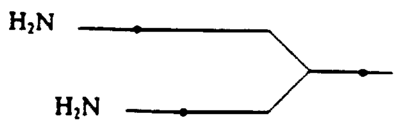 TTTTCAAAGAAGATGGCAAAACA 57

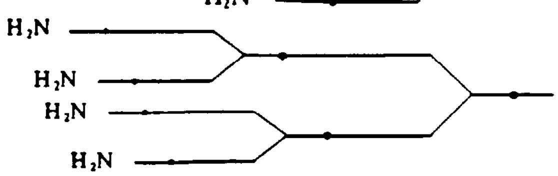 TTTTCAAAGAAGATGGCAAAACA 58

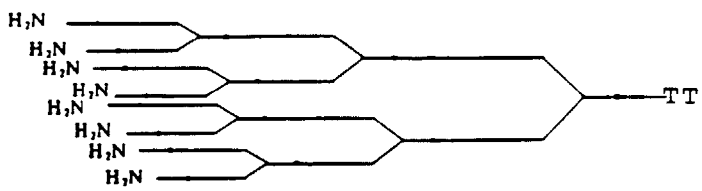 TTTTCAAAGAAGATGGCAAAACA 59

Après clivage de la chaîne oligonucléotidique ainsi modifiée de son support solide et déprotection des bases et des phosphates, les sondes 57, 58 et 59 sont biotinylées pour conduire aux sondes biotinylées correspondantes 60, 61 et 62.(Fig. 8).

Si l'on appelle B l'unité biotine provenant de la condensation de la biotine N hydroxysuccinimide sur les fonctions amine primaire, les sondes polybiotinylées en 5'(OH) qui ont été obtenues sont les suivantes :

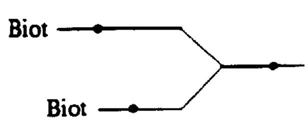 TTTTCAAAGAAGATGGCAAAACA 60

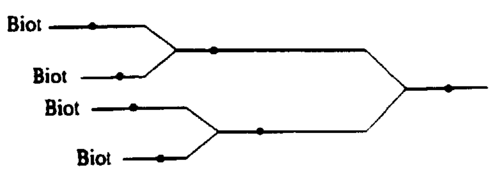 TTTTCAAAGAAGATGGCAAAACA 61

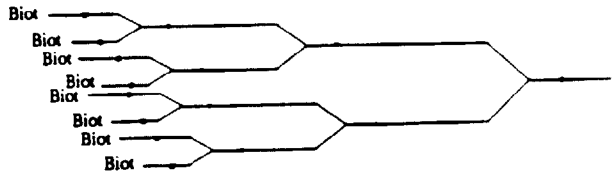


TTTTCAAAGAAGATGGCAAAACA 62

Procédé de préparation de molécules mixtes oligodésoxyribonucléotidiques portant en 5'(OH) un enchevêtrement ramifié de chaînes alkyles se terminant par des chaînes aminées.

Une sonde ADN a été synthétisée avec une séquence oligodésoxynucléotidique complémentaire d'une séquence cible. On prépare en phase solide, dans un synthétiseur automatique d'ADN ABI 394, simultanément trois sondes identiques (dans trois réacteurs) de séquence: 5' TTTTCAAAGAAGATGGCAAAACA 3'. Le synthétiseur est programmé pour continuer des cycles de fixation en 5' d'unités 47 puis en fin de synthèse d'une unité 48 pour conduire respectivement aux sondes 57, 58 et 59.

Pour ce faire, on a utilisé 6 mg de support CPG fonctionnalisé avec de la diméthoxytrityl benzoyl adénosine à 34 µmoles/g, ce qui correspond à 0.2 µmole. Les dérivés 47 et 48 sont solubilisés dans l'acétonitrile anhydre à une concentration 0.4 M pour 47 et 0.8 M pour 48. Ils sont introduits dans le synthétiseur aux endroits prévus pour les phosphoramidites non classiques.

Après synthèse automatique des biopolymères 57, 58 et 59, les chaînes oligonucléotidiques ainsi modifiées sont clivées des supports CPG par quatre traitements successifs de 15 minutes avec 500 µl de $NH_4OH$ 32%. Les solutions ammoniacales obtenues sont portées 5 h à 55°C. Ce deuxième traitement a pour but de déprotéger les bases et les phosphates des chaînes oligonucléotidiques. Les solutions ammoniacales sont liophylisées, les quatre résidus sont soumis à une chromatographie par filtration moléculaire (gel sephadex G50) puis à une électrophorèse sur gel de polyacrylamide 20%.

Procédé de biotinylation de molécules mixtes oligodésoxyribonucléotidiques portant en 5'(OH) un enchevêtrement ramifié de chaînes alkyles se terminant par des chaînes aminées.

Dans un tube Eppendorf, on dissout 20 DO de sonde aminée 57, 58 ou 59 dans 120 µl de tampon phosphate 0.01 M pH 7.5. On ajoute une solution de 20 mg de sulfosuccinimidyl 6-biotinamidocaproate dans 240 µl de diméthylformamide. On laisse incuber à température ambiante 16 h. Chaque solution est soumise à une chromatographie par filtration moléculaire (gel sephadex G50) puis à une électrophorèse sur gel de polyacrylamide 20%.

Les sondes 57, 58, 59, 60, 61 et 62 ont été analysées en électrophorèse capillaire sur un ABI 270A, colonne Micro-$gel_{100}$ diamètre interne 50 µm, longueur 50 cm, voltage 15 kV, tempon 75 mM tris-phosphate - 10% méthanol pH 7.6.

EXEMPLE XIII: Utilisation des sondes biotinylées décrites dans l'exemple XII en hybridation sur support solide - Limites de détection.

Afin de mettre en évidence les avantages de la présente invention, les sondes polybiotinylées en 5'(OH) 60, 61 et 62 ont été testées en hybridation en vue d'étudier leur potentiel d'utilisation en diagnostic.

De manière générale, l'hybridation s'est faite entre un oligonucléotide (30 mer) fixé sur un support solide en plastique par son extrémité 5'(OH) et complémentaire des sondes à tester (23 mer) par son extrémité 3'(OH). Chaque support solide (tube) revêt de l'ordre de 10 ng d'oligonucléotide (30 mer) complémentaire aux sondes à tester, ce qui représente environ $10^{12}$ copies.

Chaque oligonucléotide biotinylé mentionné ci-dessus est soumis à 8 hybridations différentes, partant de 1 pg d'oligonucléotide marqué (~ $10^8$ copies) et allant jusqu'à 300 atg (3 x $10^4$ copies) en procédant à des dilutions 1/3, d'une cuvette à l'autre. Après hybridation, la révélation se fait par chemiluminescence (CSPD - Tropix); la lecture se fait au luminomètre.

Les résultats des hybridations des sondes 60, 61 et 62 sont repris au schéma IX.

| Schéma IX | | | |
|---|---|---|---|
| OLIGO | Nombre de Biotines | Limite de détection | |
| | | fg | nombre de copies |
| 60 | 2 5'(OH) | 10 | $10^6$ |

(suite)

| Schéma IX | | | |
|---|---|---|---|
| OLIGO | Nombre de Biotines | Limite de détection | |
| | | fg | nombre de copies |
| 61 | 4 " | 10 | $10^6$ |
| 62 | 8 " | 3 | $3 \times 10^5$ |

Procédé d'hybridation d'une sonde mono ou polybiotinylée (23 mer) avec un oligomer complémentaire (30 mer) fixé sur un tube en plastique.

Les tubes, recouverts de l'oligonucléotide complémentaire (30 mer) sont d'abord saturés une heure à 37°C avec 200 µl d'une solution à 5% de lait écrémé liophylisé dans le TBS 1x, contenant une trace d'azide. Les tubes sont ensuite préhybridés 2 h à 50°C avec le tampon d'hybridation (100 µl): 5x Denhardt's, 6xSSC, 0.1% SDS, 100 µg/ml de DNA de sperme de saumon.

Pour chaque oligonucléotide à tester, on réalise 8 essais avec les quantités suivantes (dans 100 µl de tampon d'hybridation) : 1 pg ($10^8$ copies), 300 fg, 100 fg ($10^7$ copies), 30 fg, 10 fg ($10^6$ copies), 3 fg, 1 fg ($10^5$ copies) 0.3 fg. On réalise également une série de huit tubes avec un oligomère non complémentaire et non biotinylé et huit tubes sans oligonucléotide. L'ensemble des tubes est mis en hybridation 2 h à 50°C puis chaque tube est lavé trois fois dix minutes à 50°C avec une solution SSC 6x, ils sont ensuite lavés une fois cinq minutes avec le tampon I.

Tampon I: 0.1 M tris, 2 mM $MgCl_2$, 0.05% triton, 1 M NaCl.

On prépare alors une solution de streptavidine-phosphatase alcaline (fournie avec le kit Tropix): 1 µl de la solution du kit dans 5500µl de tampon I. On met 100 µl de cette solution dans chaque tube, on laisse incuber trente minutes à température ambiante, on lave ensuite chaque tube six fois avec le tampon I. On prépare la solution d'agent chemiluminescent, le CSPD: (3-(4-méthoxyspiro[1,2 -dioxétane-3,2'-tricyclo[3.3.1.1.]chlorodécane]-4-yl)phenylphosphate. La solution est préparée comme suit: 80 µl de CSPD (Tropix), 500 µl d'emerald (Tropix) et 4420 µl de tampon de révélation.

Tampon de révélation: 0.1 M diéthanolamine, 1 mM $MgCl_2$, 0.02% azide de sodium. On dépose 100 µl de cette solution dans chaque tube, on laisse incuber 45' puis on lit au luminomètre.

**Revendications**

**1.** Sonde d'acides nucléiques pour détecter une molécule d'ADN ou d'ARN, comportant

a) une partie oligonucléotidique ou oligodésoxynucléotidique constituée de

S : une séquence d'acide nucléiques ADN ou ARN en fonction du type de molécule à détecter et

b) une partie non nucléotidique possédant des propriétés chimiques qui permettent la fixation directe ou indirecte d'une ou plusieurs unités de détection ou éléments de marquage M détectables de manière non isotopique par production de couleur ou de lumière,

caractérisée en ce que la partie b) est constituée d'une chaîne d'unités phosphates entrecoupées de motifs alkyle à savoir

b1) certains motifs alkyle unissant les différents groupes phosphates et ne présentant aucune fonctionnalité particulière

b2) des motifs alkyle présentant des fonctions amines primaires qui permettent le couplage avec des réactifs variés pour réaliser la détection de manière directe ou indirecte,

les motifs b2) étant liés à la partie a) ou séquence S par l'intermédiaire des motifs b1).

**2.** Sonde d'acides nucléiques suivant la revendication 1,
caractérisée en ce que la partie b) est en forme de chaîne linéaire dans laquelle les motifs b1) forment des "bras

chimiques" L servant à introduire des espacements entre les motifs b2) qui constituent les éléments de marquage M proprement dits.

**3.** Sonde d'acides nucléiques suivant la revendication 2, répondant à la formule

$$[(M)y'-(L)x']z' -S-[(L)x-(M)y]z \qquad (I)$$

dans laquelle S, L et M ont les significations données dans les revendications 1 et 2, et

x, x', z, z' sont des nombres égaux ou supérieurs à 0, avec la restriction que z et z' ne sont jamais simultanément égaux à 0
y et y' sont des nombres supérieurs à 0.

**4.** Sonde d'acides nucléiques suivant la revendication 3, caractérisée en ce qu'elle répond a la formule générale la

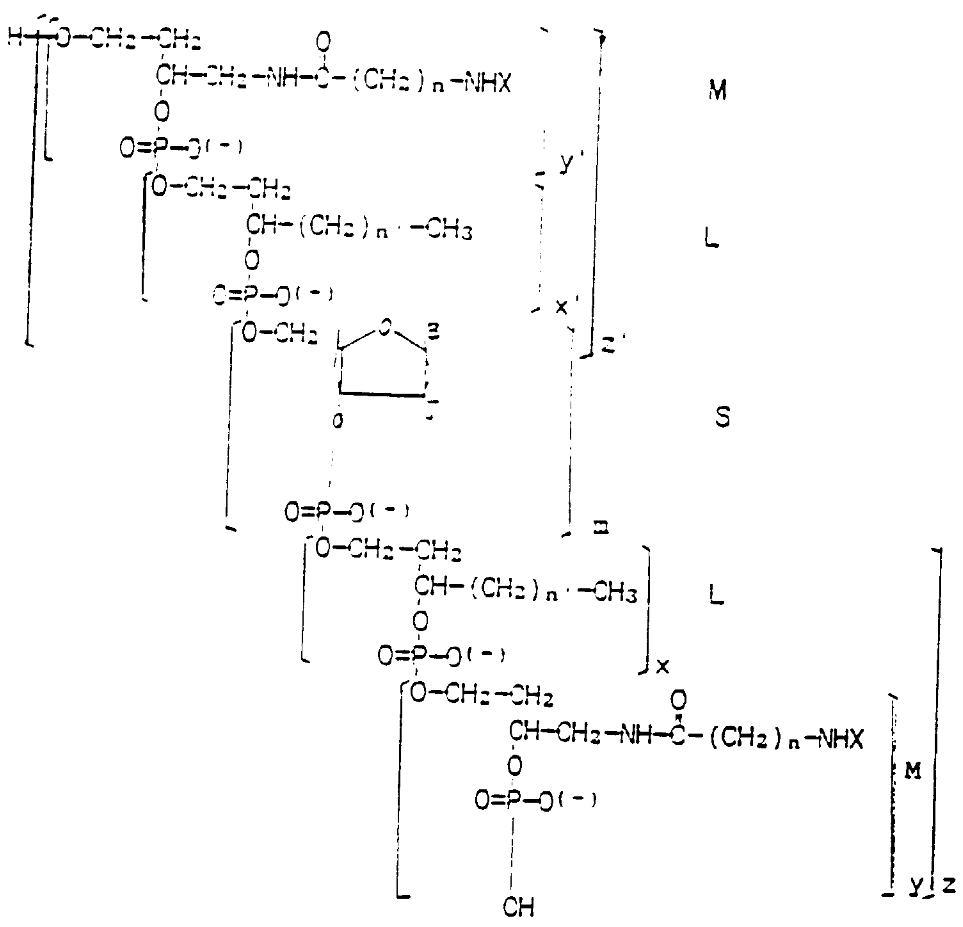

(Ia)

dans laquelle :

S, L et M ont les significations données dans les revendications 1 et 2, et dans la séquence S,

J désigne H ou OH
B désigne une base d'acide nucléique. purique ou pyrimidique variable selon les nucléotides et
m = 1 à 1.000.

dans la séquence L

x et x' = 0 à 100
n' = 0 à 20

dans la séquence M

y et y' = 1 à 100
n = 2 à 20

X désigne le marqueur proprement dit, pouvant être :

soit direct, auquel cas il est choisi parmi les enzymes telles que phosphatase alcaline, peroxydase et fluorescéine, capables de produire une coloration ou une lumière en présence d'un substrat,
soit indirect, auquel cas il est choisi parmi les haptènes tels que la biotine ou la digoxigénine, capables d'être reconnus par des anticorps marqués de manière non isotopique,
l'élément LM responsable de la détection de la sonde pouvant être introduit

en 5'(OH) pour z'≠0 et z=0
ou en 3'(OH) pour z'=0 et z≠0
ou simultanément en 5'(OH) et en 3'(OH) pour z'≠0 et z≠0

z et z' étant de manière générale = 0 à 100.

**5.** Sonde d'acides nucléiques suivant la revendication 1, caractérisée en ce que la partie b) formant l'élément de marquage M est constituée d'une chaîne ramifiée d'unités phosphates entrecoupées de motifs alkyle comprenant les motifs b1), comme motifs internes à la ramification et les motifs b2) comme motifs externes à la ramification, c'est-à-dire terminant les différents bras de la ramification.

**6.** Sonde d'acides nucléiques suivant la revendication 5, caractérisée en ce qu'elle répond à la formule générale XX

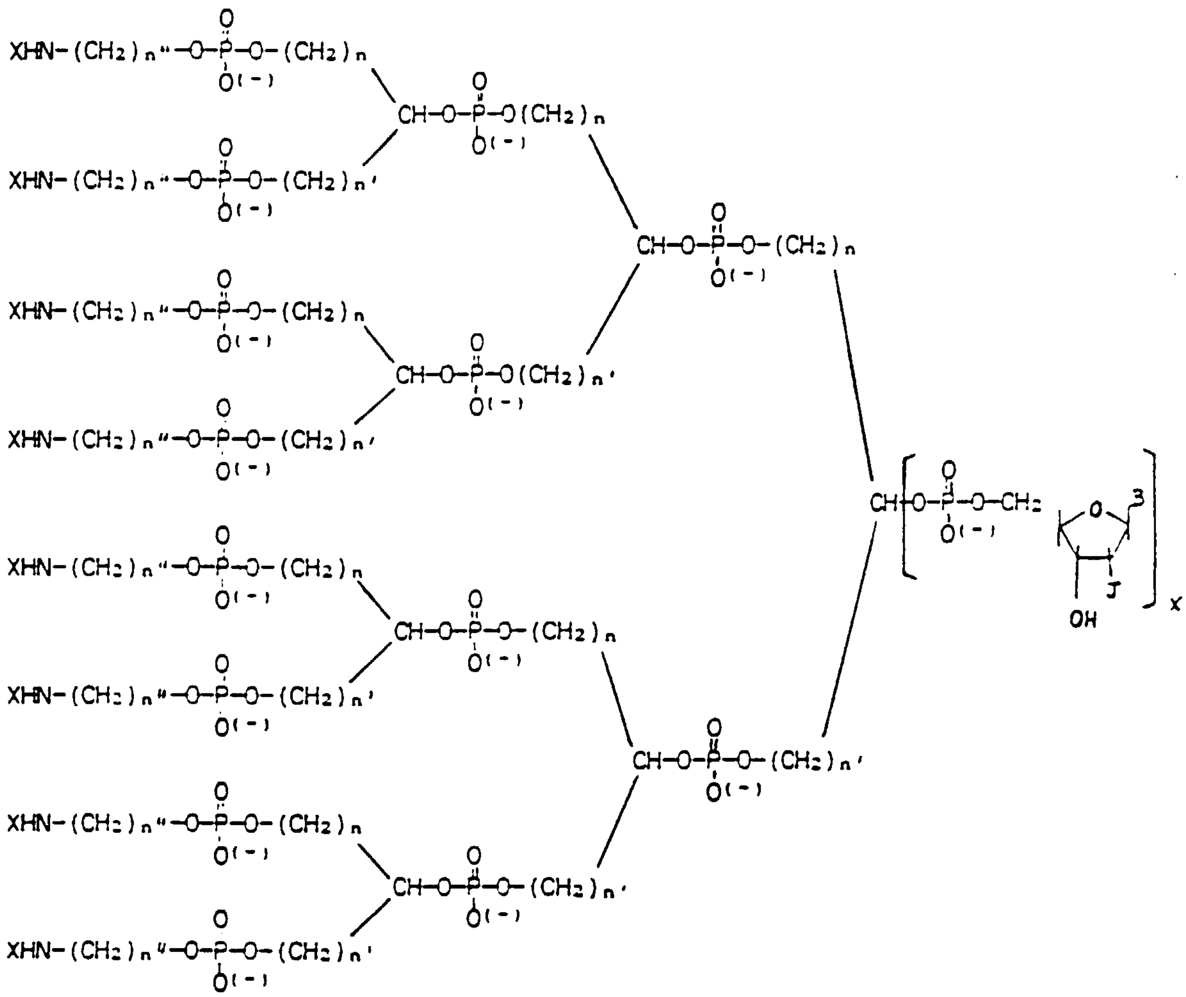

M S

XX

dans laquelle

dans la séquence S,

J désigne H ou OH

B désigne une base nucléique, purique ou pyrimidique, variable selon les nucléotides

x représente le nombre de nucléotides de 1 à 1.000

n, n' et n" sont égaux à 1 à 20

M désigne l'élément de marquage dont l'ordre de ramification peut prendre des valeurs de 2 à 128, par exemple 8, l'ordre supérieur étant toujours double de celui qui le précède;

X désigne le marqueur proprement dit, pouvant être

soit direct, auquel cas il est choisi parmi les enzymes telles que phosphatase alcaline, peroxydase et fluorescéine, capables de produire une coloration ou une lumière en présence d'un substrat,

soit indirect, auquel cas il est choisi parmi les haptènes tels que la biotine ou la digoxigénine, capables d'être reconnus par des anticorps marqués de manière non isotopique.

**7.** Procédé de préparation de sondes de formule la telle que définie à la revendication 4, comportant

A1) la synthèse d'une séquence d'acides nucléiques S

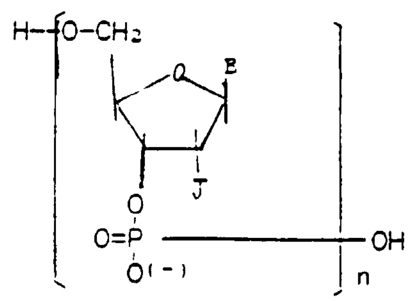

dans laquelle B et J et n ont les significations données à la revendication 4 par toute méthode de synthèse d'assemblage internucléotidique manuelle ou automatique connue, de préférence sur support solide, et

A2) la fixation d'un marqueur ,

caractérisé en ce que, pour la fixation du marqueur, ladite séquence est soumise de préférence par la même méthode de synthèse, notamment sur support solide,

soit à une extension en son extrémité 5'(OH) par une série d'unités M et L

soit à une extension en son extrémité 3'(OH) par une série d'unité M et L

soit à une extension en ses extrémités 5'(OH) et 3'(OH) par deux séries d'unités M et L, M et L étant tels que définis à la revendication 4,

B) les dites unités L sont obtenues chacune par synthèse d'un polymère non nucléotidique

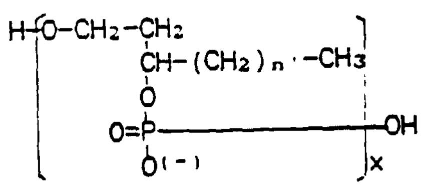

dans laquelle n' et x ont les significations données à la revendication 4,

C) lesdites unités M sont obtenues chacune par synthèse d'un polymère non nucléotidique

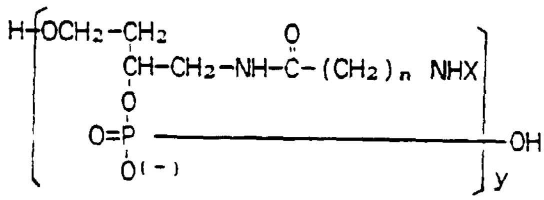

dans laquelle

n et y ont les significations données à la revendication 4

et

X représente soit un marqueur tel que phosphatase alcaline, peroxydase, fluorescéine, biotine, digoxigénine, tout haptène capable d'être reconnu par des anticorps marqués de manière non isotopique, soit un groupe protecteur de la fonction amine primaire destiné à être éliminé après synthèse totale de la sonde d'acides nucléiques, par exemple un groupe acétyle (VI), trifluoracétyle (VII) ou benzoyle (VIII):

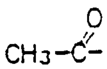

VI

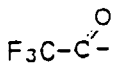

VII

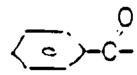

VIII

et les synthèses B et C ci-dessus se font plus particulièrement par toute méthode de synthèse d'assemblage internucléotidique manuelle ou automatique connue, de préférence sur support solide.

**8.** Procédé suivant la revendication 7 pour la préparation d'une sonde constituée par une chaîne oligonucléotidique portant en 5'(OH) un enchevêtrement de bras chimiques entrecoupés de bras aminés utiles dans la détection de séquences d'ADN par des méthodes non radioactives, caractérisé par les opérations consistant à

- synthétiser sur un support solide une chaîne oligonucléotidique dont les fonctions phosphates et les bases sont protégées;
- condenser à l 'extrémité 5'(OH) de ladite chaîne une succession de dérivés générateurs de bras chimiques et de bras aminés en mettant en oeuvre les mêmes cycles de condensation que pour la synthèse de la chaîne oligonucléotidique;
- séparer la chaîne oligonucléotidique 5' modifiée de son support solide;
- déprotéger les bases et phosphates de ladite chaîne;
- introduire au niveau des groupes amino primaire la partie qui fournit la réactivité responsable de la détection.

**9.** Procédé suivant la revendication 7, pour la préparation d'une sonde constituée par une chaîne oligonucléotidique portant en 3'(OH) un enchevêtrement de bras chimiques entrecoupés de bras aminés utiles dans la détection de séquences d'ADN par des méthodes non-radioactives, caractérisé par les opérations suivantes consistant à

- condenser sur un support solide des dérivés générateurs de bras chimiques et de bras aminés selon les mêmes cycles de condensation que pour la synthèse d'acides nucléiques, pour former une séquence souhaitée de bras chimiques et de bras aminés,
- construire à la suite de cette séquence, une chaîne oligonucléotidique, pour obtenir ainsi une chaîne oligonucléotidique 3'(OH) modifiée, dont les fonctions phosphates et bases sont protégées,
- séparer cette chaîne modifiée du support solide,
- déprotéger les phosphates et bases,
- introduire au niveau des fonctions amino primaires la partie qui fournit la réactivité responsable de la détection.

**10.** Procédé suivant la revendication 7, pour la préparation de sondes à chaîne oligodésoxyribonucléotidique portant en 3'(OH) et en 5'(OH) un enchevêtrement de bras chimiques entrecoupés de bras aminés utiles dans la détection de séquences d'ADN par des méthodes non-radioactives, caractérisé par les opérations consistant à

- condenser sur un support solide des dérivés générateurs de bras chimiques et de bras aminés sur un support solide selon les mêmes cycles de condensation que pour la synthèse d'acides nucléiques, pour former une séquence souhaitée de bras chimiques et de bras aminés,
- construire, à la suite de cette séquence, une chaîne oligonucléotidique fixée à son extrémité 3'(OH) modifiée dont les fonctions phosphates et bases sont protégées,
- construire, à l'extrémité 5'(OH) de la chaîne oligonucléotidique, une séquence de bras chimiques et de bras aminés,
- séparer la chaîne oligonucléotidique modifiée en 3'(OH) et en 5'(OH) de son support solide, déprotéger les phosphates et bases et
- introduire au niveau des fonctions amino primaires la partie fournissant la réactivité responsable de la détection.

**11.** Procédé suivant l'une quelconque des revendications 7 à 10, caractérisé en ce que chaque élément de bras chimique L est introduit par condensation d'un composé de formule

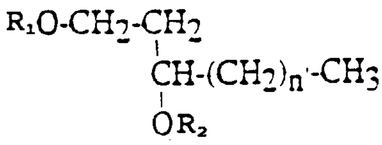

(II)

où $R_1$ représente un groupe 4,4'-diméthoxytrityle (en abrégé DMT) de formule

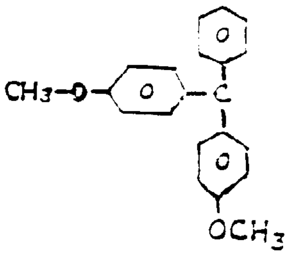

DMT (IV)

et où $R_2$ représente un groupe phosphorylé éventuellement protégé approprié à l'introduction du composé II (synthon) à l'extrémité d'un nucléotide ou bien d'un synthon déjà existant

et plus particulièrement, $R_2$ est un groupe diisopropylaminocyanoéthoxyphosphine ou cyanoéthoxydiisopropylaminophosphoramidite de formule

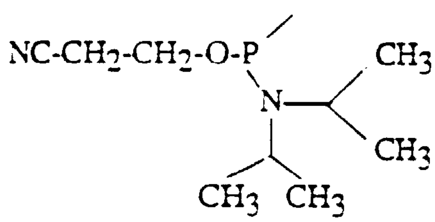

(V)

**12.** Procédé suivant l'une quelconque des revendications 7 à 11, caractérisé en ce que chaque élément ou unité de détection M est obtenu au départ de butène-3ol-1 de formule

$$HO\text{-}CH_2\text{-}CH_2\text{-}CH{=}CH_2 \quad (XXX)$$

par les étapes de

a) protection de la fonction alcool primaire au moyen d'un groupe protecteur $R_1$ pour obtenir un composé de formule

$$R_1\text{-}O\text{-}CH_2\text{-}CH_2\text{-}CH{=}CH_2 \quad (XXXI)$$

b) époxydation de la double liaison du composé (XXXI) pour former un composé de formule

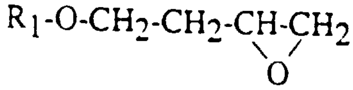

(XIIa)

c) ouverture de l'époxyde au moyen d'ammoniaque pour former un composé de formule

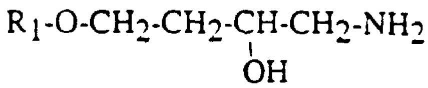

(XIIIa)

d) protection du groupe amino primaire d'un composé de formule

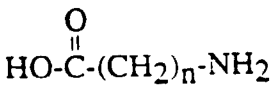

(XXXII)

pour former un composé de formule

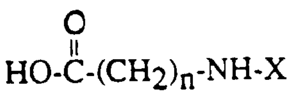

(XXXIII)

où X a les significations données à la revendication 7.

e) activation du composé (XXXIII),

f) condensation des composés (XIIIa) et (XXXIII) entre eux pour former un compose de formule

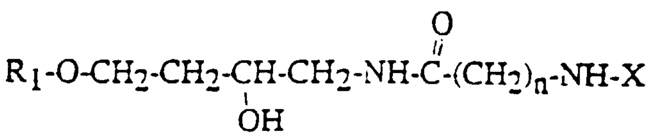

(XVa)

et

g) phosphorylation de composé (XVa) pour former un composé de formule

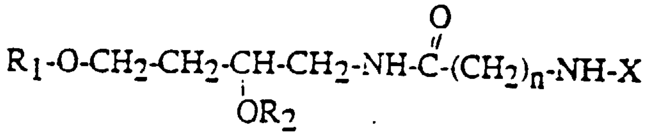

(III)

dans laquelle $R_2$ représente un groupe phosphorylé apte à l'introduction du composé IIIa (synthon) à l'extrémité (3' et/ou 5') d'un nucléotide ou bien à l'extrémité d'un synthon déjà existant.

**13.** Procédé suivant la revendication 12, caractérisé en ce que,

à l'étape a) $R_1$ signifie 4,4'-diméthoxytrityle (en abrégé DMT) et la protection de la fonction alcool primaire est réalisée au moyen de chlorure de 4,4'-diméthoxytrityle (DMT-Cl) pour obtenir un composé de formule

$$DMTO\text{-}CH_2\text{-}CH_2\text{-}CH{=}CH_2 \qquad (XXXIa)$$

à l'étape b) l'époxydation est réalisée par l'acide métachloroperbenzoïque pour former un composé de formule

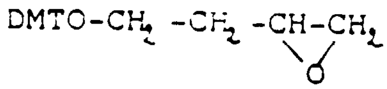
(XII)

à l'étape c) l'ouverture de l'époxyde est réalisée au moyen d'ammoniaque pour former un composé de formule

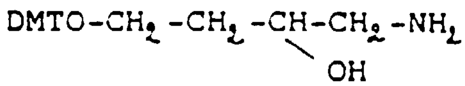
(XIII)

à l'étape d) la protection du groupe amino primaire de composé (XXVI) est réalisé par un groupe trifluoracétyle pour former un composé

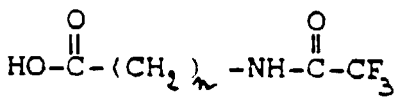
(XXXIIa)

à l'étape e) le composé (XXXIIa) est activé par la N-hydroxysuccinimide pour former le composé

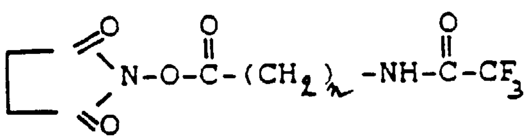
(XIVa)

à l'étape f) les composés (XIII) et (XIVa) sont condensés pour former un composé de formule

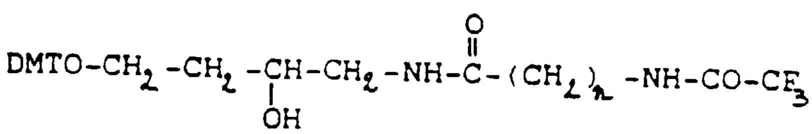
(XVb)

à l'étape g) le composé (XVb) est phosphorylé pour obtenir un composé de formule

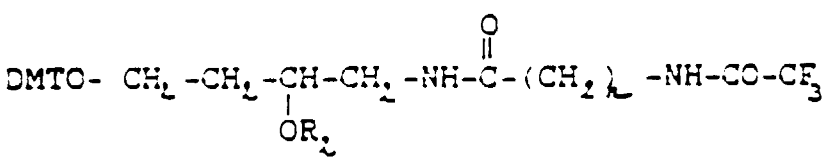
(IIIa)

dans laquelle $R_2$ représente un groupe phosphorylé éventuellement protégé, approprié à l'introduction du composé ou synthon (IIIa) à l'extrémité d'un nucléotide ou bien d'un synthon déjà condensé sur un support

solide approprié pour un type donné de synthèse d'assemblage internucléotidique.

**14.** Procédé suivant la revendication 13, lorsqu'il est appliqué à la synthèse aux phosphoramidites, caractérisé en ce que l'agent de phosphorylation utilisé à l'étape g) est la diisopropylaminocyanoéthoxychlorophosphine de formule

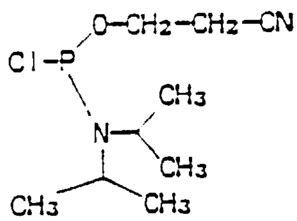

XI

si bien que dans le composé de formule III obtenu à l'issue de cette étape, $R_2$ est un groupe diisopropylamino-cyanoéthoxyphosphino ou cyanoéthoxydiisopropylaminophosphoramidite de formule

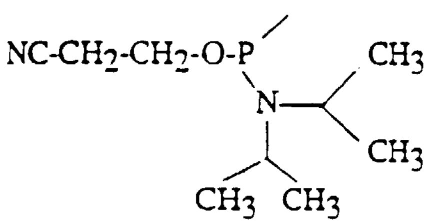

(V)

**15.** Procédé suivant la revendication 16 caractérisé en ce que dans les formules (XXXIIa, XIVa, XVb et IIIa) n est égal à 5.

**16.** Procédé de préparation de sondes de formule XX telle que définie à la revendication 6 comportant la synthèse d'une séquence d'acides nucléiques S

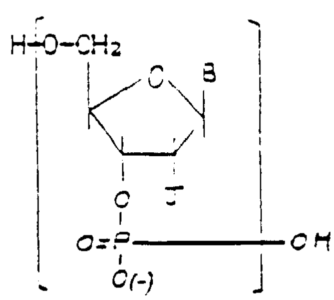

dans laquelle B et J ont les significations données précédemment à la revendication 4, par toute méthode de synthèse d'assemblage internucléotidique manuelle ou automatique connu, de préférence sur support solide,
et la fixation d'un marqueur
caractérisé en ce que pour la fixation du moyen de marquage, ladite séquence est soumise, de préférence par la même méthode de synthèse, notamment sur support solide, à une extension en son extrémité 5'(OH) par un élément de marquage M tel que défini à la revendication 6 formant un édifice moléculaire ramifié dont les bras terminaux portent chacun une fonction amino primaire.

**17.** Procédé selon la revendication 16 pour la préparation d'une sonde constituée par une chaîne oligonucléotidique portant en 5'(OH) un enchevêtrement ramifié de chaînes alkyles se terminant par des chaînes aminées utiles dans la détection de séquences d'ADN par des méthodes non radioactives, caractérisé par les opérations consistant à

a) synthétiser sur un support solide une chaîne oligonucléotidique dont les fonctions phosphates et les bases sont protégées;

b) condenser à l'extrémité 5'(OH) de ladite chaîne une série de composés de formule

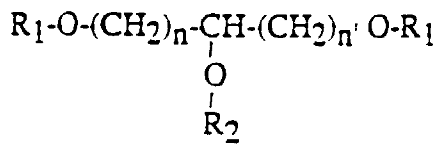

XXI

où $R_1$ signifie 4,4'-diméthoxytrityle (en abrégé DMT) de formule

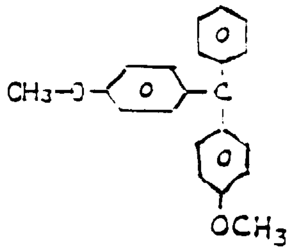

DMT (IV)

et $R_2$ représente un groupe phosphorylé approprié à l'introduction du composé XXI (synthon) à l'extrémité d'un nucléotide ou d'un synthon déjà existant, ce qui donne lieu à n sites de condensation étant des sites alcool primaire et plus particulièrement $R_2$ est un groupe diisopropylaminocyanoéthoxyphosphine ou cyanoéthoxydiisopropylaminophosphoramidite de formule

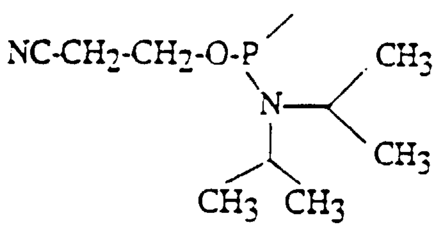

(V)

c) effectuer une condensation finale sur les n sites de condensation créés d'un composé de formule

$$XHN\text{-}(CH_2)_{n''}\text{-}OR_2 \qquad XXII$$

dans laquelle

$R_2$ a les significations données ci-dessus et X représente soit un marqueur : phosphatase alcaline, peroxydase, fluorescéine, biotine, digoxigénine, tout haptène capable d'être reconnu par des anticorps marqués de manière non isotopique soit un groupe protecteur transitoire de la fonction amine primaire destiné à être éliminé après synthèse totale de la sonde d'acides nucléique XX; à titre illustratif, dans cette approche de synthèse, X peut représenter un trifluoroacétyle VII, un fluorénylméthoxycarbonyle XXIII

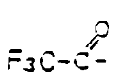

VII

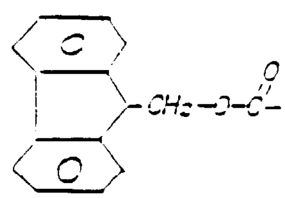

XXIII

les condensations des composés XXI et XXII mettant en oeuvre les mêmes cycles de réaction que pour la synthèse de la séquence oligoucléotidique,

d) déprotéger les bases et phosphates de ladite chaîne oligonucléoidique et

e) introduire au niveau des groupes amino primaire la partie qui fournit la réactivité responsable de la détection.

**18.** Procédé suivant la revendication 17, caractérisé en ce que, dans le composé XXIa n est égal à 1, n' est égal à 4 et $R_2$ est un groupe cyanoéthoxydiisopropylaminophosphoramidite, en sorte que le composé XXIa mis en oeuvre répond à la formule

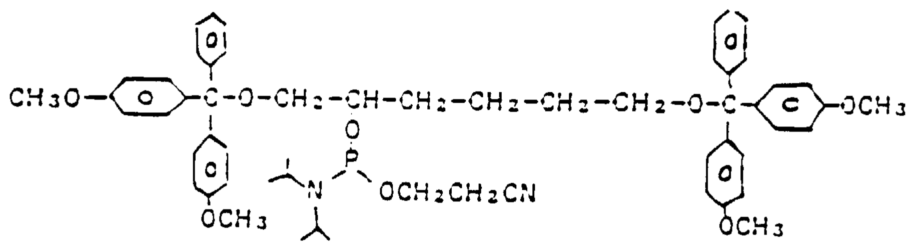

47

**19.** Procédé suivant la revendication 17, caractérisé en ce que dans le composé XXII,

| | |
|---|---|
| le groupe $R_2$ | est un groupe cyanoéthoxydiisopropylaminophosphoramidite et |
| X | est un groupe fluorénylméthoxycarbonyle et |
| n" | est égal à 6 |

en sorte que le composé XXII mis en oeuvre répond à la formule

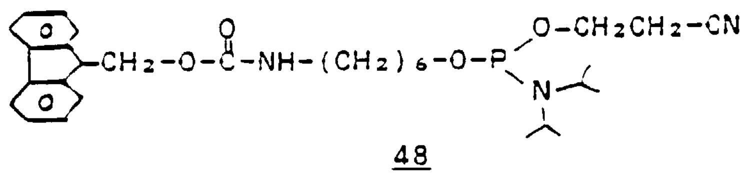

48

**20.** Procédé suivant l'une quelconque des revendications 7-19, caractérisé en ce que dans le cas de oligonucléotides marqués en 3'(OH), le CPG est fonctionnalisé par un composé de formule

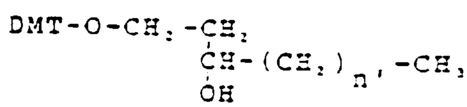

(X)

dans laquelle DMT et n' ont les significations données aux revendications 7 et 11, en sorte que, après fonctionnalisation, le support répond à la formule

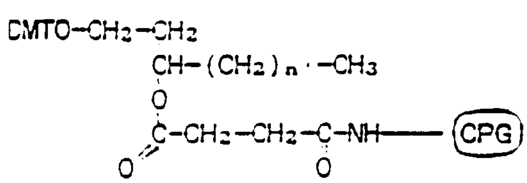

XIX

**21.** A titre d'intermédiaires utiles pour la préparation des unités de marquage M, les composés de formule

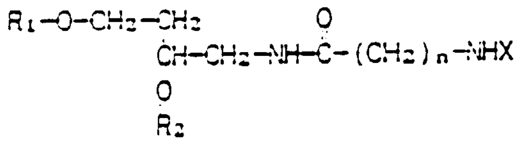

(III)

dans laquelle

- n représente un nombre entier de 2 à 20.
- $R_1$ est un groupe protecteur de la fonction alcool primaire et
- $R_2$ représente H ou tout groupe phosphorylé éventuellement protégé approprié à l'introduction du synthon III à l'extrémité d'un nucléotide ou d'un synthon déjà condensé sur un support solide pour un type donné de synthèse d'assemblage internucléotidique

et

- X représente tous des marqueurs soit un groupe protecteur transitoire de la fonction amine primaire destiné à être éliminé après synthèse totale de la sonde d'acides nucléiques.

**22.** Composés selon la revendication 21 caractérisés en ce que

- $R_1$ représente un groupe 4,4'-diméthoxytrityle (abrégé (DMT) labile en milieu acide

et

- $R_2$ représente un groupe cyanoéthoxydiisopropylaminophosphoramidite.

**23.** Composés selon les revendications 21 et 22, caractérisés en ce que

- X représente un groupe acétyle trifluoracétyle ou benzoyle.

**24.** Procédé de préparation des composés intermédiaires de formule III telle que définie à la revendication 21, caractérisé en ce qu'il part d'un butène-3-ol-1 de formule

$$HO\text{-}CH_2\text{-}CH_2\text{-}CH{=}CH_2 \qquad \text{(XXX)}$$

lequel est soumis successivement à des étapes, de protection de la fonction alcool primaire, d'époxydation de la liaison double, d'ouverture de l'époxyde, de réaction de condensation avec un composé de formule

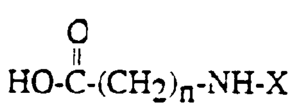

(XXXIII)

et de phosphorylation du produit de condensation telles que décrites à la revendication 12 avec, de préférence, les particularités décrites aux revendications 13 à 15.

### Patentansprüche

**1.** Nukleinsäuresonde zum Nachweis eines ADN- oder ARN-Moleküls, umfassend

a) einen Oligonukleotid- oder Oligodesoxynukleotidteil, aufgebaut aus

S: einer ADN- oder ARN-Nukleinsäuresequenz, je nach dem nachzuweisenden Molekültyp und

b) einen nichtnukleotiden Teil, der chemische Eigenschaften besitzt, die die direkte oder indirekte Fixierung eines oder mehrerer Nachweiseinheiten oder Marker M ermöglichen, die nichtisotopisch durch die Erzeugung von Färbung oder Licht nachweisbar sind,

dadurch gekennzeichnet, daß der Teil b) gebildet ist von einer Kette aus Phosphateinheiten, die von Alkyleinheiten unterbrochen sind, nämlich

b1) bestimmten Alkyleinheiten, die die verschiedenen Phosphatgruppen verbinden und keine besondere Funktionalität zeigen,

b2) primäre Aminfunktionen aufweisende Alkyleinheiten, die die Kopplung mit verschiedenen Reagenzien erlauben, um den Nachweis auf direkte oder indirekte Art und Weise zu ermöglichen,

wobei die Einheiten b2) über die Zwischeneinheiten b1) mit dem Teil a) oder der Sequenz S verbunden sind.

**2.** Nukleinsäuresonde nach Anspruch 1, dadurch gekennzeichnet, daß der Teil b) in Form einer linearen Kette vorliegt, in der die Einheiten b1) "chemische Zweige" L bilden, die zur Einführung von Zwischenräumen zwischen den Einheiten b2), welche die eigentlichen Marker M bilden, dienen.

**3.** Nukleinsäuresonde nach Anspruch 2, entsprechend der Formel

$$[(M)y'-(L)x']z'-S-[(L)x-(M)y]z \qquad (I)$$

in der S, L und M die in den Ansprüchen 1 und 2 angegebenen Bedeutungen haben, und

x, x', z, z' Zahlen gleich oder größer 0 sind, mit der Einschränkung, daß z und z' nicht gleichzeitig 0 sind und y und y' Zahlen größer 0 sind.

**4.** Nukleinsäuresonde nach Anspruch 3, dadurch gekennzeichnet, daß sie der allgemeinen Formel Ia entspricht

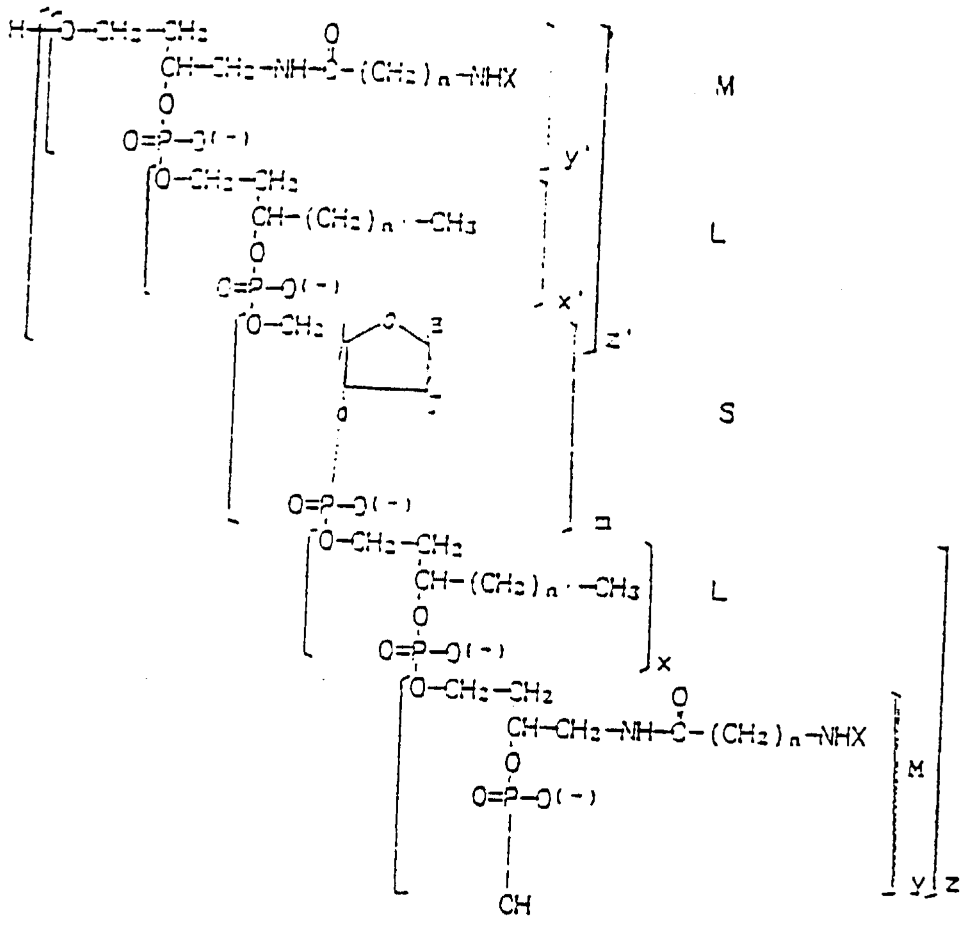

(Ia)

in der:

S, L und M die in den Ansprüchen 1 und 2 angegebenen Bedeutungen haben, und in der Sequenz S

J die Bedeutung H oder OH hat,
B eine Nuklein-, Purin- oder Pyrimidin-säurebase bedeutet, je nach den Nukleotiden veränderbar, und
m = 1 bis 1000,

in der Sequenz L

x und x' = 0 bis 100
n' = 0 bis 20

in der Sequenz M

y und y' = 1 bis 100
n = 2 bis 20 ist,

X den eigentlichen Marker bezeichnet, der:
entweder ein direkter Marker sein kann, in welchem Fall er aus den Enzymen wie alkalische Phosphatase, Peroxidase und Fluorescein ausgewählt ist, die in der Lage sind, in Gegenwart eines Substrats eine Färbung oder Licht zu erzeugen,

oder ein indirekter Marker sein kann, in welchem Fall er aus den Haptenen, wie dem Biotin oder dem Digoxigenin ausgewählt ist, die in der Lage sind, durch nichtisotopisch markierte Antikörper erkannt zu werden und

wobei das für den Nachweis der Sonde verantwortliche Element LM eingeführt werden kann bei 5'(OH), wenn $z' \neq 0$ und $z = 0$ oder bei 3'(OH), wenn $z' = 0$ und $z \neq 0$ oder gleichzeitig bei 5'(OH) und bei 3'(OH), wenn $z' \neq 0$ und $z \neq 0$,

wobei z und z' im allgemeinen = 0 bis 100 sind.

**5.** Nukleinsäuresonde nach Anspruch 1, dadurch gekennzeichnet, daß der den Marker M bildende Teil b) eine verzweigte Kette aus Phosphateinheiten aufweist, die unterbrochen sind von Alkyleinheiten, die die Einheiten bl) als verzweigungsinnere Einheiten und die Einheiten b2) verzweigungsäußere Einheiten, d.h. am Ende der verschiedenen Zweige der Verzweigung, umfassen.

**6.** Nukleinsäuresonde nach Anspruch 5, dadurch gekennzeichnet, daß sie der allgemeinen Formel XX entspricht

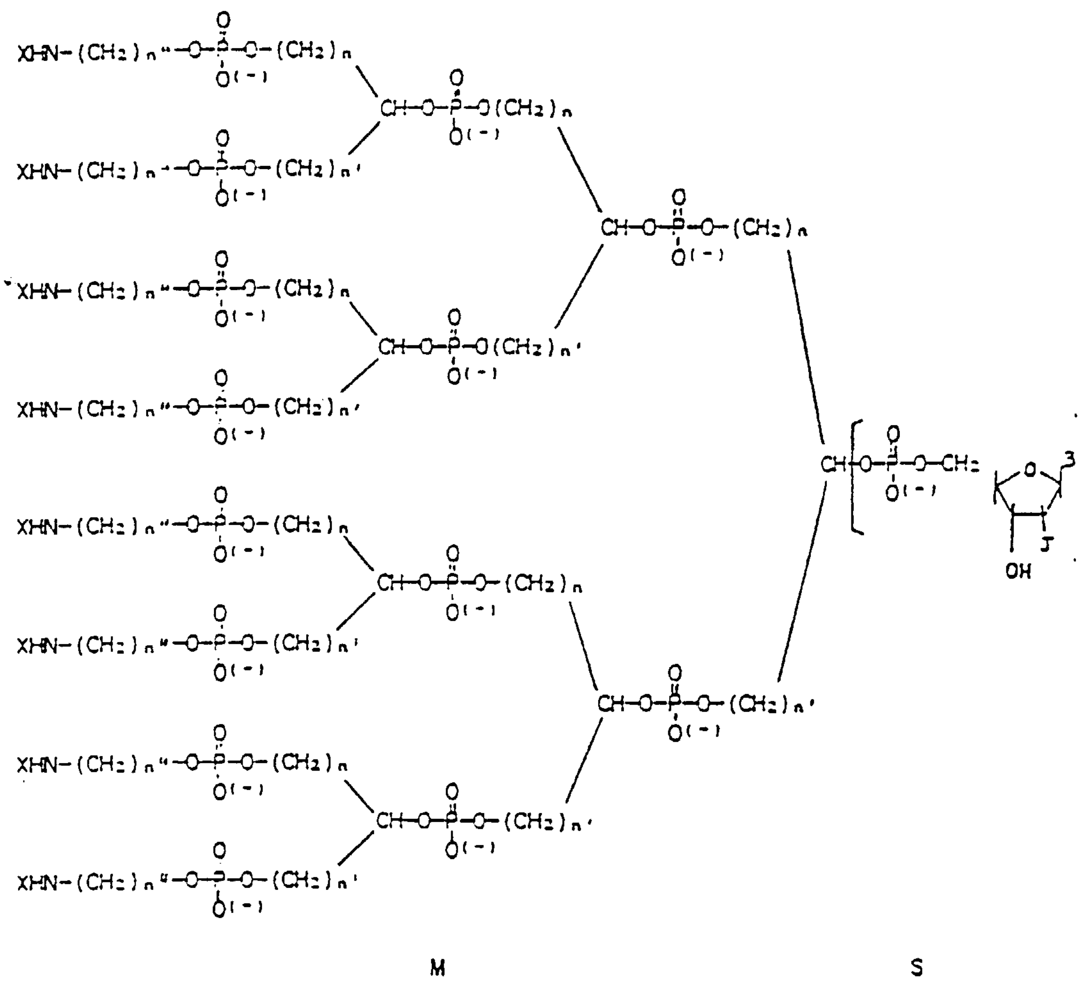

M S

XX

in der

in der Sequenz S,

J die Bedeutung H oder OH hat,

B eine Nuklein- Purin- oder Pyrimidin-säurebase bedeutet, je nach den Nukleotiden veränderbar,
x die Zahl der Nukleotide von 1 bis 1.000 darstellt,

n, n' und n" gleich 1 bis 20 sind,

M den Marker bedeutet, dessen Verzweigungsgrad Werte von 2 bis 128 annehmen kann, z.B. 8, wobei der höhere Grad immer das Doppelte des Vorhergehenden ist;

X den eigentlichen Marker bezeichnet, der entweder ein direkter Marker sein kann, wobei er aus den Enzymen wie alkalischer Phosphatase, Peroxidase und Fluorescein ausgewählt ist, die in der Lage sind, in Gegenwart eines Substrats eine Färbung oder Licht zu erzeugen,
oder ein indirekter Marker sein kann, wobei er aus den Haptenen, wie Biotin oder Digoxigenin ausgewählt ist, die in der Lage sind, durch nichtisotopisch markierte Antikörper erkannt zu werden.

**7.** Verfahren zur Herstellung von Sonden der Formel Ia, wie in Anspruch 4 definiert, umfassend

A1) die Synthese einer Nukleinsäuresequenz S

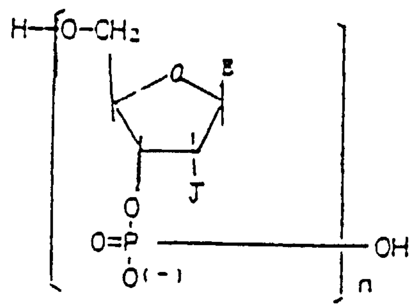

in der B und J und n die in Anspruch 4 angegebenen Bedeutungen haben,
durch jedes bekannte manuelle oder automatische Internukleotidverbindungs-Syntheseverfahren, bevorzugt auf einem festen Träger,
und
A2) die Fixierung eines Markers,

dadurch gekennzeichnet, daß für die Fixierung des Markers die Sequenz, vorzugsweise nach demselben Syntheseverfahren, insbesondere auf einem festen Träger,

entweder einer Verlängerung an ihrem 5'(OH)-Ende durch eine Reihe von Einheiten M und L

oder einer Verlängerung an ihrem 3'(OH)-Ende durch eine Reihe von Einheiten M und L

oder einer Verlängerung an ihrem 5'(OH)- und ihrem 3'(OH)-Ende durch zwei Reihen von Einheiten M und L,

unterzogen wird,
wobei M und L wie in Anspruch 4 definiert sind,

B) die Einheiten L jeweils durch Synthese eines nichtnukleotiden Polymers

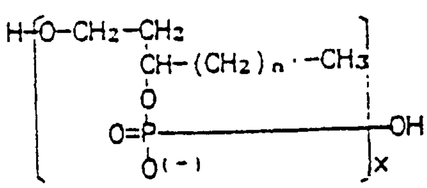

erhalten werden, wobei n' und x die in Anspruch 4 angegebenen Bedeutungen haben,

C) die Einheiten M jeweils durch Synthese eines nichtnukleotiden Polymers

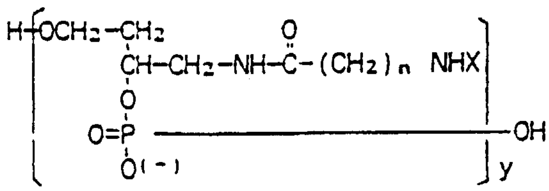

erhalten werden, wobei n und y die in Anspruch 4 angebenen Bedeutungen haben, und

X entweder einen Marker wie alkalische Phosphatase, Peroxidase, Fluorescein, Biotin, Digoxigenin, jedes Hapten, das durch nichtisotopisch markierte Antikörper erkennbar ist, oder eine Schutzgruppe für die primäre Aminfunktion, die nach der Gesamtsynthese der Nukleinsäuresonde entfernt wird, zum Beispiel eine Acetylgruppe (VI), eine Trifluoracetylgruppe (VII) oder eine Benzoylgruppe (VIII):

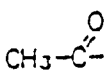

VI

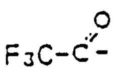

VII

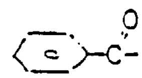

VIII

darstellt,

und die obigen Synthesen B und C insbesondere durch jedes bekannte manuelle oder automatische Internukleotidverbindungs-Syntheseverfahren, bevorzugt auf einem festen Träger, durchgeführt werden.

**8.** Verfahren nach Anspruch 7 zur Herstellung einer Sonde, aufgebaut durch eine Oligonukleotidkette, die bei 5'(OH) ein Geflecht chemischer Zweige, die von Aminzweigen unterbrochen sind, trägt, verwendbar für den Nachweis von ADN-Sequenzen durch nichtradioaktive Verfahren, gekennzeichnet durch die Verfahrensschritte

- Synthetisieren einer Oligonukleotidkette, deren Phosphatfunktionen und Basen geschützt sind, auf einem festen Träger;
- Kondensieren einer Reihe von Entwicklungsderivaten für chemische Zweige und Aminzweige am 5'(OH)-Ende der Kette unter Verwendung der gleichen Kondensationszyklen, wie für die Oligonukleotidkettensynthese;
- Abtrennen der 5'-modifizierten Oligonukleotidkette von ihrem festen Träger;
- Entfernen des Schutzes der Basen und Phosphate der Kette;
- Einführen des Teils, der die für den Nachweis verantwortliche Reaktivität bereitstellt, an den primären Amingruppen.

**9.** Verfahren nach Anspruch 7 zur Herstellung einer Sonde, aufgebaut durch eine Oligonukleotidkette, die bei 3'(OH) ein Geflecht chemischer Zweige, die von Aminzweigen unterbrochen sind, trägt, verwendbar für den Nachweis von ADN-Sequenzen durch nichtradioaktive Verfahren, gekennzeichnet durch die nachfolgenden Verfahrensschritte

- Kondensieren von Entwicklungsderivaten für chemische Zweige und Aminzweige auf einem festen Träger, nach gemäß den gleichen Kondensationszyklen, wie für die Nukleinsäuresynthese, um eine gewünschte Sequenz von chemischen Zweigen und Aminzweigen zu bilden,
- Aufbauen einer Oligonukleotidkette anschließend an diese Sequenz, um so eine 3'(OH)-modifizierte Oligonukleotidkette zu erhalten, deren Phosphatfunktionen und Basen geschützt sind,
- Abtrennen dieser modifizierten Kette vom festen Träger,
- Entfernen des Schutzes der Phosphate und Basen,

- Einführen des Teils, der die für den Nachweis verantwortliche Reaktivität bereitstellt, an den primären Aminfunktionen.

**10.** Verfahren nach Anspruch 7 zur Herstellung von Sonden mit einer Oligodesoxyribonukleotidkette, die bei 3'(OH) und bei 5'(OH) ein Geflecht chemischer Zweige, die von Aminzweigen unterbochen sind, trägt, verwendbar für den Nachweis von ADN-Sequenzen durch nichtradioaktive Verfahren, gekennzeichnet durch die Verfahrensschritte

- Kondensieren von Entwicklungsderivaten für chemische Zweige und Aminzweige auf einem festen Träger gemäß den gleichen Kondensationszyklen, wie für die Nukleinsäuresynthese, um eine gewünschte Sequenz von chemischen Zweigen und Aminzweigen zu bilden,
- Aufbauen einer an ihrem modifizierten 3'(OH)-Ende fixierten Oligonukleotidkette anschließend an diese Sequenz, deren Phosphatfunktionen und Basen geschützt sind,
- Aufbauen einer Sequenz aus chemischen Zweigen und Aminzweigen am 5'(OH)-Ende der Oligonukleotidkette,
- Abtrennen der bei 3'(OH) und bei 5'(OH) modifizierten Oligonukleotidkette von ihrem festen Träger, Entfernen des Schutzes der Phosphate und Basen und
- Einführen des Teils, der die für den Nachweis verantwortliche Reaktivität bereitstellt, an den primären Aminfunktionen.

**11.** Verfahren nach irgendeinem der Ansprüche 7 bis 10, dadurch gekennzeichnet, daß jedes Element des chemischen Zweigs L durch Kondensation einer Verbindunq der Formel

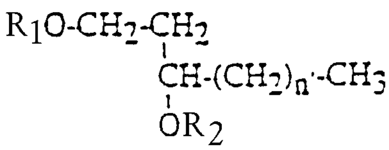

(II)

eingeführt wird, worin $R_1$ eine 4,4'-Dimethoxytritylgruppe (abgekürzt DMT) der Formel

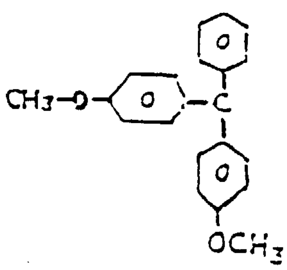

DMT (IV)

darstellt und worin $R_2$ eine phosphorylierte, gegebenenfalls geschützte Gruppe darstellt, die zur Einführung in eine Verbindung II (Synthon) am Ende eines Nukleotids oder eines bereits vorhandenen Synthons geeignet ist,

und insbesondere $R_2$ eine Diisopropylaminocyanoethoxyphosphin- oder Cyanoethoxydiisopropylamino-phosphoramidit-Gruppe ist mit der Formel

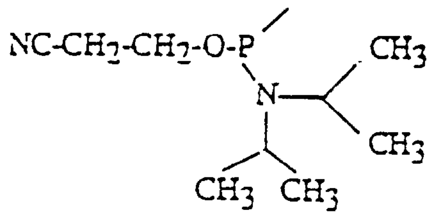

(V)

**12.** Verfahren nach irgendeinem der Ansprüche 7 bis 11, dadurch gekennzeichnet, daß jeder Marker oder jede Nachweiseinheit M ausgehend von 3-Buten-1-ol der Formel

$$HO\text{-}CH_2\text{-}CH_2\text{-}CH{=}CH_2 \qquad \text{(XXX)}$$

erhalten wird durch die Schritte:

a) Schutz der primären Alkoholfunktion mittels einer Schutzgruppe $R_1$, um eine Verbindung der Formel

$$R_1\text{-}O\text{-}CH_2\text{-}CH_2\text{-}CH{=}CH_2 \qquad \text{(XXXI)}$$

zu erhalten,

b) Epoxidierung der Doppelbindung der Verbindung (XXXI) um eine Verbindung der Formel

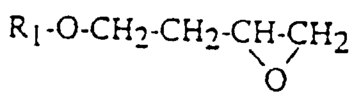

(XIIa)

zu bilden,

c) Öffnen des Epoxids mit Ammoniak, um eine Verbindung der Formel

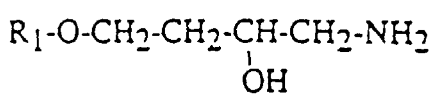

(XIIIa)

zu bilden,

d) Schutz der primären Amingruppe einer Verbindung der Formel

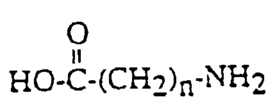

(XXXII)

um eine Verbindung der Formel

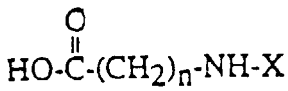

(XXXIII)

zu bilden, worin X die in Anspruch 7 angegebenen Bedeutungen hat,

e) Aktivierung der Verbindung (XXXIII),

f) Kondensation der Verbindungen (XIIIa) und (XXXIII) miteinander, um eine Verbindung der Formel

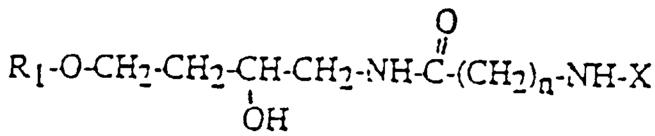

(XVa)

zu bilden, und

g) Phosphorylierung der Verbindung (XVa), um eine Verbindung der Formel

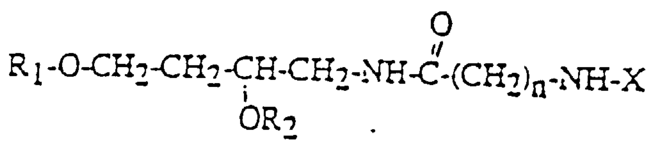

(III)

zu bilden, in der $R_2$ eine phosphorylierte Gruppe darstellt, die für die Einführung der Verbindung IIIa (Synthon) am Ende eines Nukleotids (3' und/oder 5') oder am Ende eines bereits vorhandenen Synthons geeignet ist.

**13.** Verfahren nach Anspruch 12, dadurch gekennzeichnet, daß

in Schritt a) $R_1$ 4,4'-Dimethoxytrityl (abgekürzt DMT) bedeutet und der Schutz der primären Alkoholfunktion mittels 4,4' -Dimethoxytritylchlorid (DMT-C1) durchgeführt wird, um eine Verbindung der Formel

$$DMTO\text{-}CH_2\text{-}CH_2\text{-}CH{=}CH_2 \quad (XXXIa)$$

zu erhalten,

in Schritt b) die Epoxidierung mit m-Chlorperbenzoesäure durchgeführt wird, um eine Verbindung der Formel

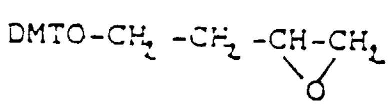

(XII)

zu bilden,

in Schritt c) die Öffnung des Epoxids mit Ammoniak durchgeführt wird, um eine Verbindung der Formel

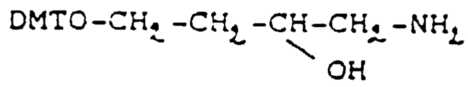

(XIII)

zu bilden,

in Schritt d) der Schutz der primären Aminogruppe der Verbindung (XXVI) mit einer Trifluoracetylgruppe durchgeführt wird, um eine Verbindung

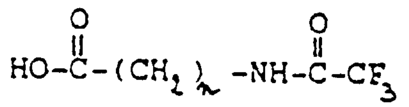

(XXXIIa)

zu bilden,

in Schritt e) die Verbindung (XXXIIa) mit N-Hydroxysuccinimid aktiviert wird, um die Verbindung

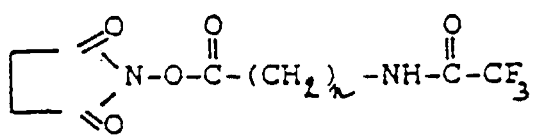

(XIVa)

zu bilden,

in Schritt f) die Verbindungen (XIII) und (XIVa) kondensiert werden, um eine Verbindunq der Formel

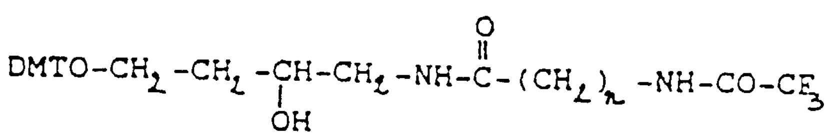

(XVb)

zu bilden,

in Schritt g) die Verbindung (XVb) phosphoryliert wird, um eine Verbindung der Formel

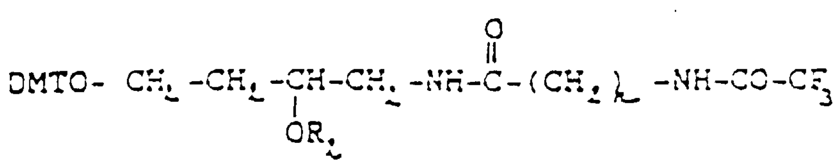

(IIIa)

zu erhalten, in der $R_3$ eine phosphorylierte, gegebenenfalls geschützte Gruppe darstellt, die zur Einführung der Verbindung oder des Synthons (IIIa) am Ende eines Nukleotids oder eines bereits auf einem für einen vorgegebenen Internukleotidverbindungs-Synthesetyp verwendbaren festen Träger kondensierten Synthons geeignet ist.

**14.** Verfahren nach Anspruch 13, das bei der Synthese der Phosphoramidite verwendet wird, dadurch gekennzeichnet, daß das im Schritt g) verwendete Phosphorylierungsmittel das Diisopropylaminocyanoethoxychlorphosphin der Formel

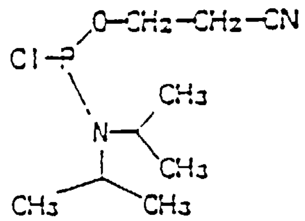

XI

ist, so daß in der am Ende dieses Schritts erhaltenen Verbindung der Formel III $R_2$ eine Diisopropylaminocyanoethoxyphosphino- oder Cyanoethoxydiisopropylaminophosphoramidit-Gruppe mit der Formel

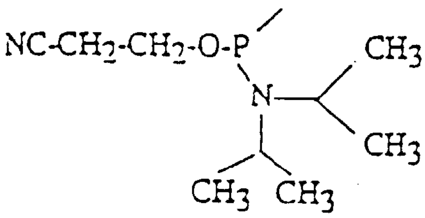

(V)

ist.

**15.** Verfahren nach Anspruch 16, dadurch gekennzeichnet, daß in den Formeln (XXXIIa, XIVa, XVb und IIIa) n gleich 5 ist.

**16.** Verfahren zur Herstellung von Sonden der Formel XX, wie sie in Anspruch 6 definiert sind, umfassend die Synthese einer Nukleinsäuresequenz S

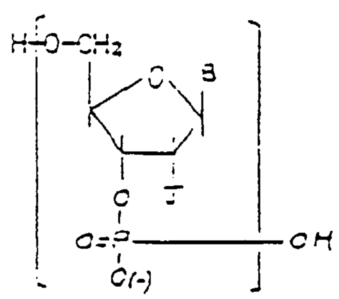

in der B und J die zuvor in Anspruch 4 angegebenen Bedeutungen haben, durch jedes bekannte manuelle oder automatische Internukleotidverbindungs-Syntheseverfahren, bevorzugt auf einem festen Träger,
und die Fixierung eines Markers,
dadurch gekennzeichnet, daß für die Fixierung des Markers die Sequenz, vorzugsweise mit demselben Syntheseverfahren, insbesondere auf einem festen Träger, an ihrem 5'(OH)-Ende durch einen Marker M, wie in Anspruch 6 definiert, verlängert wird, der eine verzweigte molekulare Struktur bildet, von der die Endzweige jeweils eine primäre Aminfunktion tragen.

**17.** Verfahren nach Anspruch 16 zur Herstellung einer Sonde, aufgebaut aus einer Oligonukleotidkette, die bei 5'(OH) ein verzweigtes Geflecht von Alkylketten, die in Aminketten enden, trägt, verwendbar für den Nachweis von ADN-Sequenzen durch nichtradioaktive Verfahren, gekennzeichnet durch die Verfahrensschritte

a) Synthetisieren einer Oligonukleotidkette, deren Phosphatfunktionen und Basen geschützt sind, auf einem festen Träger;
b) Kondensieren einer Reihe von Verbindungen der Formel

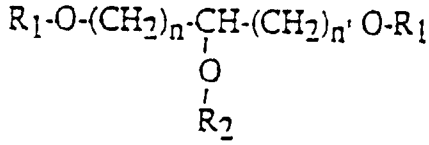

XXI

am 5'(OH)-Ende der Kette, worin $R_1$ 4,4'-Dimethoxytrityl (abgekürzt DMT) der Formel

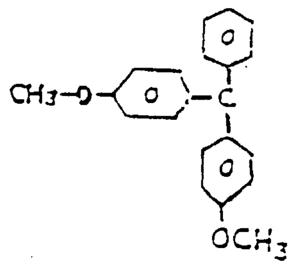

DMT (IV)

bedeutet, und $R_2$ eine phosphorylierte Gruppe darstellt, die zur Einführung der Verbindung XXI (Synthon) am Ende eines Nukleotids oder eines bereits vorhandenen Synthons geeignet ist, was zu n primären Alkoholstellen als Kondensationsstellen führt, und insbesondere $R_2$ eine Diisopropylaminocyanoethoxyphosphin- oder Cyanoethoxydiisopropylaminophosphoramidit-Gruppe ist mit der Formel

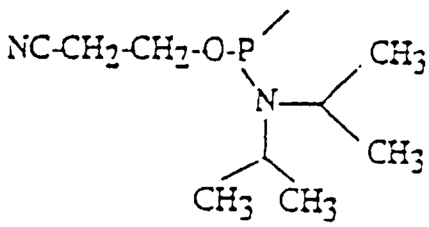

(V)

c) Durchführen einer Endkondensation über die erzeugten n Kondensationsstellen einer Verbindung der Formel

$$XHN\text{-}(CH_2)_{n''}\text{-}OR_2 \qquad \text{(XXII)}$$

in der

$R_2$ die oben angegebenen Bedeutungen hat und X entweder einen Marker: alkalische Phosphatase, Peroxidase, Fluorescein, Biotin, Digoxigenin, jedes Hapten, das durch nichtisotopisch markierte Antikörper erkennbar ist,
oder eine Übergangsschutzgruppe für die primäre Aminfunktion, die nach der Gesamtsynthese der Nukleinsäuresonde XX entfernt wird, darstellt; beispielsweise kann in diesem Syntheseansatz X ein Trifluoracetyl VII oder Fluorenylmethoxycarbonyl XXIII darstellen

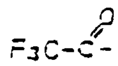

VII

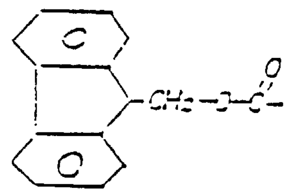

XXIII

wobei die Kondensationen der Verbindungen XXI und XXII dieselben Reaktionszyklen wie für die Oligonukleotidsequenzsynthese verwenden,

d) Entfernen des Schutzes der Basen und Phosphate der Oligonukleotidkette und

e) Einführen des Teils, der die für den Nachweis verantwortliche Reaktivität bereitstellt, auf der Ebene der primären Aminogruppen.

**18.** Verfahren nach Anspruch 17, dadurch gekennzeichnet, daß in der Verbindung XXIa n gleich 1, n' gleich 4 und $R_2$ eine Cyanoethoxydiisopropylaminophosphoramidit-Gruppe ist, so daß die hergestellte Verbindung XXIa der Formel

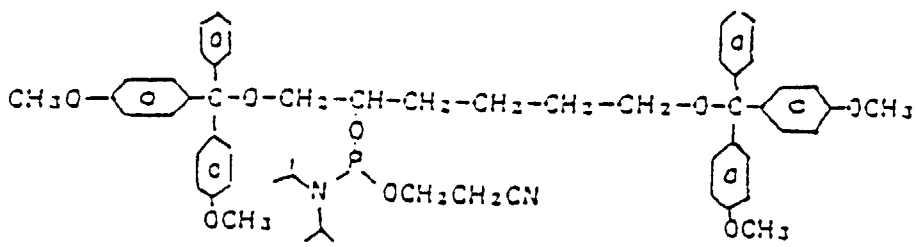

47

entspricht.

**19.** Verfahren nach Anspruch 17, dadurch gekennzeichnet, daß in der Verbindung XXII

die Gruppe $R_2$ eine Cyanoethoxydiisopropylaminophosphoramidit-Gruppe ist und
X eine Fluorenylmethoxycarbonylgruppe und
n" gleich 6 ist,

so daß die hergestellte Verbindung XXII der Formel

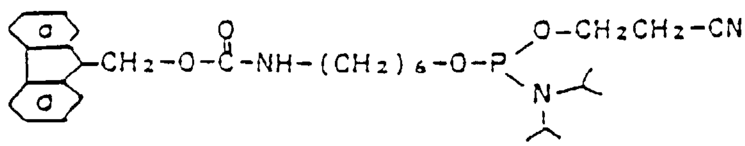

48

entspricht.

**20.** Verfahren nach irgendeinem der Ansprüche 7 bis 19, dadurch gekennzeichnet, daß im Falle von in 3'(OH) markierten Oligonukleotiden das CPG durch eine Verbindung der Formel

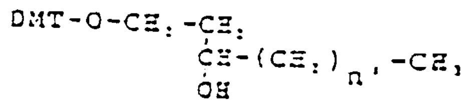

(X)

funktionalisiert ist, in der DMT und n' die in den Ansprüchen 7 bis 11 angegebenen Bedeutungen haben, so daß nach der Funktionalisierung der Träger der Formel

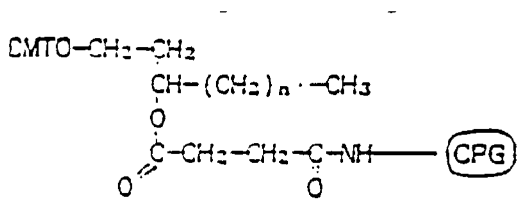

XIX

entspricht.

**21.** Als Zwischenprodukte für die Herstellung der Marker M verwendbare Verbindungen der Formel

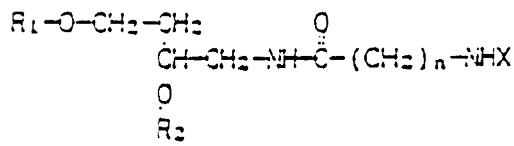

(III)

in der

n eine ganze Zahl von 2 bis 20 darstellt,
$R_1$ eine Schutzgruppe für die primäre Alkoholfunktion ist, und
$R_2$ H oder jede phosphorylierte, gegebenenfalls geschützte Gruppe darstellt, die zur Einführung des Synthons III am Ende eines Nukleotids oder eines bereits auf einem für einen vorgegebenen Internukleotidverbindungs-Synthesetyp verwendbaren festen Träger kondensierten Synthons geeignet ist,

und

X irgendeinen Marker oder eine Übergangsschutzgruppe für die primäre Aminfunktion, die nach der Gesamtsynthese der Nukleinsäuresonde entfernt wird, darstellt.

**22.** Verbindungen nach Anspruch 21, dadurch gekennzeichnet, daß

$R_1$ eine im sauren Milieu labile 4,4'-Dimethoxytritylgruppe (abgekürzt DMT) darstellt, und
$R_2$ eine Cyanoethoxydiisopropylaminophosphoramidit-Gruppe darstellt.

**23.** Verbindungen nach den Ansprüchen 21 und 22, dadurch gekennzeichnet, daß

X eine Acteyl-, Trifluoracetyl- oder Benzoylgruppe darstellt.

**24.** Verfahren zur Herstellung der Zwischenverbindungen der Formel III, wie in Anspruch 21 definiert, dadurch gekennzeichnet, daß es von einem 3-Buten-1-ol der Formel

$$HO\text{-}CH_2\text{-}CH_2\text{-}CH{=}CH_2 \qquad (XXX)$$

ausgeht, das nacheinander den Schritten: Schutz der primären Alkoholfunktion, Epoxidierung der Doppelbindung, Öffnung des Epoxids, Kondensationsreaktion mit einer Verbindung der Formel

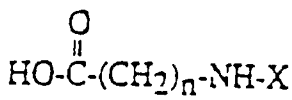

(XXXIII)

und Phosphorylierung des Kondensationsprodukts, wie in Anspruch 12 beschrieben, vorzugsweise mit den in den Ansprüchen 13 bis 15 beschriebenen Besonderheiten, unterzogen wird.

## Claims

1. A nucleic acid probe for detecting a DNA or RNA molecule, comprising:

   a) an oligonucleotide or oligodeoxynucleotide part constituted by

   S : a DNA or RNA nucleic acid sequence, depending on the type of molecule to be detected, and

   b) a non-nucleotide part possessing chemical properties which allow the direct or indirect attachment of one or more units of detection or marking elements M detectable in a non-isotopic manner by production of colour or light,

   characterised in that part b) is constituted by a chain of phosphate units interspersed with alkyl groups, viz.

   b1) certain alkyl groups uniting the different phosphate groups and presenting no special functionality

   b2) alkyl groups presenting primary amine groups which allow splicing with varied reagents to achieve direct or indirect detection,

   the b2) groups being bonded to part a) or sequence S via groups b1).

2. A nucleic acid probe according to claim 1,
   characterised in that part b) is in the form of a linear chain in which groups b1) form "chemical arms" L serving to introduce spacings between groups b2) which constitute the marking elements M proper.

3. A nucleic acid probe according to claim 2, satisfying the formula

$$[(M)y'\text{-}(L)x']z'\text{-}S\text{-}[(L)x\text{-}(M)y]z \qquad (I)$$

   in which S, L and M have the meanings given in claims 1 and 2, and

   x, x', z, z' are numbers equal to or higher than 0, with the proviso that
   z and z' are never simultaneously equal to 0,
   y and y' are numbers higher than 0.

4. A nucleic acid probe according to claim 3, characterised in that it satisfies the general formula Ia

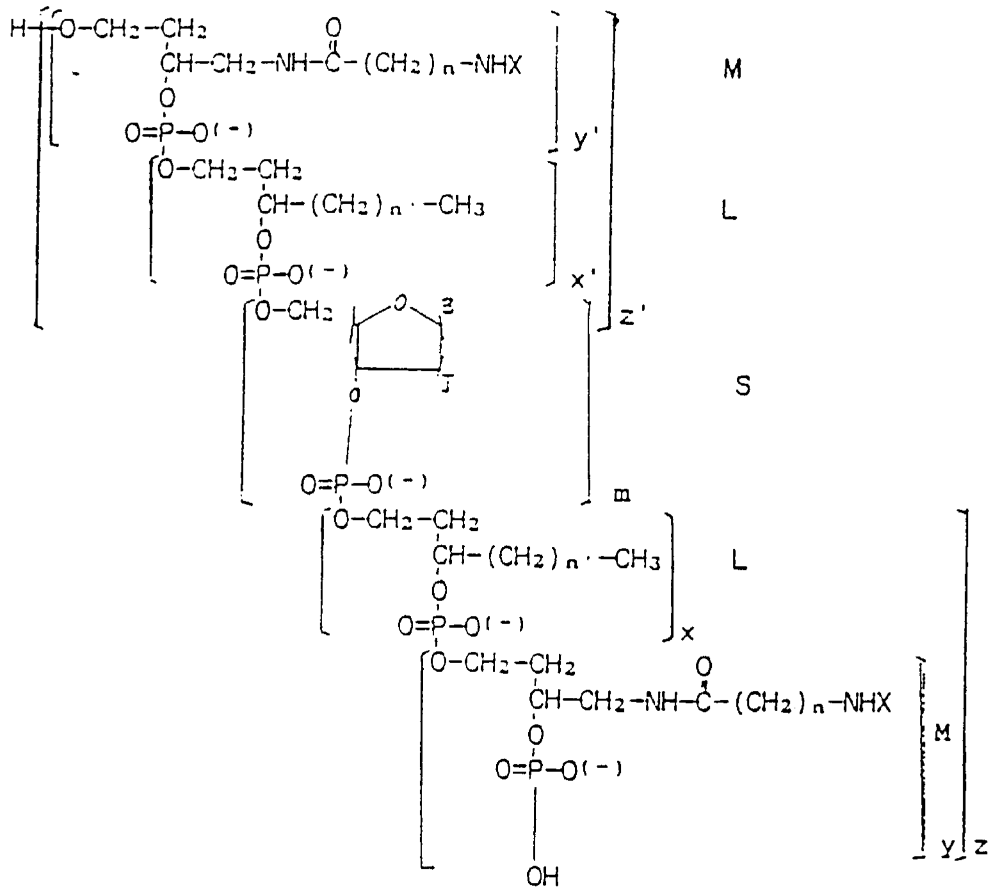

(Ia)

in which:

S, L and M have the meanings given in claims 1 and 2, and in sequence S,

J denotes H or OH
B denotes a nucleic acid, purine or pyrimidine acid base which may vary according to the nucleotides, and
m = 1 to 1000.

in sequence L

x and x' = 0 to 100
n' = 0 to 20

in sequence M

y and y'= 1 to 100
n = 2 to 20

X designates the marker proper, which may be:

either direct, in which case it is chosen from enzymes such as alkaline phosphatase, peroxydase and fluorescein, capable of producing a coloration or a light in the presence of a substrate,
or else indirect, in which case it is chosen from haptens such as biotin or digoxigenin capable of being recognised by non-isotopically labelled antibodies, where the element LM responsible for detection of probe may be introduced

at 5'(OH) for z' ≠ 0 and z = 0
or at 3'(OH) for z' = 0 and z ≠ 0
or simultaneously at 5'(OH) and at 3'(OH)
for z' ≠ 0 and z ≠ 0
z and z' generally being from 0 to 100.

5. A nucleic acid probe according to claim 1, characterised in that part b) forming the marking element is constituted by a branched chain of phosphate units interspersed with alkyl groups comprising groups b1), as groups internal to the branch, and groups b2) as groups external to the branch, in other words terminating the various arms of the branching arrangement.

6. A nucleic acid probe according to claim 5, characterised in that it satisfies the general formula XX

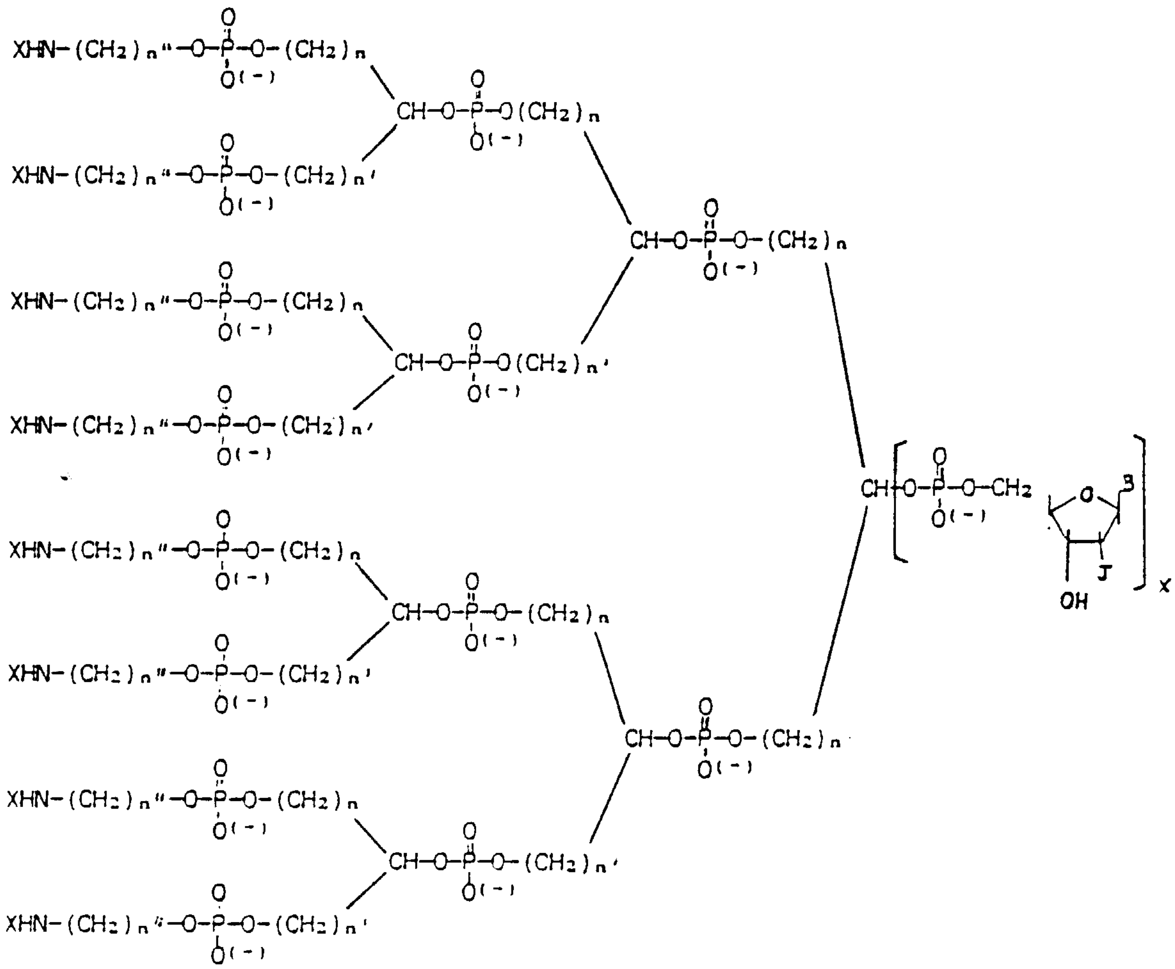

M S

XX

in which

in sequence S,
J denotes H or OH
B denotes a nucleic, purine or pyrimidine base, which may vary depending on the nucleotides,
x represents the number of nucleotides from 1 to 1000

n, n' and n" are numbers from 1 to 20

M denotes the marking element of which the branching order may assume values from 2 to 128, for example 8, the higher order always being double the preceding value;

X denotes the marker proper, which may be either direct, in which case it is chosen from enzymes such as alkaline phosphatase, peroxydase and fluorescein, capable of producing a coloration or a light in the presence of a substrate,
or else indirect, in which case it is chosen from haptens such as biotin or digoxigenin, capable of being recognised by non-isotopically labelled antibodies.

**7.** A method for preparing probes of formula Ia as defined in claim 4, comprising

A1) the synthesis of a nucleic acid sequence S

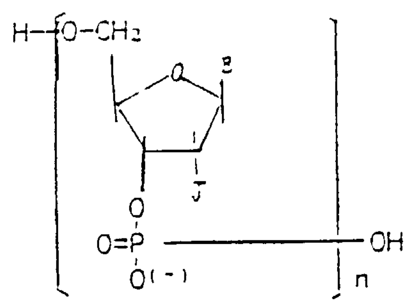

in which B and J and n have the meanings given in claim 4 by any known method of manual or automatic internucleotide linkage synthesis, preferably on a solid support,
and
A2) the attachment of a marker,

characterised in that for attaching the marker the said sequence is preferably subjected, by the same synthesis method, notably on a solid support,

either to an extension at its 5'(OH) extremity by a series of units M and L

or to an extension at its 3'(OH) extremity by a series of units M and L

or to an extension at its 5'(OH) and 3'(OH) extremities by two series of units M and L,
M and L being as defined in claim 4,

B) said units L are each obtained by synthesis of a non-nucleotide polymer

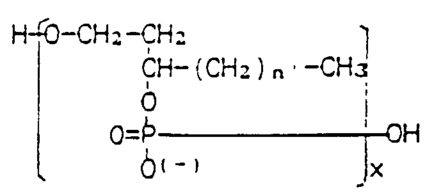

in which n' and x have the meanings given in claim 4,

C) the said units M are each obtained by synthesis of a non-nucleotide polymer

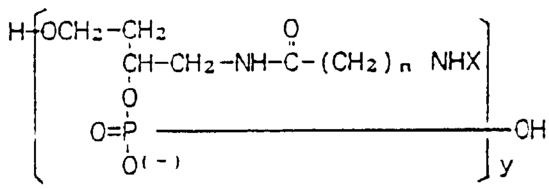

in which

n and y have the meanings given in claim 4 and
X represents either a marker such as alkaline phosphatase, peroxydase, fluorescein, biotin, digoxigenin, any hapten capable of being recognised by non-isotopically labelled antibodies, or else a protecting group of the primary amine group which it is intended to remove after total synthesis of the nucleic acid probe, for example an acetyl (VI), trifluoracetyl (VII) or benzoyl (VIII) group:

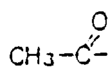

VI

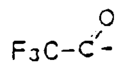

VII

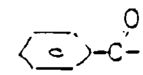

VIII

and syntheses B and C above are more particularly carried out by any known method of manual or automatic internucleotide linkage synthesis, preferably on a solid support.

**8.** The method according to claim 7 for preparing a probe constituted by an oligonucleotide chain bearing at 5'(OH) a tangle of chemical arms interspersed with aminated arms useful in the detection of DNA sequences by non-radioactive methods, characterised by the following operations:

- synthesising, on a solid support, an oligonucleotide chain whose phosphate groups and bases are protected;
- condensing at the 5'(OH) extremity of said chain a succession of derivatives generating chemical arms and aminated arms, employing the same condensation cycles as for the synthesis of the oligonucleotide chain;
- separating the modified 5' oligonucleotide chain from its solid support;
- deprotecting the bases and phosphates of the said chain;
- introducing at the primary amino groups the part that provides the reactivity responsible for detection.

**9.** The method according to claim 7, for preparing a probe constituted by an oligonucleotide chain bearing at 3'(OH) a tangle of chemical arms interspersed with aminated arms useful in detecting DNA sequences by non-radioactive methods, characterised by the following operations:

- condensing, on a solid support, derivatives generating chemical arms and aminated arms in accordance with the same condensation cycles as for the synthesis of nucleic acids, so as to form a desired sequence of chemical arms and aminated arms,
- building an oligonucleotide chain onto the said sequence, so as to thereby obtain a modified 3'(OH) oligonucleotide chain of which the phosphate groups and bases are protected,
- separating the said modified chain from the solid support,
- deprotecting the phosphates and bases,
- introducing at the primary amine groups the part that provides the reactivity responsible for detection.

**10.** The method according to claim 7, for preparing oligodeoxyribonucleotide chain probes bearing at 3'(OH) and at 5'(OH) a tangle of chemical arms interspersed with aminated arms useful in detecting DNA sequences by non-radioactive methods, characterised by the following operations:

- condensing, on a solid support, derivatives generating chemical arms and aminated arms on a solid support in accordance with the same condensation cycles as for the synthesis of nucleic acids, so as to form a desired

sequence of chemical arms and aminated arms,

- building onto the said sequence an oligonucleotide chain attaching to its modified 3'(OH) extremity of which the phosphate groups and bases are protected,
- constructing a sequence of chemical arms and aminated arms at the 5'(OH) end of the oligonucleotide chain,
- separating the oligonucleotide chain modified at 3'(OH) and 5'(OH) from its solid support, deprotecting the phosphates and bases, and
- introducing at the primary amine groups the part that provides the reactivity responsible for detection.

**11.** The method according to any of claims 7 to 10, characterised in that each element of chemical arm L is introduced by condensing a compound of formula

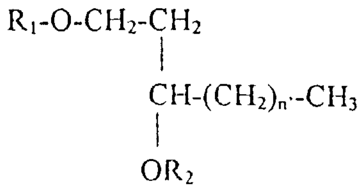

(II)

where $R_1$ represents a 4,4'-dimethoxytrityl group (abbreviated to DMT) of formula

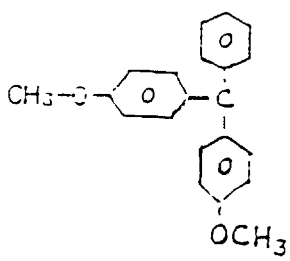

DMT (IV)

where $R_2$ represents a phosphorylated group, possibly protected, suited to the introduction of compound II (synthon) at the end of a nucleotide or else of a pre-existing synthon

and more particularly $R_2$ is a diisopropylaminocyanoethoxyphosphine or cyanoethoxydiisopropylamino-phosphoramidite group of formula

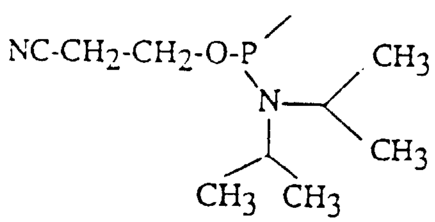

(V)

**12.** The method according to any of claims 7 to 11, characterised in that each detection element or unit M is obtained starting from butene-3ol-1 of formula

$$HO-CH_2-CH_2-CH=CH_2 \qquad (XXX)$$

by the steps of

a) protecting the primary alcohol group by means of a protecting group $R_1$ so as to obtain a compound having the formula

$$R_1\text{-O-}CH_2\text{-}CH_2\text{-}CH{=}CH_2 \qquad \text{(XXXI)}$$

b) epoxidation of the double bond of compound (XXXI) so as to form a compound of formula

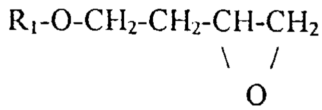

(XIIa)

c) opening of the epoxide by means of ammonia to form a compound of formula

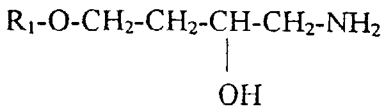

(XIIIa)

d) protecting the primary amine group by a compound of formula

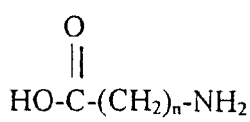

(XXXII)

so as to form a compound of formula

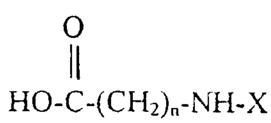

(XXXIII)

wherein X has the meanings given in claim 7,

e) activation of compound (XXXIII),

f) condensation of compounds (XIIIa) and (XXXIII) between themselves to form a compound of formula

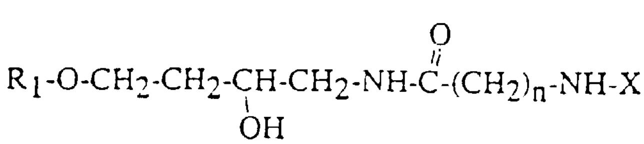

(XVa)

and

g) phosphorylation of compound (XVa) so as to form a compound of formula

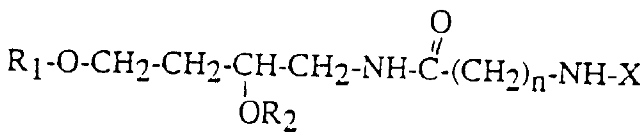

(III)

wherein $R_2$ represents a phosphorylated group suited to the introduction of compound IIIa (synthon) at the end (3' and/or 5') of a nucleotide or alternatively at the end of a synthon already in existence.

**13.** The method according to claim 12, characterised in that

at stage a) $R_1$ denotes 4,4'-dimethoxytrityl (abbreviated as DMT) and the protection of the primary alcohol group is carried out by means of 4,4'-dimethoxytrityl chloride (DMT-Cl) so as to obtain a compound of formula

$$DMTO\text{-}CH_2\text{-}CH_2\text{-}CH{=}CH_2 \qquad (XXXIa)$$

at stage b) epoxidation is carried out using metachloroperbenzoic acid so as to form a compound of formula

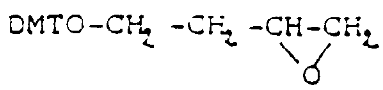

(XII)

at stage c) the opening of the epoxide is carried out using ammonia so as to form a compound of formula

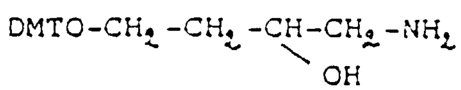

(XIII)

at stage d) the protection of the primary amine group of compound (XXVI) is carried out using a trifluoroacetyl group so as to form a compound

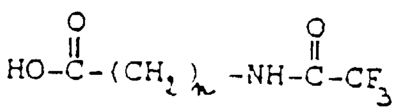

(XXXIIa)

at stage e) compound (XXXIIa) is activated using N-hydroxysuccinimide to as to form the compound

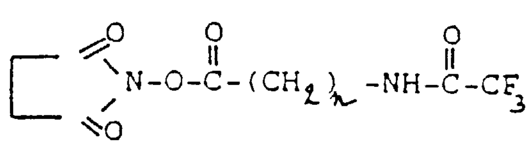

(XIVa)

at stage f) compounds (XIII) and (XIVa) are condensed so as to form a compound of formula

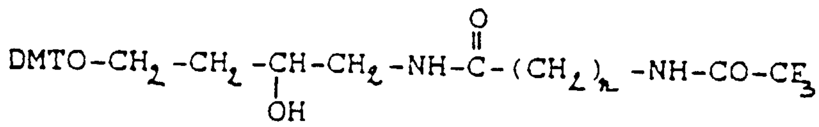

(XVb)

at stage g) compound (XVb) is phosphorylated so as to obtain a compound of formula

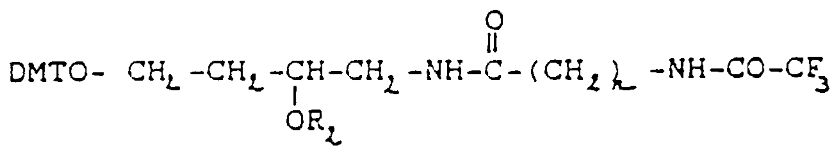

(IIIa)

in which $R_2$ represents a phosphorylated group, protected if necessary, suited to the introduction of the compound or synthon (IIIa) at the end of a nucleotide or else of a synthon already condensed on a solid support appropriate for a given type of internucleotide linkage synthesis.

**14.** The method according to claim 13, when applied to synthesis with phosphoramidites, characterised in that the phosphorylating agent used in stage g) is diisopropylaminocyanoethoxychlorophosphine of formula

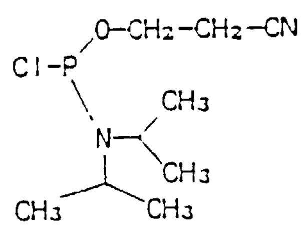

XI

such that in the compound of formula III obtained at the outcome of this stage, $R_2$ is a diisopropylaminocyanoethoxyphosphino or cyanoethoxydiisopropylaminophosphoramidite group of formula

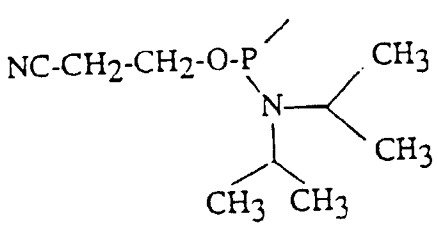

(V)

**15.** The method according to claim 16, characterised in that in formulas (XXXIIa, XIVa, XVb and IIIa) n equals 5.

**16.** A method for preparing probes of formula XX as defined in claim 6, comprising the synthesis of a nucleic acid sequence S

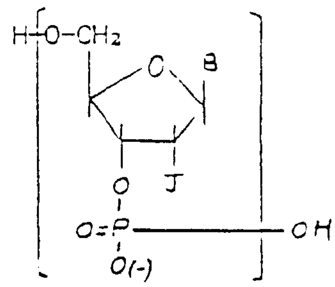

in which B and J have the meanings given previously in claim 4, by any known method of automatic or manual internucleotide linkage synthesis, preferably on a solid support,
and the attachment of a marker,
characterised in that for attaching the marking element, said sequence is subjected, preferably by the same method of synthesis, namely on a solid support, to an extension at its 5'(OH) end by a marking element M as defined in claim 6, forming a branched molecular structure whose terminal arms each carry a primary amino group.

**17.** The method according to claim 16 for preparing a probe constituted by an oligonucleotide chain bearing at 5'(OH) a branched tangle of alkyl chains terminating in aminated chains useful in the detection of DNA sequences by non-radioactive methods, characterised by the following operations:

a) synthesising on a solid support an oligonucleotide chain of which the phosphate groups and the bases are protected;
b) condensing at the 5'(OH) end of said chain a series of compounds of formula

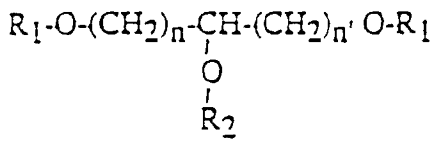

XXI

wherein $R_1$ denotes 4,4'-dimethoxytrityl (abbreviated as DMT) of formula

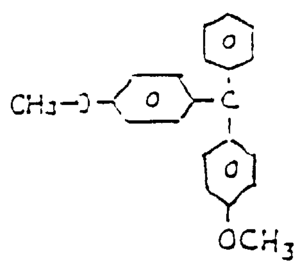

DMT (IV)

and $R_2$ represents a phosphorylated group suited to the introduction of compound XXI (synthon) at the end of a nucleotide or of a pre-existing synthon, which gives rise to n condensation sites being primary alcohol sites, and more particularly $R_2$ is a diisopropylaminocyanoethoxyphosphine or cyanoethoxydiisopropylamino-phosphoramidite group of formula

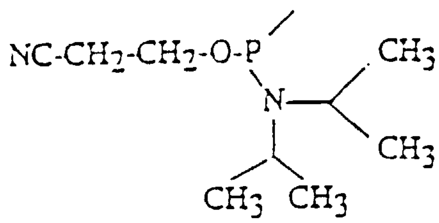

(V)

c) on each of the n primary alcohol sites created, effecting final condensation of a compound of formula

$$XHN\text{-}(CH_2)_{n''}\text{-}OR_2 \qquad (XXII)$$

in which

$R_2$ has the meanings given above, and X represents either a marker : alkaline phosphatase, peroxydase, fluorescein, biotin, digoxigenin, any hapten capable of being recognised by non-isotopically marked antibodies

or else a transitory group protecting the primary amine intended to be removed after total synthesis of the nucleic acid probe XX; by way of example, in this synthesis procedure X may represent a trifluoroacetyl VII, a fluorenylmethoxycarbonyl XXIII

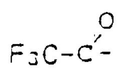

VII

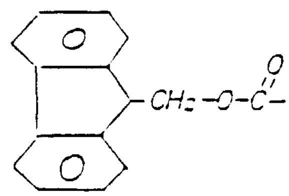

XXIII

the condensations of compounds XXI and XXII employing the same reaction cycles as for the synthesis of the oligonucleotide sequence,

d) deprotecting the bases and phosphates of said oligonucleotide chain, and

e) introducing at the primary groups the part that provides the reactivity responsible for detection.

**18.** The method according to claim 17, characterised in that in compound XXIa n equals 1, n' equals 4 and $R_2$ is a cyanoethoxydiisopropylaminophosphoramidite group, with the result that the compound XXIa employed satisfies the formula

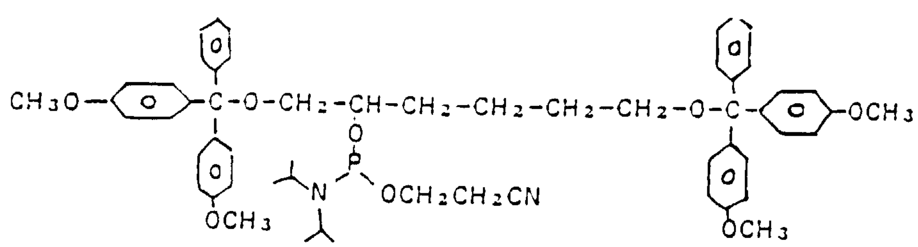

47

**19.** The method according to claim 17, characterised in that in compound XXII,

group $R_2$ is a cyanoethoxydiisopropylaminophosphoramidite group and

X is a fluorenylmethoxycarbonyl group and

n" equals 6

with the result that compound XXII employed satisfies the formula

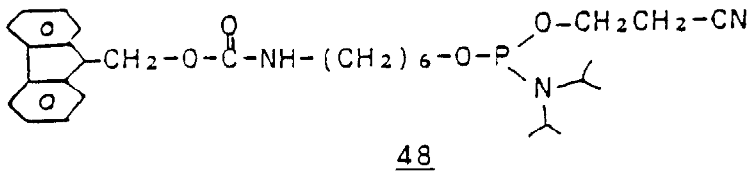

48

**20.** The method according to any of claims 7 to 19, characterised in that in the case of oligonucleotides labelled at 3' (OH), the CPG is functionalised by a compound of formula

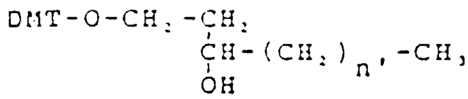

(X)

in which DMT and n' have the meanings given in claims 7 and 11, with the result that, after functionalisation, the support satisfies the formula

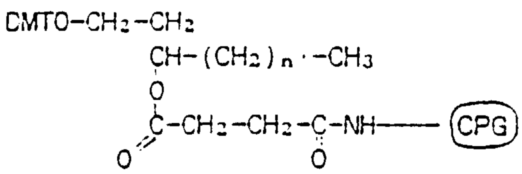

XIX

**21.** As intermediates useful for the preparation of marking units M, the compounds of formula

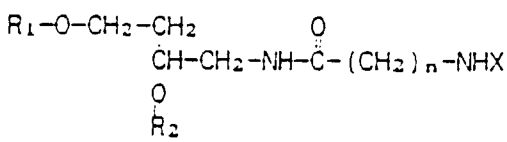

(III)

in which

n represents an integer from 2 to 20,
$R_1$ is a group protecting the primary alcohol group and
$R_2$ represents H or any phosphorylated group, protected if necessary, suited to the introduction of synthon III at the end of a nucleotide or of a synthon already condensed on a solid support for a given type of internucleotide linkage synthesis

and

X represents all the markers, i.e. a transitory protecting group of the primary amine function intended to be removed after total synthesis of the nucleic acid probe.

**22.** Compounds according to claim 21, characterised in that

$R_1$ represents a 4,4'-dimethoxytrityl group (abbreviated as DMT) labile in acid medium

and

$R_2$ represents a cyanoethoxydiisopropylaminophosphoramidite group.

**23.** Compounds according to claims 21 and 22, characterised in that

X represents a trifluoroacetyl or benzoyl group.

**24.** A method for preparing intermediate compounds of formula III as defined in claim 21, characterised in that it proceeds from butene-3-ol-1 of formula

$$HO-CH_2-CH_2-CH=CH_2 \qquad (XXX)$$

which is successively subjected to the stages of protecting the primary alcohol group, epoxidation of the double bond, opening of the epoxide, condensation reaction with a compound of formula

$$HO-\overset{O}{\overset{\|}{C}}-(CH_2)_n-NH-X \qquad (XXXIII)$$

and of phosphorylation of the condensation product, as described in claim 12 with, preferably, the special features described in claims 13 to 15.

Réactivité du bras aminé :

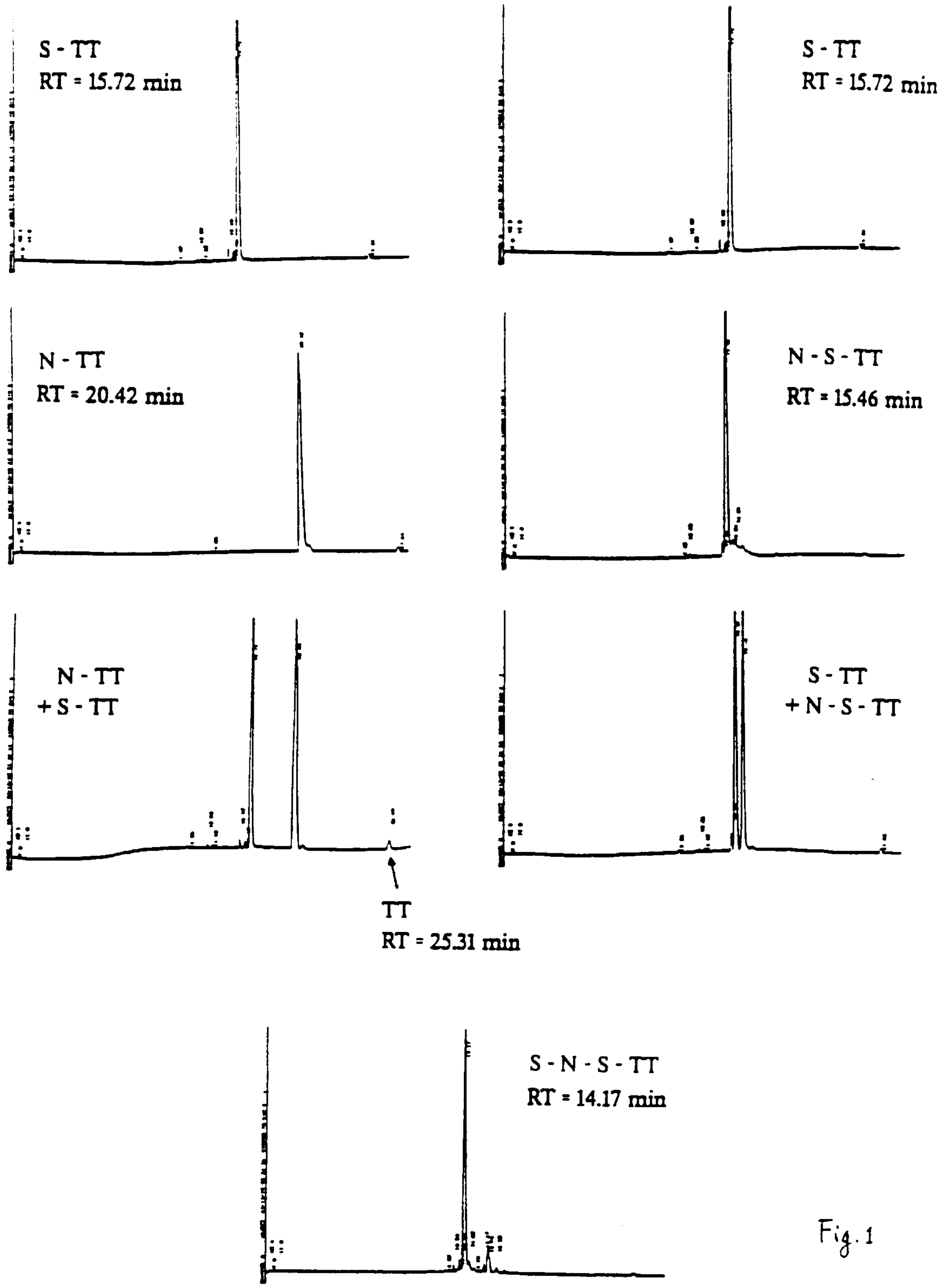


Fig. 1

# Oligo 23 mer avec bras aminés en 5' OH

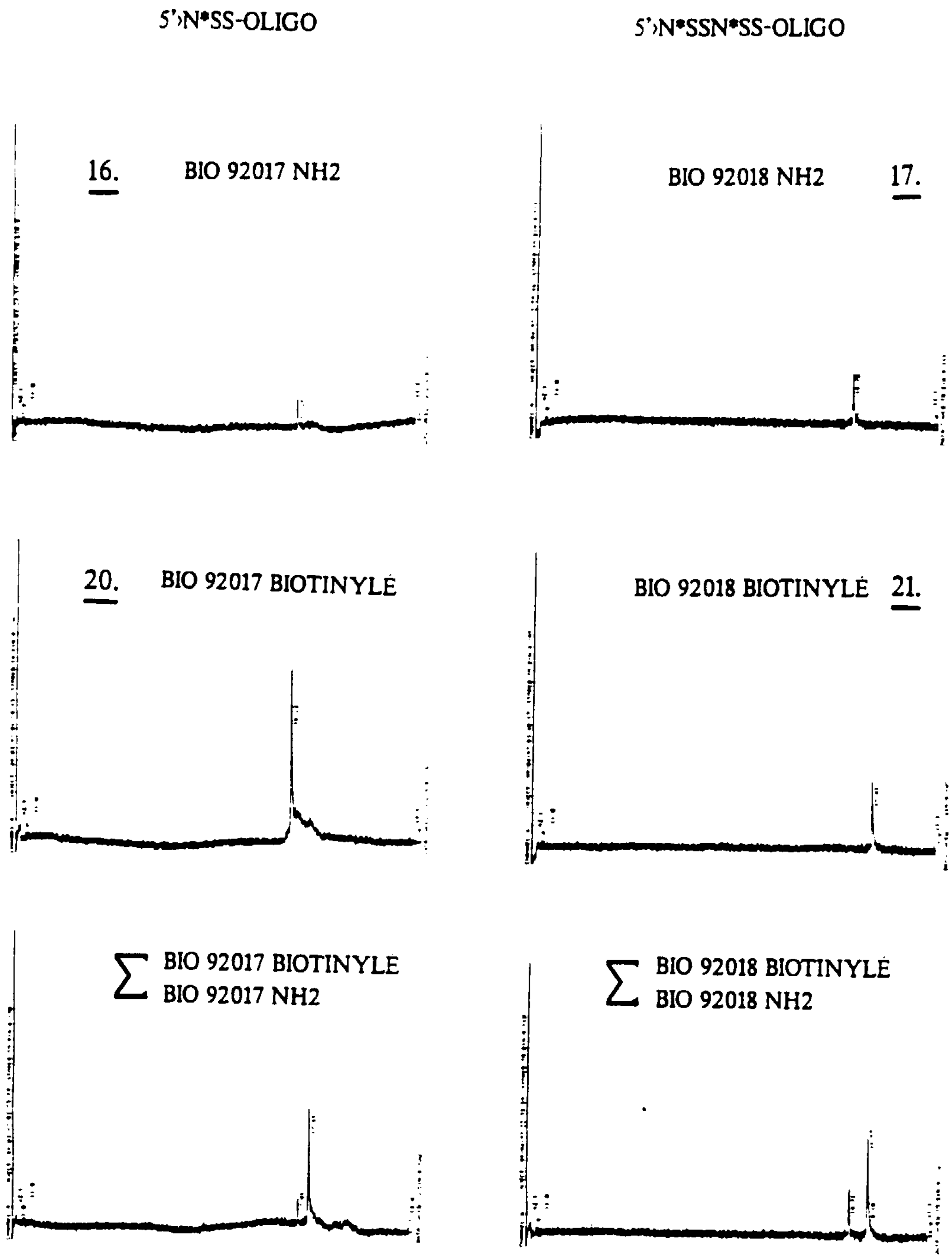


Fig. 2a

# Oligo 23 mer avec bras aminés en 5' OH

5'›N•SSN•SSN•SS-OLIGO

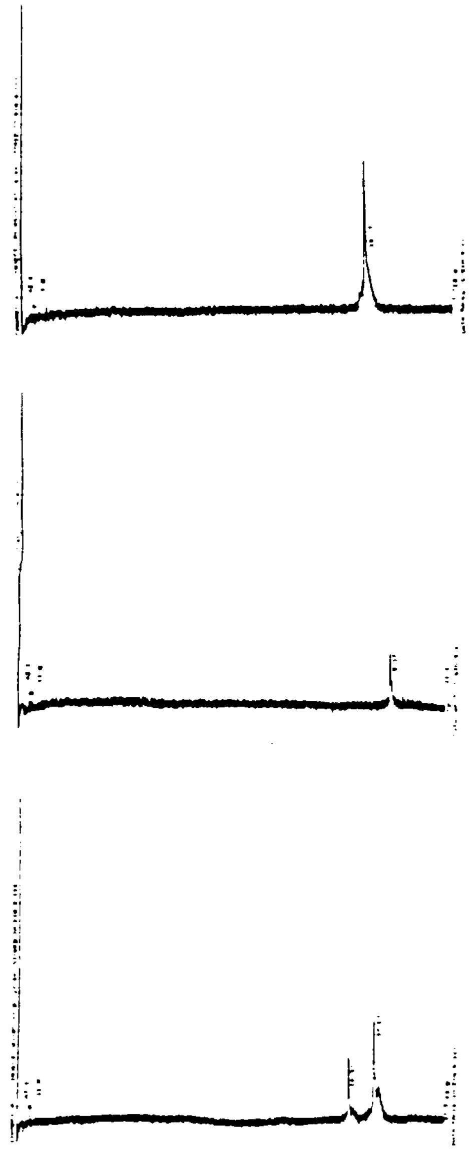

BIO 92019 NH2

18.

BIO 92019 BIOTINYLE

22.

Σ BIO 92019 BIOTINYLE
BIO 92019 NH2

Fig. 2b

ESSAIS AVEC CPG FONCTIONNALISE AVEC "S",
BRAS "N" A 0,1 M

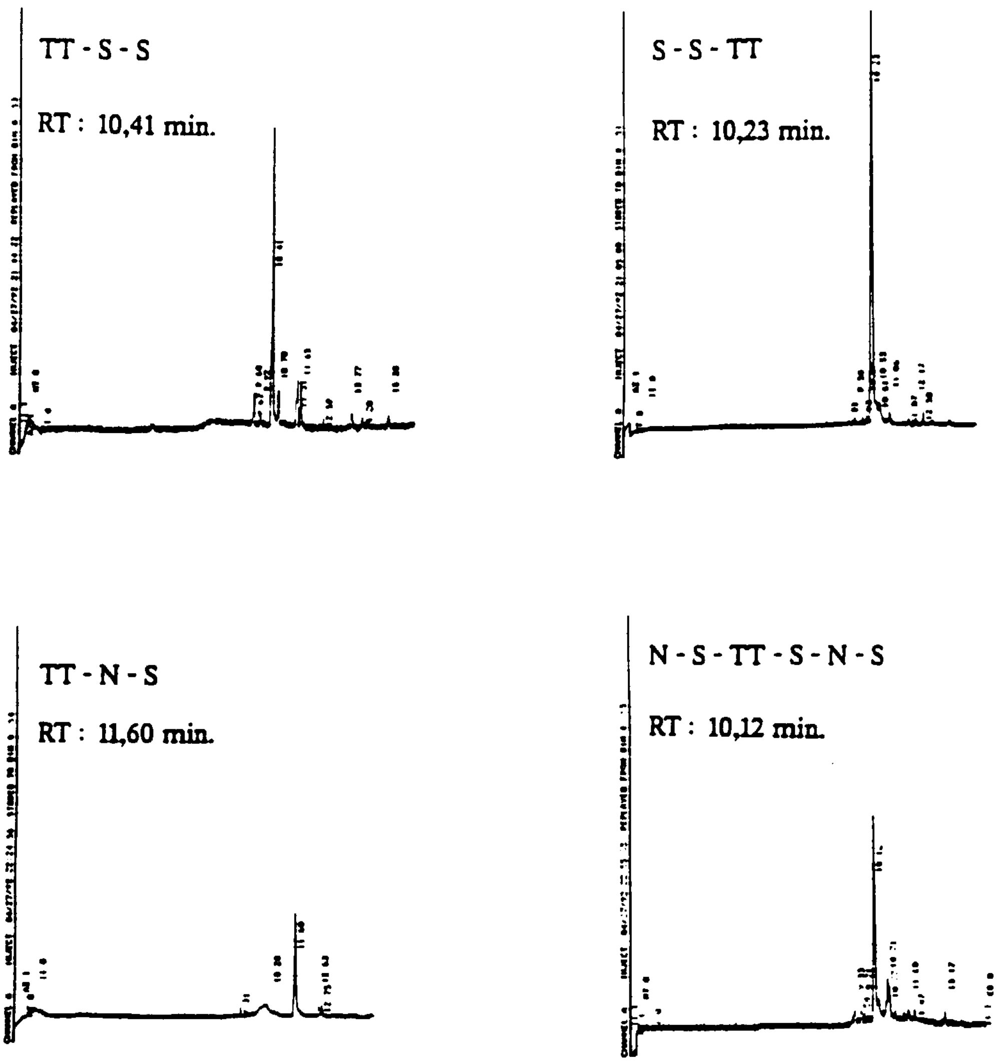


Fig. 3

# Oligonucléotides biotinylés en 3'OH

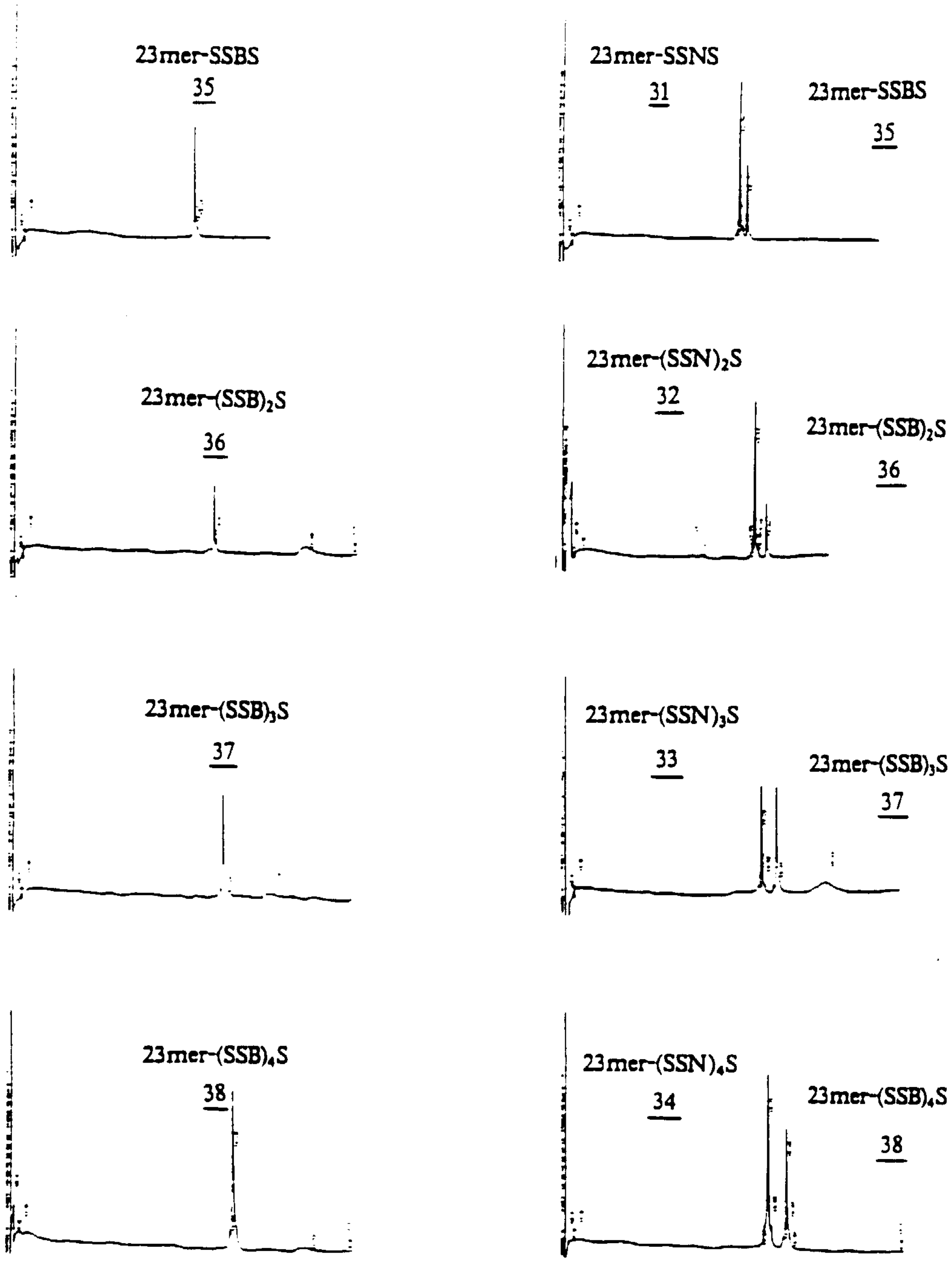


Fig. 4

# Oligonucléotides biotinylés en 3' et 5' OH

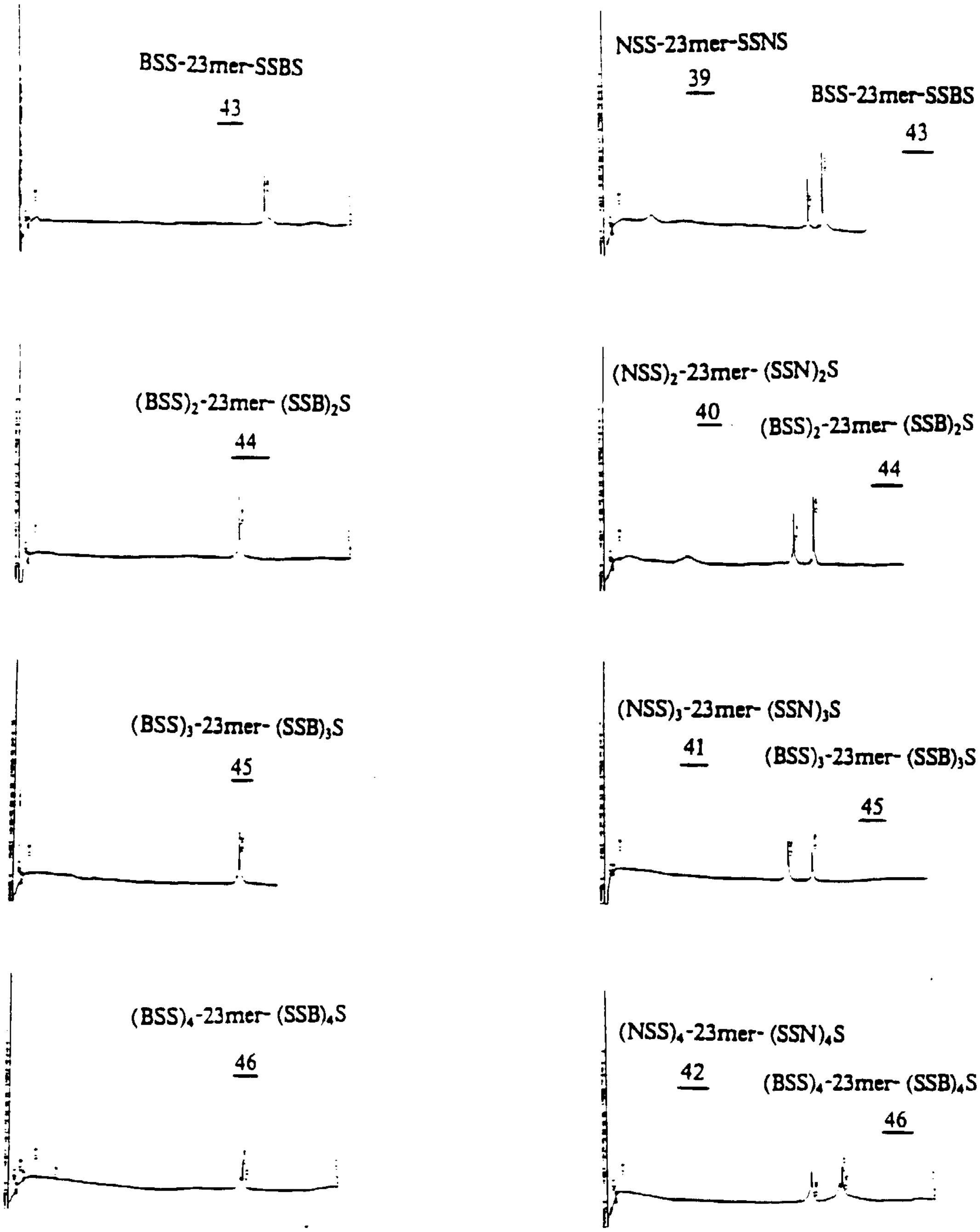


Fig. 5

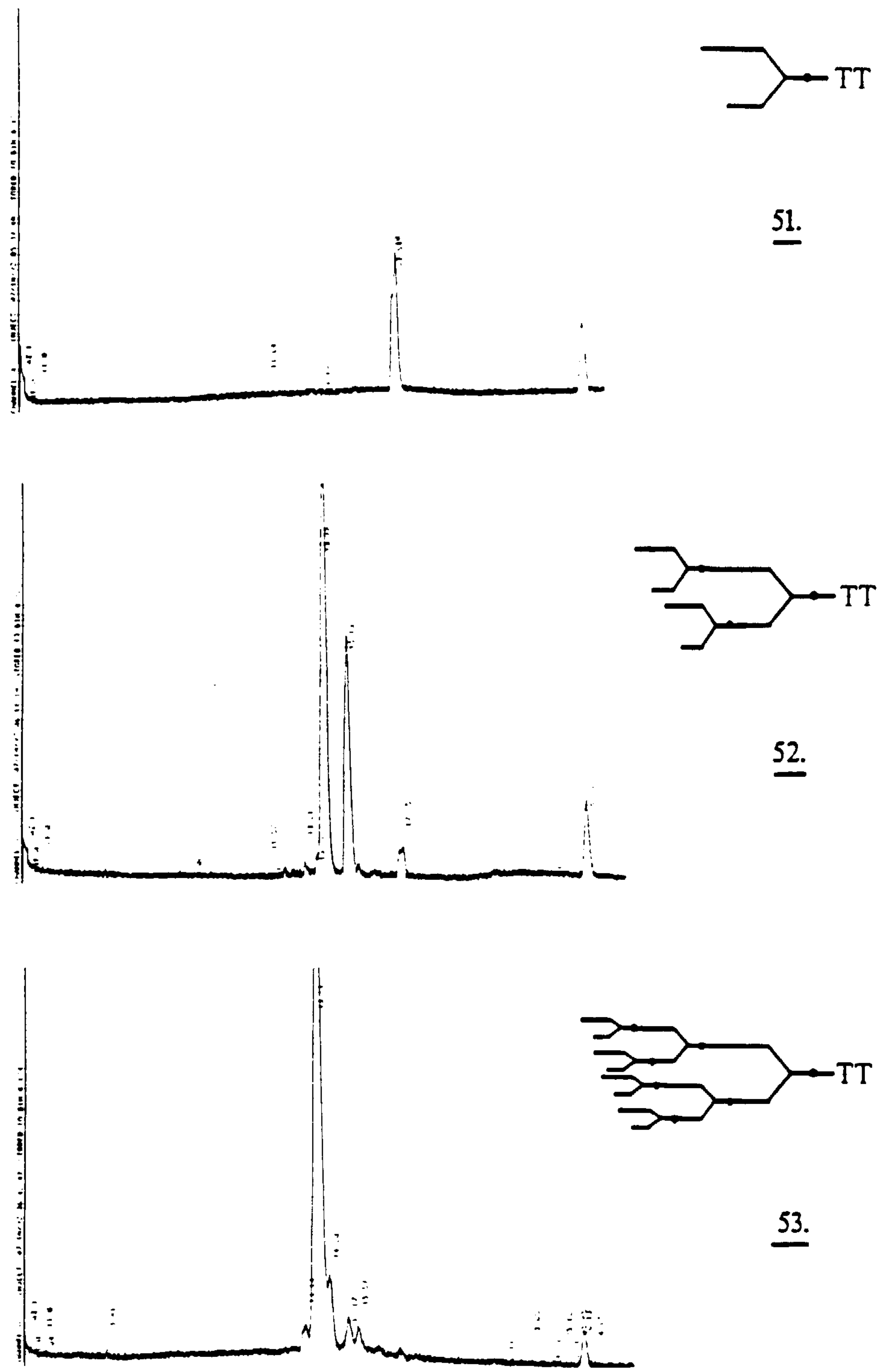


Fig. 6

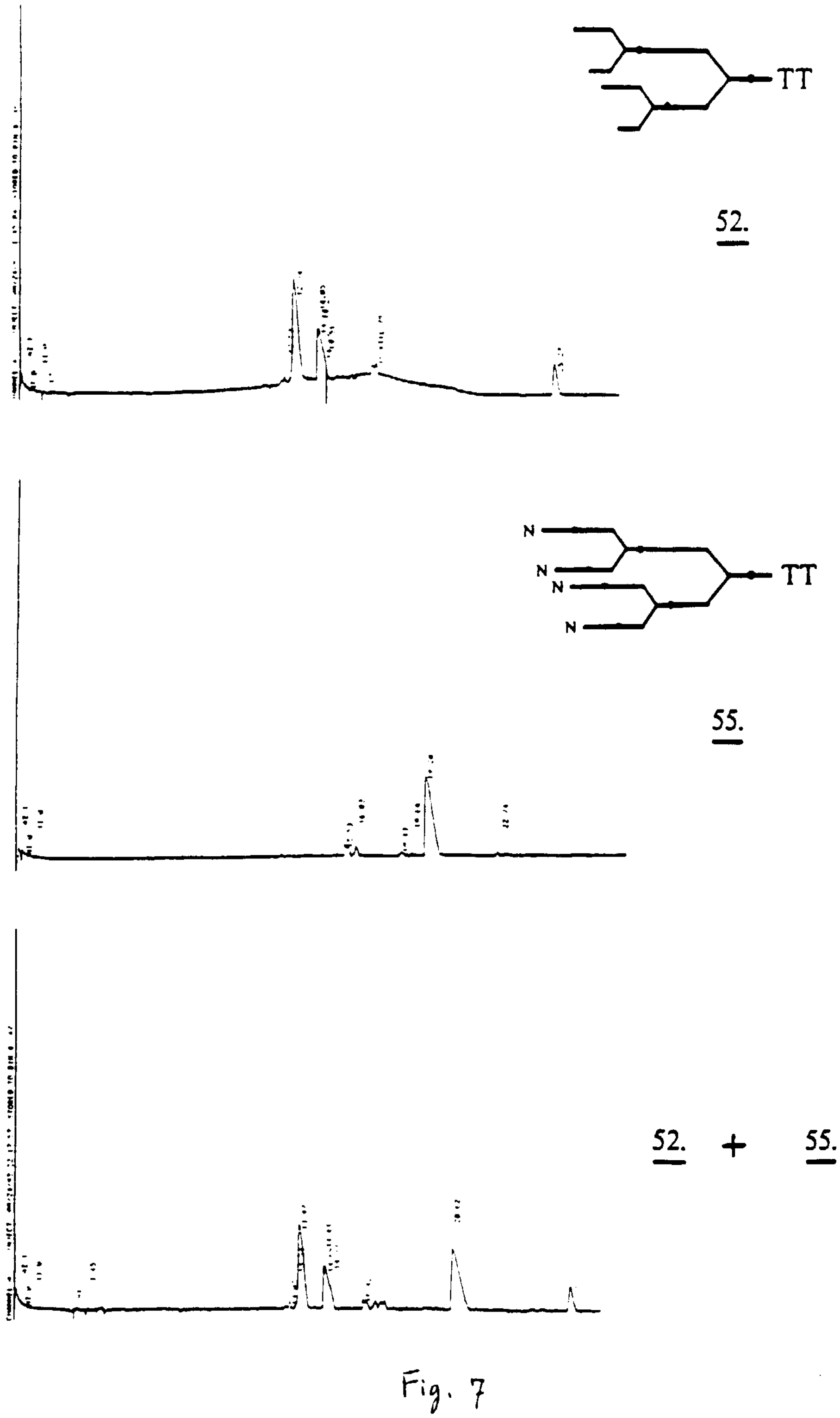


52.

55.

52. + 55.

Fig. 7

Constructions en candélabre

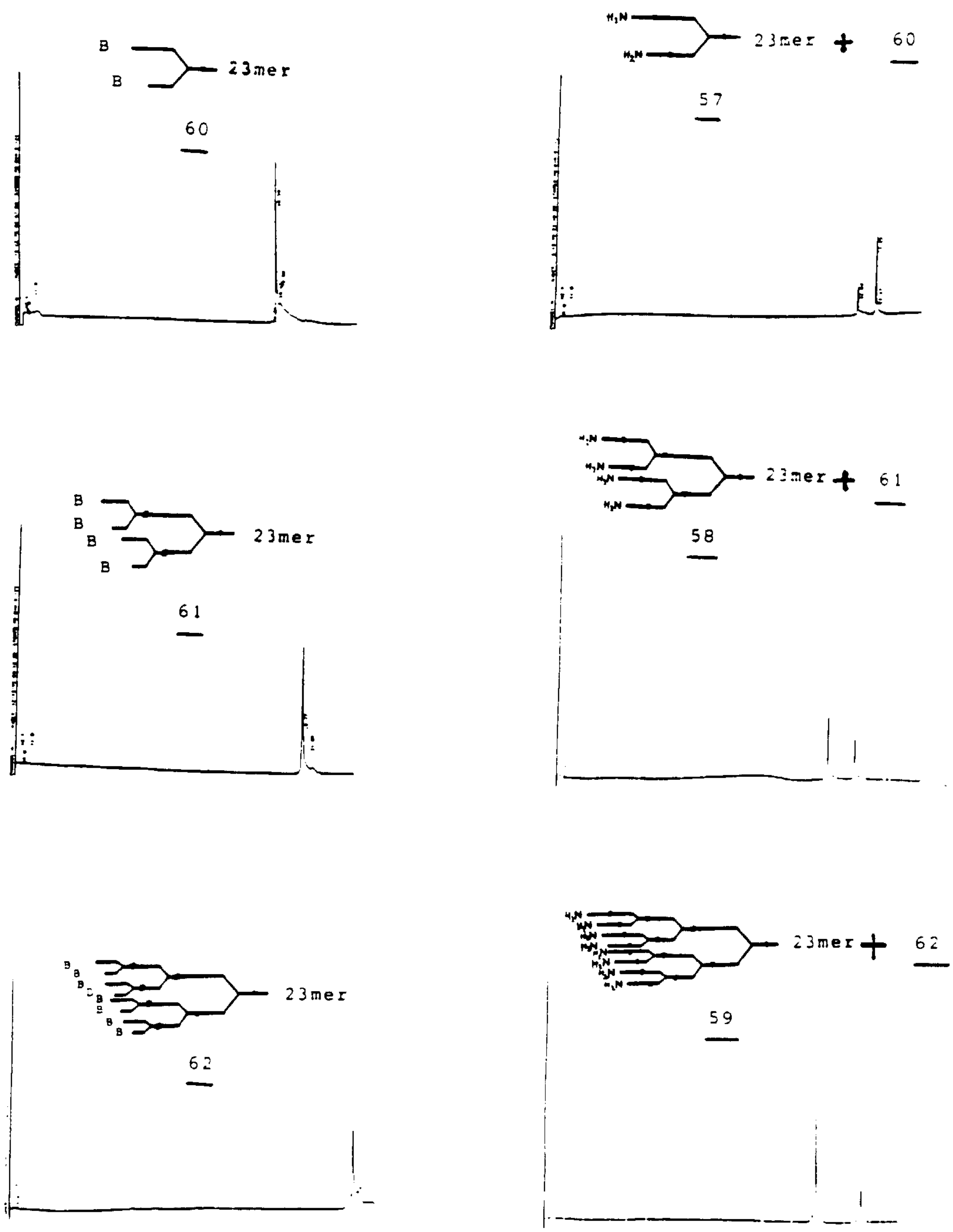


Fig. 8